# EUROPEAN PATENT APPLICATION

(11) **EP 4 461 715 A1**
(43) Date of publication of application: **13.11.2024**
(21) Application number: 23737304.8
(22) Date of filing: 06.01.2023
(51) Int. Cl.: C07B 51/00, C07C 209/62, C07C 211/29, C07C 211/45, C07C 211/58, C07D 211/26, C07D 213/16, C07D 295/03, C07D 401/10, C40B 50/14

(54) **METHOD FOR REMOVING TERT-BUTOXYCARBONYL GROUPS**

(30) Priority: 06.01.2022 JP 2022000838; 06.07.2022 JP 2022109105
(71) Applicant: CHUGAI SEIYAKU KABUSHIKI KAISHA, Tokyo 115-8543 (JP)
(72) Inventor: ITO, Taisuke, Gotemba-shi, Shizuoka 412-8513 (JP); KAMAKURA, Daiki, Gotemba-shi, Shizuoka 412-8513 (JP); HAYASE, Tadakatsu, Gotemba-shi, Shizuoka 412-8513 (JP)
(74) Representative: Vossius & Partner Patentanwälte Rechtsanwälte mbB
(86) International application number: PCT/JP2023/000123
(87) International publication number: WO 2023/132353

(57) **Abstract**

Provided herein is a method for removing a tert-butoxycarbonyl (Boc) group in a compound having a Boc group on a nitrogen atom, comprising treating the compound with a trifluoromethanesulfonic acid metal salt (metal triflate: M(OTf)ₙ) or rare-earth metal halide (MXₙ) in the presence of a base to obtain a compound in which the Boc group is converted to H-.

## Description

### TECHNICAL FIELD

The present invention relates to a method for removing a tert-butoxycarbonyl (Boc) group introduced as a protecting group. The present invention also relates to a method for producing a Boc-deprotected compound by removing a tert-butoxycarbonyl (Boc) group introduced as a protecting group. The present invention further relates to a method for producing an amine compound by removing a tert-butoxycarbonyl group introduced as a protecting group for an amino group.

### BACKGROUND ART

The tert-butoxycarbonyl (Boc) group is widely used as a protecting group for an amino group in the development and production of pharmaceuticals. Acidic conditions such as trifluoroacetic acid (TFA) are known as reaction conditions for deprotection by removal of the Boc group from the amino group (NPL 1).

It has been reported that deprotection by removal of the Boc group with TFA also proceeds with the cleavage of an O-paramethoxybenzyl group (O-PMB group) when the substrate has the O-PMB group, which is an alkoxybenzyl ether structure (NPLs 7 and 8). The methods for selectively removing the Boc group have been studied for the cases where the substrate has other reaction points decomposed under acidic conditions in the molecule. For example, although the use of TFA causes the cleavage of the tert-butyl ether structure when the substrate has a tert-butyl ether structure, it has been reported that the conditions using TMSOTf in combination with 2,6-lutidine allow de-Boc reaction while maintaining the tert-butyl ether structure (NPL 6).

The use of ZnBr₂ or Sn(OTf)₂ has also been reported as another Boc-deprotection method. The method using ZnBr₂ has been reported as an example of performing de-Boc reaction of a Boc-protected secondary amine in the presence of a Boc-protected primary amine (NPL 9). The method using Sn(OTf)₂ has been reported as an example of performing de-Boc reaction while maintaining tert-butyl ester and triisopropyl silyl ether (NPLs 10 and 11).

When the deprotection reaction of the Boc group is performed by solid phase synthesis, it is necessary to perform deprotection under different conditions from the cleavage conditions of the linker that links target compounds to a resin. For example, when a peptide is synthesized using a Boc method, the cleavage process of the product is performed with strong acids such as hydrofluoric acid (NPLs 2 and 3).

Solid-phase synthesis in which the cleavage process from the resin is performed under acidic conditions such as TFA is another common method. For example, when the compounds are immobilized on a Wang resin with a linker having an alkoxybenzyl ester structure, an alkoxybenzyl ether structure, or the like, the cleavage process of the product is performed with acids such as TFA (NPLs 2 and 4). There is also a report for conditions for deprotection by removal of the Boc group in solid phase synthesis with a Rink amide resin. It reports that the use of TFA causes cleavage from the solid phase, but a condition using trimethylsilyl triflate (TMSOTf) in combination with 2,6-lutidine allows the deprotection by removal of the Boc group while reducing the cleavage (NPL 5).

### CITATION LIST

### NON PATENT LITERATURE

NPL 1: Wuts, Peter G. M. "Greene's Protective Groups in Organic Synthesis, 5th edition" 2014, 930-946.
NPL 2: Doemer, B. et al. "Novabiochem Solid Phase Synthesis Handbook" 2018 Merck KGaA
NPL 3: Lenard, J. et al. J. Am. Chem. Soc. 1967, 89, 181-182.
NPL 4: James, I. W. Tetrahedron Lett. 1999, 55, 4855-5946.
NPL 5: Burgess, K. et al. Tetrahedron Lett. 1998, 39, 7439-7442.
NPL 6: Ottenheijm, H. C. J. et al. J. Org. Chem. 1997, 62, 3880-3889.
NPL 7: White, J. D.; Amedio, Jr. J. C. J. Org. Chem. 1989, 54, 736.
NPL 8: Fujii, N. et al. Bioorg. Med. Chem. 2013, 21, 1972-1977.
NPL 9: Nigama, S. C. et al. Synth. Commun. 1989, 19, 3139-3142.
NPL 10: Bose, D. S. et al. Synth. Commun. 2003, 33, 445-450.
NPL 11: Ricci, L. et al. Org. Biomol. Chem. 2017, 15, 6826-6836.

### SUMMARY OF INVENTION

### TECHNICAL PROBLEM

The Boc protecting group is widely used as a protecting group for an amino group in compound synthesis such as in the development of pharmaceuticals. However, problems that have not yet been solved under conventional deprotection conditions still remain, such as some substrates causing a significant decrease in yield in the step of deprotection by removal of Boc protecting group and/or generating a large number of byproducts. For example, a step of deprotection by removal of a Boc group has been attempted by (i) immobilizing the substrate on a resin with a linker containing an alkoxybenzyl ether structure that is commonly used in solid-phase synthesis, but a significant decrease in yield has been confirmed under existing Boc-deprotection conditions. A step of deprotection by removal of a Boc group has also been attempted by (ii) using a PAL AM resin, and immobilizing the substrate on the resin with a linker containing a 2,4,6-trialkoxybenzylaminocarbonyl structure, (iii) using a Rink Amide resin and immobilizing the substrate on the resin with a linker containing a 2',4'-dialkoxyphenyl-4-alkoxyphenylmethylaminocarbonyl structure, or (iv) using a Sieber Amide resin and immobilizing the substrate on the resin with a linker containing a 3-alkoxyxanthen-9-ylaminocarbonyl structure. However, in each case, a significant decrease in yield has been confirmed under existing Boc-deprotection conditions. Even though the de-Boc condition using TMSOTf and 2,6-lutidine referred to NPL 5 has been applied in the solid-phase synthesis with those linker structures, the improvement in yield has been minor.

In addition, when a step of deprotection of the Boc group is performed in a liquid phase synthesis, for example, with an alkoxybenzyl ether structure, decomposition of the alkoxybenzyl ether structure has been confirmed under existing Boc-deprotection conditions. Although the condition using TMSOTf and 2,6-lutidine referred to NPL 6, the condition using ZnBr₂ referred to NPL 9, and the condition using Sn(OTf)₂ referred to NPL 10 have been applied, it has been confirmed that they are not conditions that can effectively suppress the decomposition of the alkoxybenzyl ether structure in the Boc-deprotection reaction.

The present invention has been made in view of such situations, and in one aspect, an object of the present invention is to provide a Boc group-deprotection method capable of improving yield and/or suppressing byproducts in a deprotection reaction of a Boc protecting group. Also in one aspect, another object of the present invention is to provide a method for synthesizing a compound, comprising the method described above. In another aspect, an object of the present invention is to provide a method for improving yield and/or a method for suppressing byproducts due to decomposition of structures susceptible to acid decomposition, such as an alkoxybenzyl ether structure, a trialkoxybenzyl aminocarbonyl structure, a dialkoxyphenyl-alkoxyphenylmethyl aminocarbonyl structure, or an alkoxyxanthen-9-yl aminocarbonyl structure, applicable in a Boc group-deprotection method.

### SOLUTION TO PROBLEM

The present inventors have found that, in the deprotection reaction of a Boc group, a condition comprising a metal triflate or rare-earth metal halide and a base can improve the yield of an amine compound in which the Boc group is removed and/or produce an amine compound while suppressing byproducts. The present inventors have further found that this condition can be applied to the deprotection reactions of various Boc groups, and have completed the present invention. In one aspect, the present invention discloses the following invention.

[A-1] A method for producing a compound, comprising treating a compound having a tert-butoxycarbonyl (Boc) group on a nitrogen atom with a trifluoromethanesulfonic acid metal salt (metal triflate: M(OTf)ₙ) or rare-earth metal halide (MXₙ) in the presence of a base to obtain a compound in which the Boc group is removed.

[A-2] The method according to [A-1], wherein the metal triflate or rare-earth metal halide is selected from the group consisting of tin triflate (Sn(OTf)₂), calcium triflate (Ca(OTf)₂), barium triflate (Ba(OTf)₂), zinc triflate (Zn(OTf)₂), aluminum triflate (Al(OTf)₃), gallium triflate (Ga(OTf)₃), indium triflate (In(OTf)₃), bismuth triflate (Bi(OTf)₃), lithium triflate (Li(OTf)), scandium triflate (Sc(OTf)₃), yttrium triflate (Y(OTf)₃), lanthanum triflate (La(OTf)₃), cerium triflate (Ce(OTf)₃, Ce(OTf)₄), praseodymium triflate (Pr(OTf)₃), neodymium triflate (Nd(OTf)₃), samarium triflate (Sm(OTf)₃), europium triflate (Eu(OTf)₃), gadolinium triflate (Gd(OTf)₃), terbium triflate (Tb(OTf)₃), dysprosium triflate (Dy(OTf)₃), holmium triflate (Ho(OTf)₃), erbium triflate (Er(OTf)₃), thulium triflate (Tm(OTf)₃), ytterbium triflate (Yb(OTf)₃), lutetium triflate (Lu(OTf)₃), hafnium triflate (Hf(OTf)₄), erbium chloride (ErCl₃), erbium bromide (ErBr₃), erbium iodide (ErI₃), thulium chloride (TmCl₃), thulium bromide (TmBr₃), thulium iodide (TmI₃), ytterbium chloride (YbCl₃), ytterbium bromide (YbBr₃), and ytterbium iodide (YbI₃), and two or more of the metal triflate or rare-earth metal halide may be used.

[A-3] The method according to [A-1] or [A-2], wherein the metal triflate or rare-earth metal halide is selected from the group consisting of Sn(OTf)₂, Er(OTf)₃, Tm(OTf)₃, Y(OTf)₃, Yb(OTf)₃, ErCl₃, ErBr₃, and ErI₃, and two or more of the metal triflate or rare-earth metal halide may be used.

[A-4] The method according to any one of [A-1] to [A-3], wherein the metal triflate is selected from the group consisting of Sn(OTf)₂, Er(OTf)₃, Tm(OTf)₃, and Y(OTf)₃, and two or more of the metal triflate may be used.

[A-5] The method according to any one of [A-1] to [A-4], wherein the base is selected from the group consisting of organic bases having pKa of conjugate acid in water of 1 to 14, and two or more of the base may be used.

[A-6] The method according to any one of [A-1] to [A-5], wherein the base is selected from the group consisting of organic bases having pKa of conjugate acid in water of 2 to 12, 3 to 11, 3 to 10, 3 to 9, 3 to 8, or 3.5 to 7.5, and two or more of the base may be used.

[A-7] The method according to any one of [A-1] to [A-6], wherein the base is selected from the group consisting of bases represented by formula (B1) or (B2): 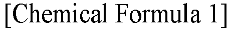 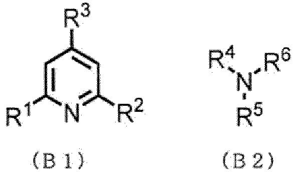
wherein R¹, R², and R³ are each independently selected from a hydrogen atom, C₁₋₆ alkyl, or C₆₋₁₀ aryl;
R⁴ and R⁵ together with a nitrogen atom to which they are attached form a 5- to 7-membered saturated heterocycle, the heterocycle optionally containing O, S, or N, or R⁴ and R⁵ are each independently C₁₋₆ alkyl, C₆₋₁₀ aryl, or tri(C₁₋₆ alkyl)silyl; and
R⁶ is a hydrogen atom or C₁₋₆ alkyl, and
two or more of the base may be used.

[A-8] The method according to [A-7], wherein the R¹ and R² are C₁₋₆ alkyl, and R³ is a hydrogen atom or C₁₋₆ alkyl;
R⁴ and R⁵ together with a nitrogen atom to which they are attached form a 5- to 7-membered saturated heterocycle, the heterocycle optionally containing O, or R⁴ and R⁵ are each independently C₁₋₆ alkyl or tri(C₁₋₆ alkyl)silyl; and
R⁶ is a hydrogen atom or C₁₋₆ alkyl.

[A-9] The method according to any one of [A-1] to [A-8], wherein the base is selected from the group consisting of 2,6-lutidine, N,N-diisopropylethylamine, N-methylmorpholine, 2,6-di-tert-butylpyridine, and di(trimethylsilyl)amine, and two or more of the base may be used.

[A-10] The method according to any one of [A-1] to [A-9], wherein the base is selected from the group consisting of 2,6-lutidine, N-methylmorpholine, and 2,6-di-tert-butylpyridine, and two or more of the base may be used.

[A-11] The method according to any one of [A-1] to [A-10], wherein the treating is performed in the presence of a solvent.

[A-12] The method according to any one of [A-1] to [A-11], wherein the treating is performed in the presence of a solvent, and the solvent is selected from the group consisting of an ether-based solvent, a halogen-based solvent, a nitrile-based solvent, an aromatic hydrocarbon-based solvent, and an ester-based solvent, and two or more of the solvent may be used.

[A-13] The method according to [A-12], wherein the ether-based solvent is selected from the group consisting of tetrahydrofuran, 2-methyltetrahydrofuran, 4-methyltetrahydropyran, 1,2-dimethoxyethane, diethyl ether, and 1,4-dioxane.

[A-14] The method according to [A-12], wherein the halogen-based solvent is selected from the group consisting of dichloromethane, 1,2-dichloroethane, and chloroform.

[A-15] The method according to [A-12], wherein the nitrile-based solvent is selected from the group consisting of acetonitrile and propionitrile.

[A-16] The method according to [A-12], wherein the aromatic hydrocarbon-based solvent is selected from the group consisting of benzene, toluene, and xylene.

[A-17] The method according to any one of [A-1] to [A-16], wherein a molar ratio of the metal triflate or rare-earth metal halide and the base used is metal triflate or rare-earth metal halide/base = 10/1 to 1/20.

[A-18] The method according to any one of [A-1] to [A-17], wherein a molar ratio of the metal triflate or rare-earth metal halide and the base used is metal triflate or rare-earth metal halide/base = 5/1 to 1/10, 2/1 to 1/5, 1/1 to 1/5, or 1/1 to 1/2.

[A-19] The method according to any one of [A-1] to [A-18], wherein a molar ratio of the metal triflate or rare-earth metal halide used to the compound having a Boc group on a nitrogen atom is 100 to 0.1 equivalents.

[A-20] The method according to any one of [A-1] to [A-19], wherein a molar ratio of the metal triflate or rare-earth metal halide used to the compound having a Boc group on a nitrogen atom is 70 to 0.2 equivalents, 50 to 0.5 equivalents, 30 to 1 equivalents, or 20 to 1 equivalents.

[A-21] The method according to any one of [A-1] to [A-20], wherein the treating is performed at a reaction temperature of 0°C to 120°C.

[A-22] The method according to any one of [A-1] to [A-21], wherein the treating is performed at a reaction temperature of 10°C to 100°C, 15°C to 100°C, 20°C to 90°C, 25°C to 80°C, 30°C to 80°C, 35°C to 80°C, or 40°C to 80°C.

[A-23] The method according to any one of [A-1] to [A-22], wherein the compound having a Boc group on a nitrogen atom is a resin for solid-phase synthesis to which the compound having a Boc group is attached via a linker.

[A-24] The method according to any one of [A-1] to [A-23], wherein the compound having a Boc group on a nitrogen atom is a compound represented by formula (A): 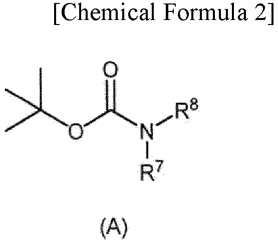 wherein R⁷ and R⁸ together with a nitrogen atom to which they are attached form a 5- to 7-membered saturated heterocycle, where the heterocycle is optionally substituted with an arbitrary substituent, or R⁷ and R⁸ are each independently a hydrogen atom, C₁₋₆ alkyl, C₂₋₆ alkenyl, C₂₋₆ alkynyl, C₃₋₈ cycloalkyl, C₇₋₁₄ aralkyl, C₆₋₁₀ aryl, or 5- to 10-membered heteroaryl containing one or more ring heteroatoms independently selected from O, N, and S, each of which is optionally substituted with one or more substituents, or
wherein the compound having a Boc group on a nitrogen atom is a resin for solid-phase synthesis to which the compound represented by formula (A) is attached via a linker.

[A-25] The method according to [A-24], wherein the R⁷ and R⁸ together with a nitrogen atom to which they are attached form a 5- to 7-membered saturated heterocycle, and the heterocycle is optionally substituted with one or more substituents independently selected from the group consisting of a halogen atom, cyano, C₁₋₆ alkyl, C₁₋₆ alkoxy, (C₁₋₆ alkoxy)carbonyl, (C₁₋₆ alkoxy)carbonylamino, (C₁₋₆ alkyl)carbonylamino, (C₆₋₁₀ aryl)carbonylamino, 5- to 10-membered heteroarylcarbonylamino containing one or more ring heteroatoms independently selected from O, N and S, di(C₁₋₆ alkyl)amino, 4- to 8-membered cyclic amino, aminocarbonyl, (C₁₋₆ alkyl)aminocarbonyl, di(C₁₋₆ alkyl)aminocarbonyl, and 4- to 8-membered cyclic aminocarbonyl.

[A-26] The method according to any one of [A-1] to [A-25], wherein the method comprises, after the treating, a step of mixing with a solution containing a compound selected from the group consisting of N,N'-dimethylethylenediamine, ethylenediamine, diethylenetriamine, tris(2-aminoethyl)amine, tris(3-aminopropyl)amine, bis(3-aminopropyl)amine, 2,2'-bipyridyl, and 4,4'-di-tert-butyl-2,2'-bipyridyl.

[A-27] The method according to any one of [A-1] to [A-25], wherein the method comprises, after the treating, a step of mixing with a solution containing N,N'-dimethylethylenediamine or 4,4'-di-tert-butyl-2,2'-bipyridyl.

[A-28] The method according to any one of [A-23] to [A-25], wherein the method comprises, after the treating, a step of washing a resin for solid-phase synthesis with a solution containing a compound selected from the group consisting of N,N'-dimethylethylenediamine, ethylenediamine, diethylenetriamine, tris(2-aminoethyl)amine, tris(3-aminopropyl)amine, bis(3-aminopropyl)amine, 2,2'-bipyridyl, and 4,4'-di-tert-butyl-2,2'-bipyridyl.

[A-29] The method according to any one of [A-23] to [A-25], wherein the method comprises, after the treating, a step of washing a resin for solid-phase synthesis with a solution containing N,N'-dimethylethylenediamine or 4,4'-di-tert-butyl-2,2'-bipyridyl.

[A-30] The method according to any one of [A-1] to [A-29], wherein the compound having a Boc group on a nitrogen atom comprises a structure capable of being decomposed by treatment under an acidic condition, the structure being other than a Boc group.

[A-31] The method according to [A-30], wherein the structure capable of being decomposed by treatment under an acidic condition is selected from the group consisting of an alkoxybenzyl ether structure, a trialkoxybenzylaminocarbonyl structure, a dialkoxyphenyl-alkoxyphenylmethylaminocarbonyl structure, and an alkoxyxanthen-9-ylaminocarbonyl structure.

[A-32] The method according to [A-30] or [A-31], wherein the structure capable of being decomposed by treatment under an acidic condition is an alkoxybenzyl ether structure.

[A-33] The method according to any one of [A-23] to [A-32], wherein the linker comprises a structure selected from the group consisting of an alkoxybenzyl ether structure, a trialkoxybenzylaminocarbonyl structure, a dialkoxyphenyl-alkoxyphenylmethylaminocarbonyl structure, and an alkoxyxanthen-9-ylaminocarbonyl structure as a structure capable of being decomposed by treatment under an acidic condition.

[A-34] The method according to any one of [A-23] to [A-32], wherein the linker comprises an alkoxybenzyl ether structure as a structure capable of being decomposed by treatment under an acidic condition.

[A-35] The method according to any one of [A-23] to [A-34], wherein the resin for solid-phase synthesis is selected from the group consisting of a Carboxylic resin, a Wang resin, a PAL AM resin, a Rink amide resin, and a Sieber amide resin.

[A-36] The method according to any one of [A-23] to [A-34], wherein the resin for solid-phase synthesis is a Carboxylic resin.

[A-37] The method according to any one of [A-23] to [A-30], wherein the resin for solid-phase synthesis is selected from the group consisting of a Carboxylic resin, a Wang resin, a PAL AM resin, a Rink amide resin, and a Sieber amide resin, and the linker comprises a structure selected from the group consisting of an alkoxybenzyl ether structure, a trialkoxybenzylaminocarbonyl structure, a dialkoxyphenyl-alkoxyphenylmethylaminocarbonyl structure, and an alkoxyxanthen-9-ylaminocarbonyl structure as a structure capable of being decomposed by treatment under an acidic condition.

[A-38] The method according to any one of [A-23] to [A-30], wherein the resin for solid-phase synthesis is a Carboxylic resin, and the linker comprises an alkoxybenzyl ether structure as a structure capable of being decomposed by treatment under an acidic condition.

[A-39] The method according to any one of [A-30] to [A-38], wherein the treatment under an acidic condition is treating in dichloromethane, 1,2-dichloroethane, chloroform, toluene, acetonitrile, tetrahydrofuran, 1,4-dioxane, 1,2-dimethoxyethane, diethyl ether, acetone, ethyl acetate, N,N-dimethylformamide, methanol, ethanol, isopropanol, 1-butanol, 2,2,2-trifluoroethanol or water containing 10 v/v% trifluoroacetic acid.

[A-40] The method according to any one of [A-1] to [A-39], wherein the method comprises treating a mixture containing two or more different compounds having a Boc group on a nitrogen atom.

[A-41] The method according to any one of [A-1] to [A-40], for producing a compound constituting a compound library.

[A-42] The method according to any one of [A-1] to [A-41], wherein the compound having a Boc group on a nitrogen atom is a resin for solid-phase synthesis to which two or more different compounds having a Boc group are attached via linkers.

[A-43] The method according to any one of [A-1] to [A-42], wherein the compound having a Boc group on a nitrogen atom is a resin for solid-phase synthesis to which 3 or more, 4 or more, 5 or more, 7 or more, or 10 or more different compounds having a Boc group are attached via linkers.

[A-44] The method according to [A-12], wherein the ester-based solvent is selected from the group consisting of ethyl acetate, isopropyl acetate, and butyl acetate.

[A-45] The method according to any one of [A-30] to [A-43], wherein the linker comprises the structure capable of being decomposed by treatment under an acidic condition.

[A-46] A method for producing a compound constituting a compound library, the method comprising producing the compound by the method according to any one of [A-1] to [A-45].

[B-1] A method for removing a tert-butoxycarbonyl (Boc) group in a compound having a Boc group on a nitrogen atom, comprising treating the compound with a trifluoromethanesulfonic acid metal salt (metal triflate: M(OTf)ₙ) or rare-earth metal halide (MXₙ) in the presence of a base to obtain a compound in which the Boc group is converted to H-.

[B-2] The method according to [B-1], wherein the metal triflate or rare-earth metal halide is selected from the group consisting of tin triflate (Sn(OTf)₂), calcium triflate (Ca(OTf)₂), barium triflate (Ba(OTf)₂), zinc triflate (Zn(OTf)₂), aluminum triflate (Al(OTf)₃), gallium triflate (Ga(OTf)₃), indium triflate (In(OTf)₃), bismuth triflate (Bi(OTf)₃), lithium triflate (Li(OTf)), scandium triflate (Sc(OTf)₃), yttrium triflate (Y(OTf)₃), lanthanum triflate (La(OTf)₃), cerium triflate (Ce(OTf)₃, Ce(OTf)₄), praseodymium triflate (Pr(OTf)₃), neodymium triflate (Nd(OTf)₃), samarium triflate (Sm(OTf)₃), europium triflate (Eu(OTf)₃), gadolinium triflate (Gd(OTf)₃), terbium triflate (Tb(OTf)₃), dysprosium triflate (Dy(OTf)₃), holmium triflate (Ho(OTf)₃), erbium triflate (Er(OTf)₃), thulium triflate (Tm(OTf)₃), ytterbium triflate (Yb(OTf)₃), lutetium triflate (Lu(OTf)₃), hafnium triflate (Hf(OTf)₄), erbium chloride (ErCl₃), erbium bromide (ErBr₃), erbium iodide (ErI₃), thulium chloride (TmCl₃), thulium bromide (TmBr₃), thulium iodide (TmI₃), ytterbium chloride (YbCl₃), ytterbium bromide (YbBr₃), and ytterbium iodide (YbI₃), and two or more of the metal triflate or rare-earth metal halide may be used.

[B-3] The method according to [B-1] or [B-2], wherein the metal triflate or rare-earth metal halide is selected from the group consisting of Sn(OTf)₂, Er(OTf)₃, Tm(OTf)₃, Y(OTf)₃, Yb(OTf)₃, ErCl₃, ErBr₃, and ErI₃, and two or more of the metal triflate or rare-earth metal halide may be used.

[B-4] The method according to any one of [B-1] to [B-3], wherein the metal triflate is selected from the group consisting of Sn(OTf)₂, Er(OTf)₃, Tm(OTf)₃, and Y(OTf)₃, and two or more of the metal triflate may be used.

[B-5] The method according to any one of [B-1] to [B-4], wherein the base is selected from the group consisting of organic bases having pKa of conjugate acid in water of 1 to 14, and two or more of the base may be used.

[B-6] The method according to any one of [B-1] to [B-5], wherein the base is selected from the group consisting of organic bases having pKa of conjugate acid in water of 2 to 12, 3 to 11, 3 to 10, 3 to 9, 3 to 8, or 3.5 to 7.5, and two or more of the base may be used.

[B-7] The method according to any one of [B-1] to [B-6], wherein the base is selected from the group consisting of bases represented by formula (B1) or (B2): 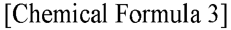 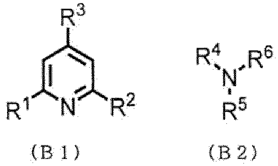
wherein R¹, R², and R³ are each independently selected from a hydrogen atom, C₁₋₆ alkyl, or C₆₋₁₀ aryl;
R⁴ and R⁵ together with a nitrogen atom to which they are attached form a 5- to 7-membered saturated heterocycle, the heterocycle optionally containing O, S, or N, or R⁴ and R⁵ are each independently C₁₋₆ alkyl, C₆₋₁₀ aryl, or tri(C₁₋₆ alkyl)silyl; and
R⁶ is a hydrogen atom or C₁₋₆ alkyl, and
two or more of the base may be used.

[B-8] The method according to [B-7], wherein the R¹ and R² are C₁₋₆ alkyl, and R³ is a hydrogen atom or C₁₋₆ alkyl;
R⁴ and R⁵ together with a nitrogen atom to which they are attached form a 5- to 7-membered saturated heterocycle, the heterocycle optionally containing O, or R⁴ and R⁵ are each independently C₁₋₆ alkyl or tri(C₁₋₆ alkyl)silyl; and
R⁶ is a hydrogen atom or C₁₋₆ alkyl.

[B-9] The method according to any one of [B-1] to [B-8], wherein the base is selected from the group consisting of 2,6-lutidine, N,N-diisopropylethylamine, N-methylmorpholine, 2,6-di-tert-butylpyridine, and di(trimethylsilyl)amine, and two or more of the base may be used.

[B-10] The method according to any one of [B-1] to [B-9], wherein the base is selected from the group consisting of 2,6-lutidine, N-methylmorpholine, and 2,6-di-tert-butylpyridine, and two or more of the base may be used.

[B-11] The method according to any one of [B-1] to [B-10], wherein the treating is performed in the presence of a solvent.

[B-12] The method according to any one of [B-1] to [B-11], wherein the treating is performed in the presence of a solvent, and the solvent is selected from the group consisting of an ether-based solvent, a halogen-based solvent, a nitrile-based solvent, an aromatic hydrocarbon-based solvent, and an ester-based solvent, and two or more of the solvent may be used.

[B-13] The method according to [B-12], wherein the ether-based solvent is selected from the group consisting of tetrahydrofuran, 2-methyltetrahydrofuran, 4-methyltetrahydropyran, 1,2-dimethoxyethane, diethyl ether, and 1,4-dioxane.

[B-14] The method according to [B-12], wherein the halogen-based solvent is selected from the group consisting of dichloromethane, 1,2-dichloroethane, and chloroform.

[B-15] The method according to [B-12], wherein the nitrile-based solvent is selected from the group consisting of acetonitrile and propionitrile.

[B-16] The method according to [B-12], wherein the aromatic hydrocarbon-based solvent is selected from the group consisting of benzene, toluene, and xylene.

[B-17] The method according to any one of [B-1] to [B-16], wherein a molar ratio of the metal triflate or rare-earth metal halide and the base is metal triflate or rare-earth metal halide/base = 10/1 to 1/20.

[B-18] The method according to any one of [B-1] to [B-17], wherein a molar ratio of the metal triflate or rare-earth metal halide and the base used is metal triflate or rare-earth metal halide/base = 5/1 to 1/10, 2/1 to 1/5, 1/1 to 1/5, or 1/1 to 1/2.

[B-19] The method according to any one of [B-1] to [B-18], wherein a molar ratio of the metal triflate or rare-earth metal halide used to the compound having a Boc group on a nitrogen atom is 100 to 0.1 equivalents.

[B-20] The method according to any one of [B-1] to [B-19], wherein a molar ratio of the metal triflate or rare-earth metal halide used to the compound having a Boc group on a nitrogen atom is 70 to 0.2 equivalents, 50 to 0.5 equivalents, 30 to 1 equivalents, or 20 to 1 equivalents.

[B-21] The method according to any one of [B-1] to [B-20], wherein the treating is performed at a reaction temperature of 0°C to 120°C.

[B-22] The method according to any one of [B-1] to [B-21], wherein the treating is performed at a reaction temperature of 10°C to 100°C, 15°C to 100°C, 20°C to 90°C, 25°C to 80°C, 30°C to 80°C, 35°C to 80°C, or 40°C to 80°C.

[B-23] The method according to any one of [B-1] to [B-22], wherein the compound having a Boc group on a nitrogen atom is a resin for solid-phase synthesis to which the compound having a Boc group is attached via a linker.

[B-24] The method according to any one of [B-1] to [B-23], wherein the compound having a Boc group on a nitrogen atom is a compound represented by formula (A): 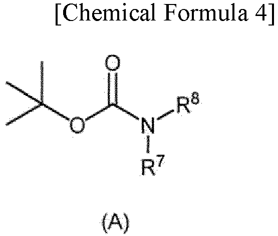 wherein R⁷ and R⁸ together with a nitrogen atom to which they are attached form a 5- to 7-membered saturated heterocycle, where the heterocycle is optionally substituted with an arbitrary substituent, or R⁷ and R⁸ are each independently a hydrogen atom, C₁₋₆ alkyl, C₂₋₆ alkenyl, C₂₋₆ alkynyl, C₃₋₈ cycloalkyl, C₇₋₁₄ aralkyl, C₆₋₁₀ aryl, or 5- to 10-membered heteroaryl containing one or more ring heteroatoms independently selected from O, N, and S, each of which is optionally substituted with one or more arbitrary substituents, or
wherein the compound having a Boc group on a nitrogen atom is a resin for solid-phase synthesis to which the compound represented by formula (A) is attached via a linker.

[B-25] The method according to [B-24], wherein the R⁷ and R⁸ together with a nitrogen atom to which they are attached form a 5- to 7-membered saturated heterocycle, and the heterocycle is optionally substituted with one or more substituents independently selected from the group consisting of a halogen atom, cyano, C₁₋₆ alkyl, C₁₋₆ alkoxy, (C₁₋₆ alkoxy)carbonyl, (C₁₋₆ alkoxy)carbonylamino, (C₁₋₆ alkyl)carbonylamino, (C₆₋₁₀ aryl)carbonylamino, 5- to 10-membered heteroarylcarbonylamino containing one or more ring heteroatoms independently selected from O, N and S, di(C₁₋₆ alkyl)amino, 4- to 8-membered cyclic amino, aminocarbonyl, (C₁₋₆ alkyl)aminocarbonyl, di(C₁₋₆ alkyl)aminocarbonyl, and 4- to 8-membered cyclic aminocarbonyl.

[B-26] The method according to any one of [B-1] to [B-25], wherein the method comprises, after the treating, a step of mixing with a solution containing a compound selected from the group consisting of N,N'-dimethylethylenediamine, ethylenediamine, diethylenetriamine, tris(2-aminoethyl)amine, tris(3-aminopropyl)amine, bis(3-aminopropyl)amine, 2,2'-bipyridyl, and 4,4'-di-tert-butyl-2,2'-bipyridyl.

[B-27] The method according to any one of [B-1] to [B-25], wherein the method comprises, after the treating, a step of mixing with a solution containing N,N'-dimethylethylenediamine or 4,4'-di-tert-butyl-2,2'-bipyridyl.

[B-28] The method according to any one of [B-23] to [B-25], wherein the method comprises, after the treating, a step of washing a resin for solid-phase synthesis with a solution containing a compound selected from the group consisting of N,N'-dimethylethylenediamine, ethylenediamine, diethylenetriamine, tris(2-aminoethyl)amine, tris(3-aminopropyl)amine, bis(3-aminopropyl)amine, 2,2'-bipyridyl, and 4,4'-di-tert-butyl-2,2'-bipyridyl.

[B-29] The method according to any one of [B-23] to [B-25], wherein the method comprises, after the treating, a step of washing a resin for solid-phase synthesis with a solution containing N,N'-dimethylethylenediamine or 4,4'-di-tert-butyl-2,2'-bipyridyl.

[B-30] The method according to any one of [B-1] to [B-29], wherein the compound having a Boc group on a nitrogen atom comprises a structure capable of being decomposed by treatment under an acidic condition, the structure being other than a Boc group.

[B-31] The method according to [B-30], wherein the structure capable of being decomposed by treatment under an acidic condition is selected from the group consisting of an alkoxybenzyl ether structure, a trialkoxybenzylaminocarbonyl structure, a dialkoxyphenyl-alkoxyphenylmethylaminocarbonyl structure, and an alkoxyxanthen-9-ylaminocarbonyl structure.

[B-32] The method according to [B-30] or [B-31], wherein the structure capable of being decomposed by treatment under an acidic condition is an alkoxybenzyl ether structure.

[B-33] The method according to any one of [B-23] to [B-32], wherein the linker comprises a structure selected from the group consisting of an alkoxybenzyl ether structure, a trialkoxybenzylaminocarbonyl structure, a dialkoxyphenyl-alkoxyphenylmethylaminocarbonyl structure, and an alkoxyxanthen-9-ylaminocarbonyl structure as a structure capable of being decomposed by treatment under an acidic condition.

[B-34] The method according to any one of [B-23] to [B-32], wherein the linker comprises an alkoxybenzyl ether structure as a structure capable of being decomposed by treatment under an acidic condition.

[B-35] The method according to any one of [B-23] to [B-34], wherein the resin for solid-phase synthesis is selected from the group consisting of a Carboxylic resin, a Wang resin, a PAL AM resin, a Rink amide resin, and a Sieber amide resin.

[B-36] The method according to any one of [B-23] to [B-34], wherein the resin for solid-phase synthesis is a Carboxylic resin.

[B-37] The method according to any one of [B-23] to [B-30], wherein the resin for solid-phase synthesis is selected from the group consisting of a Carboxylic resin, a Wang resin, a PAL AM resin, a Rink amide resin, and a Sieber amide resin, and the linker comprises a structure selected from the group consisting of an alkoxybenzyl ether structure, a trialkoxybenzylaminocarbonyl structure, a dialkoxyphenyl-alkoxyphenylmethylaminocarbonyl structure, and an alkoxyxanthen-9-ylaminocarbonyl structure as a structure capable of being decomposed by treatment under an acidic condition.

[B-38] The method according to any one of [B-23] to [B-30], wherein the resin for solid-phase synthesis is a Carboxylic resin, and the linker comprises an alkoxybenzyl ether structure as a structure capable of being decomposed by treatment under an acidic condition.

[B-39] The method according to [B-26], wherein the treatment under an acidic condition is treating in dichloromethane, 1,2-dichloroethane, chloroform, toluene, acetonitrile, tetrahydrofuran, 1,4-dioxane, 1,2-dimethoxyethane, diethyl ether, acetone, ethyl acetate, N,N-dimethylformamide, methanol, ethanol, isopropanol, 1-butanol, 2,2,2-trifluoroethanol or water containing 10 v/v% trifluoroacetic acid.

[B-40] The method according to any one of [B-1] to [B-39], wherein the method comprises treating a mixture containing two or more different compounds having a Boc group on a nitrogen atom.

[B-41] The method according to any one of [B-1] to [B-40], for producing a compound constituting a compound library.

[B-42] The method according to any one of [B-1] to [B-41], wherein the compound having a Boc group on a nitrogen atom is a resin for solid-phase synthesis to which two or more different compounds having a Boc group are attached via linkers.

[B-43] The method according to any one of [B-1] to [B-42], wherein the compound having a Boc group on a nitrogen atom is a resin for solid-phase synthesis to which 3 or more, 4 or more, 5 or more, 7 or more, or 10 or more different compounds having a Boc group are attached via linkers.

[B-44] The method according to [B-12], wherein the ester-based solvent is selected from the group consisting of ethyl acetate, isopropyl acetate, and butyl acetate.

[B-45] The method according to any one of [B-30] to [B-43], wherein the linker comprises the structure capable of being decomposed by treatment under an acidic condition.

[B-46] A method for producing a compound constituting a compound library, the method comprising removing a Boc group by the method according to any one of [B-1] to [B-41].

[C-1] A composition comprising a trifluoromethanesulfonic acid metal salt (metal triflate: M(OTf)ₙ) or rare-earth metal halide (MXₙ), for use in a deprotection reaction of a tert-butoxycarbonyl (Boc) group in a compound having a Boc group on a nitrogen atom, wherein the deprotection reaction is performed in the presence of a base.

[C-2] The composition according to [C-1], wherein the metal triflate or rare-earth metal halide is selected from the group consisting of tin triflate (Sn(OTf)₂), calcium triflate (Ca(OTf)₂), barium triflate (Ba(OTf)₂), zinc triflate (Zn(OTf)₂), aluminum triflate (Al(OTf)₃), gallium triflate (Ga(OTf)₃), indium triflate (In(OTf)₃), bismuth triflate (Bi(OTf)₃), lithium triflate (Li(OTf)), scandium triflate (Sc(OTf)₃), yttrium triflate (Y(OTf)₃), lanthanum triflate (La(OTf)₃), cerium triflate (Ce(OTf)₃, Ce(OTf)₄), praseodymium triflate (Pr(OTf)₃), neodymium triflate (Nd(OTf)₃), samarium triflate (Sm(OTf)₃), europium triflate (Eu(OTf)₃), gadolinium triflate (Gd(OTf)₃), terbium triflate (Tb(OTf)₃), dysprosium triflate (Dy(OTf)₃), holmium triflate (Ho(OTf)₃), erbium triflate (Er(OTf)₃), thulium triflate (Tm(OTf)₃), ytterbium triflate (Yb(OTf)₃), lutetium triflate (Lu(OTf)₃), hafnium triflate (Hf(OTf)₄), erbium chloride (ErCl₃), erbium bromide (ErBr₃), erbium iodide (ErI₃), thulium chloride (TmCl₃), thulium bromide (TmBr₃), thulium iodide (TmI₃), ytterbium chloride (YbCl₃), ytterbium bromide (YbBr₃), and ytterbium iodide (YbI₃), and two or more of the metal triflate or rare-earth metal halide may be used.

[C-3] The composition according to [C-1] or [C-2], wherein the metal triflate or rare-earth metal halide is selected from the group consisting of Sn(OTf)₂, Er(OTf)₃, Tm(OTf)₃, Y(OTf)₃, Yb(OTf)₃, ErCl₃, ErBr₃, and ErI₃, and two or more of the metal triflate or rare-earth metal halide may be used.

[C-4] The composition according to any one of [C-1] to [C-3], wherein the metal triflate is selected from the group consisting of Sn(OTf)₂, Er(OTf)₃, Tm(OTf)₃, and Y(OTf)₃, and two or more of the metal triflate may be used.

[C-5] The composition according to any one of [C-1] to [C-4], wherein the base is selected from the group consisting of organic bases having pKa of conjugate acid in water of 1 to 14, and two or more of the base may be used.

[C-6] The composition according to any one of [C-1] to [C-5], wherein the base is selected from the group consisting of organic bases having pKa of conjugate acid in water of 2 to 12, 3 to 11, 3 to 10, 3 to 9, 3 to 8, or 3.5 to 7.5, and two or more of the base may be used.

[C-7] The composition according to any one of [C-1] to [C-6], wherein the base is selected from the group consisting of bases represented by formula (B1) or (B2): 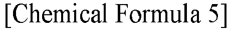 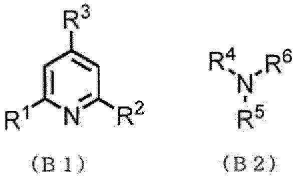
wherein R¹, R², and R³ are each independently selected from a hydrogen atom, C₁₋₆ alkyl, or C₆₋₁₀ aryl;
R⁴ and R⁵ together with a nitrogen atom to which they are attached form a 5- to 7-membered saturated heterocycle, the heterocycle optionally containing O, S, or N, or R⁴ and R⁵ are each independently C₁₋₆ alkyl, C₆₋₁₀ aryl, or tri(C₁₋₆ alkyl)silyl; and
R⁶ is a hydrogen atom or C₁₋₆ alkyl, and
two or more of the base may be used.

[C-8] The composition according to [C-7], wherein the R¹ and R² are C₁₋₆ alkyl, and R³ is a hydrogen atom or C₁₋₆ alkyl;
R⁴ and R⁵ together with a nitrogen atom to which they are attached form a 5- to 7-membered saturated heterocycle, the heterocycle optionally containing O, or R⁴ and R⁵ are each independently C₁₋₆ alkyl or tri(C₁₋₆ alkyl)silyl; and
R⁶ is a hydrogen atom or C₁₋₆ alkyl.

[C-9] The composition according to any one of [C-1] to [C-8], wherein the base is selected from the group consisting of 2,6-lutidine, N,N-diisopropylethylamine, N-methylmorpholine, 2,6-di-tert-butylpyridine, and di(trimethylsilyl)amine, and two or more of the base may be used.

[C-10] The composition according to any one of [C-1] to [C-9], wherein the base is selected from the group consisting of 2,6-lutidine, N-methylmorpholine, and 2,6-di-tert-butylpyridine, and two or more of the base may be used.

[C-11] The composition according to any one of [C-1] to [C-10], wherein the deprotection reaction is performed in the presence of a solvent.

[C-12] The composition according to any one of [C-1] to [C-11], wherein the deprotection reaction is performed in the presence of a solvent, and the solvent is selected from the group consisting of an ether-based solvent, a halogen-based solvent, a nitrile-based solvent, an aromatic hydrocarbon-based solvent, and an ester-based solvent, and two or more of the solvent may be used.

[C-13] The composition according to [C-12], wherein the ether-based solvent is selected from the group consisting of tetrahydrofuran, 2-methyltetrahydrofuran, 4-methyltetrahydropyran, 1,2-dimethoxyethane, diethyl ether, and 1,4-dioxane.

[C-14] The composition according to [C-12], wherein the halogen-based solvent is selected from the group consisting of dichloromethane, 1,2-dichloroethane, and chloroform.

[C-15] The composition according to [C-12], wherein the nitrile-based solvent is selected from the group consisting of acetonitrile and propionitrile.

[C-16] The composition according to [C-12], wherein the aromatic hydrocarbon-based solvent is selected from the group consisting of benzene, toluene, and xylene.

[C-17] The composition according to any one of [C-1] to [C-16], wherein a molar ratio of the metal triflate or rare-earth metal halide and the base used is metal triflate or rare-earth metal halide/base = 10/1 to 1/20.

[C-18] The composition according to any one of [C-1] to [C-17], wherein a molar ratio of the metal triflate or rare-earth metal halide and the base used is metal triflate or rare-earth metal halide/base = 5/1 to 1/10, 2/1 to 1/5, 1/1 to 1/5, or 1/1 to 1/2.

[C-19] The composition according to any one of [C-1] to [C-18], wherein a molar ratio of the metal triflate or rare-earth metal halide used to the compound having a Boc group on a nitrogen atom is 100 to 0.1 equivalents.

[C-20] The composition according to any one of [C-1] to [C-19], wherein a molar ratio of the metal triflate or rare-earth metal halide used to the compound having a Boc group on a nitrogen atom is 70 to 0.2 equivalents, 50 to 0.5 equivalents, 30 to 1 equivalents, or 20 to 1 equivalents.

[C-21] The composition according to any one of [C-1] to [C-20], wherein the deprotection reaction is performed at a reaction temperature of 0°C to 120°C.

[C-22] The composition according to any one of [C-1] to [C-21], wherein the deprotection reaction is performed at a reaction temperature of 10°C to 100°C, 15°C to 100°C, 20°C to 90°C, 25°C to 80°C, 30°C to 80°C, 35°C to 80°C, or 40°C to 80°C.

[C-23] The composition according to any one of [C-1] to [C-22], wherein the compound having a Boc group on a nitrogen atom is a resin for solid-phase synthesis to which the compound having a Boc group is attached via a linker.

[C-24] The composition according to any one of [C-1] to [C-23], wherein the compound having a Boc group on a nitrogen atom is a compound represented by formula (A): 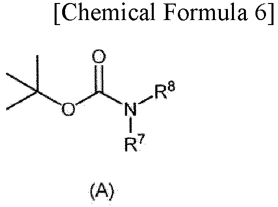 wherein R⁷ and R⁸ together with a nitrogen atom to which they are attached form a 5- to 7-membered saturated heterocycle, where the heterocycle is optionally substituted with an arbitrary substituent, or R⁷ and R⁸ are each independently a hydrogen atom, C₁₋₆ alkyl, C₂₋₆ alkenyl, C₂₋₆ alkynyl, C₃₋₈ cycloalkyl, C₇₋₁₄ aralkyl, C₆₋₁₀ aryl, or 5- to 10-membered heteroaryl containing one or more ring heteroatoms independently selected from O, N, and S, each of which is optionally substituted with one or more arbitrary substituents, or
wherein the compound having a Boc group on a nitrogen atom is a resin for solid-phase synthesis to which the compound represented by formula (A) is attached via a linker.

[C-25] The composition according to [C-24], wherein the R⁷ and R⁸ together with a nitrogen atom to which they are attached form a 5- to 7-membered saturated heterocycle, and the heterocycle is optionally substituted with one or more substituents independently selected from the group consisting of a halogen atom, cyano, C₁₋₆ alkyl, C₁₋₆ alkoxy, (C₁₋₆ alkoxy)carbonyl, (C₁₋₆ alkoxy)carbonylamino, (C₁₋₆ alkyl)carbonylamino, (C₆₋₁₀ aryl)carbonylamino, 5- to 10-membered heteroarylcarbonylamino containing one or more ring heteroatoms independently selected from O, N and S, di(C₁₋₆ alkyl)amino, 4- to 8-membered cyclic amino, aminocarbonyl, (C₁₋₆ alkyl)aminocarbonyl, di(C₁₋₆ alkyl)aminocarbonyl, and 4- to 8-membered cyclic aminocarbonyl.

[C-26] The composition according to any one of [C-1] to [C-25], wherein the compound having a Boc group on a nitrogen atom comprises a structure capable of being decomposed by treatment under an acidic condition, the structure being other than a Boc group.

[C-27] The composition according to [C-26], wherein the structure capable of being decomposed by treatment under an acidic condition is selected from the group consisting of an alkoxybenzyl ether structure, a trialkoxybenzylaminocarbonyl structure, a dialkoxyphenyl-alkoxyphenylmethylaminocarbonyl structure, and an alkoxyxanthen-9-ylaminocarbonyl structure.

[C-28] The composition according to [C-26] or [C-27], wherein the structure capable of being decomposed by treatment under an acidic condition is an alkoxybenzyl ether structure.

[C-29] The composition according to any one of [C-23] to [C-28], wherein the linker comprises a structure selected from the group consisting of an alkoxybenzyl ether structure, a trialkoxybenzylaminocarbonyl structure, a dialkoxyphenyl-alkoxyphenylmethylaminocarbonyl structure, and an alkoxyxanthen-9-ylaminocarbonyl structure as a structure capable of being decomposed by treatment under an acidic condition.

[C-30] The composition according to any one of [C-23] to [C-28], wherein the linker comprises an alkoxybenzyl ether structure as a structure capable of being decomposed by treatment under an acidic condition.

[C-31] The composition according to any one of [C-23] to [C-30], wherein the resin for solid-phase synthesis is selected from the group consisting of a Carboxylic resin, a Wang resin, a PAL AM resin, a Rink amide resin, and a Sieber amide resin.

[C-32] The composition according to any one of [C-23] to [C-30], wherein the resin for solid-phase synthesis is a Carboxylic resin.

[C-33] The composition according to any one of [C-26] to [C-32], wherein the resin for solid-phase synthesis is selected from the group consisting of a Carboxylic resin, a Wang resin, a PAL AM resin, a Rink amide resin, and a Sieber amide resin, and the linker comprises a structure selected from the group consisting of an alkoxybenzyl ether structure, a trialkoxybenzylaminocarbonyl structure, a dialkoxyphenyl-alkoxyphenylmethylaminocarbonyl structure, and an alkoxyxanthen-9-ylaminocarbonyl structure as a structure capable of being decomposed by treatment under an acidic condition.

[C-34] The composition according to any one of [C-26] to [C-32], wherein the resin for solid-phase synthesis is a Carboxylic resin, and the linker comprises an alkoxybenzyl ether structure as the structure capable of being decomposed by treatment under an acidic condition.

[C-35] The composition according to [C-26], wherein the treatment under an acidic condition is treating in dichloromethane, 1,2-dichloroethane, chloroform, toluene, acetonitrile, tetrahydrofuran, 1,4-dioxane, 1,2-dimethoxyethane, diethyl ether, acetone, ethyl acetate, N,N-dimethylformamide, methanol, ethanol, isopropanol, 1-butanol, 2,2,2-trifluoroethanol or water containing 10 v/v% trifluoroacetic acid.

[C-36] The composition according to any one of [C-1] to [C-35], wherein a mixture containing two or more different compounds having a Boc group on a nitrogen atom is subjected to the deprotection reaction.

[C-37] The composition according to any one of [C-26] to [C-36], wherein the linker comprises the structure capable of being decomposed by treatment under an acidic condition.

[C-38] The composition according to [C-12], wherein the ester-based solvent is selected from the group consisting of ethyl acetate, isopropyl acetate, and butyl acetate.

[C-39] The composition according to any one of [C-1] to [C-38], for use in the method according to any one of [A-1] to [A-46], [B-1] to [B-46], [E-1] to [E-40], and [F-1] to [F-38].

[D-1] Use of a trifluoromethanesulfonic acid metal salt (metal triflate: M(OTf)ₙ) or rare-earth metal halide (MXₙ) in a deprotection reaction of a tert-butoxycarbonyl (Boc) group in a compound having a Boc group on a nitrogen atom, wherein the deprotection reaction is performed in the presence of a base.

[D-2] The use according to [D-1], wherein the metal triflate or rare-earth metal halide is selected from the group consisting of tin triflate (Sn(OTf)₂), calcium triflate (Ca(OTf)₂), barium triflate (Ba(OTf)₂), zinc triflate (Zn(OTf)₂), aluminum triflate (Al(OTf)₃), gallium triflate (Ga(OTf)₃), indium triflate (In(OTf)₃), bismuth triflate (Bi(OTf)₃), lithium triflate (Li(OTf)), scandium triflate (Sc(OTf)₃), yttrium triflate (Y(OTf)₃), lanthanum triflate (La(OTf)₃), cerium triflate (Ce(OTf)₃, Ce(OTf)₄), praseodymium triflate (Pr(OTf)₃), neodymium triflate (Nd(OTf)₃), samarium triflate (Sm(OTf)₃), europium triflate (Eu(OTf)₃), gadolinium triflate (Gd(OTf)₃), terbium triflate (Tb(OTf)₃), dysprosium triflate (Dy(OTf)₃), holmium triflate (Ho(OTf)₃), erbium triflate (Er(OTf)₃), thulium triflate (Tm(OTf)₃), ytterbium triflate (Yb(OTf)₃), lutetium triflate (Lu(OTf)₃), hafnium triflate (Hf(OTf)₄), erbium chloride (ErCl₃), erbium bromide (ErBr₃), erbium iodide (ErI₃), thulium chloride (TmCl₃), thulium bromide (TmBr₃), thulium iodide (TmI₃), ytterbium chloride (YbCl₃), ytterbium bromide (YbBr₃), and ytterbium iodide (YbI₃), and two or more of the metal triflate or rare-earth metal halide may be used.

[D-3] The use according to [D-1] or [D-2], wherein the metal triflate or rare-earth metal halide is selected from the group consisting of Sn(OTf)₂, Er(OTf)₃, Tm(OTf)₃, Y(OTf)₃, Yb(OTf)₃, ErCl₃, ErBr₃, and ErI₃, and two or more of the metal triflate or rare-earth metal halide may be used.

[D-4] The use according to any one of [D-1] to [D-3], wherein the metal triflate is selected from the group consisting of Sn(OTf)₂, Er(OTf)₃, Tm(OTf)₃, and Y(OTf)₃, and two or more of the metal triflate may be used.

[D-5] The use according to any one of [D-1] to [D-4], wherein the base is selected from the group consisting of organic bases having pKa of conjugate acid in water of 1 to 14, and two or more of the base may be used.

[D-6] The use according to any one of [D-1] to [D-5], wherein the base is selected from the group consisting of organic bases having pKa of conjugate acid in water of 2 to 12, 3 to 11, 3 to 10, 3 to 9, 3 to 8, or 3.5 to 7.5, and two or more of the base may be used.

[D-7] The use according to any one of [D-1] to [D-6], wherein the base is selected from the group consisting of bases represented by formula (B1) or (B2): 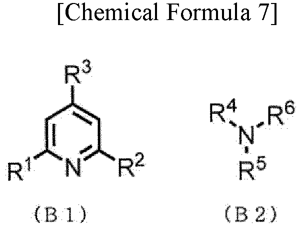
wherein R¹, R², and R³ are each independently selected from a hydrogen atom, C₁₋₆ alkyl, or C₆₋₁₀ aryl;
R⁴ and R⁵ together with a nitrogen atom to which they are attached form a 5- to 7-membered saturated heterocycle, the heterocycle optionally containing O, S, or N, or R⁴ and R⁵ are each independently C₁₋₆ alkyl, C₆₋₁₀ aryl, or tri(C₁₋₆ alkyl)silyl; and
R⁶ is a hydrogen atom or C₁₋₆ alkyl, and
two or more of the base may be used.

[D-8] The use according to [D-7], wherein the R¹ and R² are C₁₋₆ alkyl, and R³ is a hydrogen atom or C₁₋₆ alkyl;
R⁴ and R⁵ together with a nitrogen atom to which they are attached form a 5- to 7-membered saturated heterocycle, the heterocycle optionally containing O, or R⁴ and R⁵ are each independently C₁₋₆ alkyl or tri(C₁₋₆ alkyl)silyl; and
R⁶ is a hydrogen atom or C₁₋₆ alkyl.

[D-9] The use according to any one of [D-1] to [D-8], wherein the base is selected from the group consisting of 2,6-lutidine, N,N-diisopropylethylamine, N-methylmorpholine, 2,6-di-tert-butylpyridine, and di(trimethylsilyl)amine, and two or more of the base may be used.

[D-10] The use according to any one of [D-1] to [D-9], wherein the base is selected from the group consisting of 2,6-lutidine, N-methylmorpholine, and 2,6-di-tert-butylpyridine, and two or more of the base may be used.

[D-11] The use according to any one of [D-1] to [D-10], wherein the deprotection reaction is performed in the presence of a solvent.

[D-12] The use according to any one of [D-1] to [D-11], wherein the deprotection reaction is performed in the presence of a solvent, and the solvent is selected from the group consisting of an ether-based solvent, a halogen-based solvent, a nitrile-based solvent, an aromatic hydrocarbon-based solvent, and an ester-based solvent, and two or more of the solvent may be used.

[D-13] The use according to [D-12], wherein the ether-based solvent is selected from the group consisting of tetrahydrofuran, 2-methyltetrahydrofuran, 4-methyltetrahydropyran, 1,2-dimethoxyethane, diethyl ether, and 1,4-dioxane.

[D-14] The use according to [D-12], wherein the halogen-based solvent is selected from the group consisting of dichloromethane, 1,2-dichloroethane, and chloroform.

[D-15] The use according to [D-12], wherein the nitrile-based solvent is selected from the group consisting of acetonitrile and propionitrile.

[D-16] The use according to [D-12], wherein the aromatic hydrocarbon-based solvent is selected from the group consisting of benzene, toluene, and xylene.

[D-17] The use according to any one of [D-1] to [D-16], wherein a molar ratio of the metal triflate or rare-earth metal halide and the base used is metal triflate or rare-earth metal halide/base = 10/1 to 1/20.

[D-18] The use according to any one of [D-1] to [D-17], wherein a molar ratio of the metal triflate or rare-earth metal halide and the base used is metal triflate or rare-earth metal halide/base = 5/1 to 1/10, 2/1 to 1/5, 1/1 to 1/5, or 1/1 to 1/2.

[D-19] The use according to any one of [D-1] to [D-18], wherein a molar ratio of the metal triflate or rare-earth metal halide used to the compound having a Boc group on a nitrogen atom is 100 to 0.1 equivalents.

[D-20] The use according to any one of [D-1] to [D-19], wherein a molar ratio of the metal triflate or rare-earth metal halide used to the compound having a Boc group on a nitrogen atom is 70 to 0.2 equivalents, 50 to 0.5 equivalents, 30 to 1 equivalents, or 20 to 1 equivalents.

[D-21] The use according to any one of [D-1] to [D-20], wherein the deprotection reaction is performed at a reaction temperature of 0°C to 120°C.

[D-22] The use according to any one of [D-1] to [D-21], wherein the deprotection reaction is performed at a reaction temperature of 10°C to 100°C, 15°C to 100°C, 20°C to 90°C, 25°C to 80°C, 30°C to 80°C, 35°C to 80°C, or 40°C to 80°C.

[D-23] The use according to any one of [D-1] to [D-22], wherein the compound having a Boc group on a nitrogen atom is a resin for solid-phase synthesis to which the compound having a Boc group is attached via a linker.

[D-24] The use according to any one of [D-1] to [D-23], wherein the compound having a Boc group on a nitrogen atom is a compound represented by formula (A): 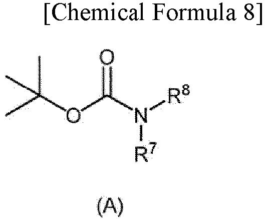 wherein R⁷ and R⁸ together with a nitrogen atom to which they are attached form a 5- to 7-membered saturated heterocycle, where the heterocycle is optionally substituted with an arbitrary substituent, or R⁷ and R⁸ are each independently a hydrogen atom, C₁₋₆ alkyl, C₂₋₆ alkenyl, C₂₋₆ alkynyl, C₃₋₈ cycloalkyl, C₇₋₁₄ aralkyl, C₆₋₁₀ aryl, or 5- to 10-membered heteroaryl containing one or more ring heteroatoms independently selected from O, N, and S, each of which is optionally substituted with one or more arbitrary substituents, or
wherein the compound having a Boc group on a nitrogen atom is a resin for solid-phase synthesis to which the compound represented by formula (A) is attached via a linker.

[D-25] The use according to [D-24], wherein the R⁷ and R⁸ together with a nitrogen atom to which they are attached form a 5- to 7-membered saturated heterocycle, and the heterocycle is optionally substituted with one or more substituents independently selected from the group consisting of a halogen atom, cyano, C₁₋₆ alkyl, C₁₋₆ alkoxy, (C₁₋₆ alkoxy)carbonyl, (C₁₋₆ alkoxy)carbonylamino, (C₁₋₆ alkyl)carbonylamino, (C₆₋₁₀ aryl)carbonylamino, 5- to 10-membered heteroarylcarbonylamino containing one or more ring heteroatoms independently selected from O, N and S, di(C₁₋₆ alkyl)amino, 4- to 8-membered cyclic amino, aminocarbonyl, (C₁₋₆ alkyl)aminocarbonyl, di(C₁₋₆ alkyl)aminocarbonyl, and 4- to 8-membered cyclic aminocarbonyl.

[D-26] The use according to any one of [D-1] to [D-25], wherein the compound having a Boc group on a nitrogen atom comprises a structure capable of being decomposed by treatment under an acidic condition, the structure being other than a Boc group.

[D-27] The use according to [D-26], wherein the structure capable of being decomposed by treatment under an acidic condition is selected from the group consisting of an alkoxybenzyl ether structure, a trialkoxybenzylaminocarbonyl structure, a dialkoxyphenyl-alkoxyphenylmethylaminocarbonyl structure, and an alkoxyxanthen-9-ylaminocarbonyl structure.

[D-28] The use according to [D-26] or [D-27], wherein the structure capable of being decomposed by treatment under an acidic condition is an alkoxybenzyl ether structure.

[D-29] The use according to any one of [D-23] to [D-28], wherein the linker comprises a structure selected from the group consisting of an alkoxybenzyl ether structure, a trialkoxybenzylaminocarbonyl structure, a dialkoxyphenyl-alkoxyphenylmethylaminocarbonyl structure, and an alkoxyxanthen-9-ylaminocarbonyl structure as a structure capable of being decomposed by treatment under an acidic condition.

[D-30] The use according to any one of [D-23] to [D-28], wherein the linker comprises an alkoxybenzyl ether structure as a structure capable of being decomposed by treatment under an acidic condition.

[D-31] The use according to any one of [D-23] to [D-30], wherein the resin for solid-phase synthesis is selected from the group consisting of a Carboxylic resin, a Wang resin, a PAL AM resin, a Rink amide resin, and a Sieber amide resin.

[D-32] The use according to any one of [D-23] to [D-30], wherein the resin for solid-phase synthesis is a Carboxylic resin.

[D-33] The use according to any one of [D-23] to [D-32], wherein the resin for solid-phase synthesis is selected from the group consisting of a Carboxylic resin, a Wang resin, a PAL AM resin, a Rink amide resin, and a Sieber amide resin, and the linker comprises a structure selected from the group consisting of an alkoxybenzyl ether structure, a trialkoxybenzylaminocarbonyl structure, a dialkoxyphenyl-alkoxyphenylmethylaminocarbonyl structure, and an alkoxyxanthen-9-ylaminocarbonyl structure as a structure capable of being decomposed by treatment under an acidic condition.

[D-34] The use according to any one of [D-23] to [D-33], wherein the resin for solid-phase synthesis is a Carboxylic resin, and the linker comprises an alkoxybenzyl ether structure as the structure capable of being decomposed by treatment under an acidic condition.

[D-35] The use according to [D-26], wherein the treatment under an acidic condition is treating in dichloromethane, 1,2-dichloroethane, chloroform, toluene, acetonitrile, tetrahydrofuran, 1,4-dioxane, 1,2-dimethoxyethane, diethyl ether, acetone, ethyl acetate, N,N-dimethylformamide, methanol, ethanol, isopropanol, 1-butanol, 2,2,2-trifluoroethanol or water containing 10 v/v% trifluoroacetic acid.

[D-36] The use according to any one of [D-1] to [D-35], wherein a mixture containing two or more different compounds having a Boc group on a nitrogen atom is subjected to the deprotection reaction.

[D-37] The use according to [D-12], wherein the ester-based solvent is selected from the group consisting of ethyl acetate, isopropyl acetate, and butyl acetate.

[D-38] The use according to any one of [D-26] to [D-37], wherein the linker comprises the structure capable of being decomposed by treatment under an acidic condition.

[D-39] The use according to any one of [D-1] to [D-38] in the method according to any one of [A-1] to [A-46], [B-1] to [B-46], [E-1] to [E-40], and [F-1] to [F-38].

[E-1] A method for improving a yield in a deprotection reaction of a tert-butoxycarbonyl (Boc) group in a compound having a Boc group on a nitrogen atom, comprising treating the compound with a trifluoromethanesulfonic acid metal salt (metal triflate: M(OTf)ₙ) or rare-earth metal halide (MXₙ) in the presence of a base to obtain a compound in which the Boc group is converted to H-.

[E-2] The method according to [E-1], wherein the metal triflate or rare-earth metal halide is selected from the group consisting of tin triflate (Sn(OTf)₂), calcium triflate (Ca(OTf)₂), barium triflate (Ba(OTf)₂), zinc triflate (Zn(OTf)₂), aluminum triflate (Al(OTf)₃), gallium triflate (Ga(OTf)₃), indium triflate (In(OTf)₃), bismuth triflate (Bi(OTf)₃), lithium triflate (Li(OTf)), scandium triflate (Sc(OTf)₃), yttrium triflate (Y(OTf)₃), lanthanum triflate (La(OTf)₃), cerium triflate (Ce(OTf)₃, Ce(OTf)₄), praseodymium triflate (Pr(OTf)₃), neodymium triflate (Nd(OTf)₃), samarium triflate (Sm(OTf)₃), europium triflate (Eu(OTf)₃), gadolinium triflate (Gd(OTf)₃), terbium triflate (Tb(OTf)₃), dysprosium triflate (Dy(OTf)₃), holmium triflate (Ho(OTf)₃), erbium triflate (Er(OTf)₃), thulium triflate (Tm(OTf)₃), ytterbium triflate (Yb(OTf)₃), lutetium triflate (Lu(OTf)₃), hafnium triflate (Hf(OTf)₄), erbium chloride (ErCl₃), erbium bromide (ErBr₃), erbium iodide (ErI₃), thulium chloride (TmCl₃), thulium bromide (TmBr₃), thulium iodide (TmI₃), ytterbium chloride (YbCl₃), ytterbium bromide (YbBr₃), and ytterbium iodide (YbI₃), and two or more of the metal triflate or rare-earth metal halide may be used.

[E-3] The method according to [E-1] or [E-2], wherein the metal triflate or rare-earth metal halide is selected from the group consisting of Sn(OTf)₂, Er(OTf)₃, Tm(OTf)₃, Y(OTf)₃, Yb(OTf)₃, ErCl₃, ErBr₃, and ErI₃, and two or more of the metal triflate or rare-earth metal halide may be used.

[E-4] The method according to any one of [E-1] to [E-3], wherein the metal triflate is selected from the group consisting of Sn(OTf)₂, Er(OTf)₃, Tm(OTf)₃, and Y(OTf)₃, and two or more of the metal triflate may be used.

[E-5] The method according to any one of [E-1] to [E-4], wherein the base is selected from the group consisting of organic bases having pKa of conjugate acid in water of 1 to 14, and two or more of the base may be used.

[E-6] The method according to any one of [E-1] to [E-5], wherein the base is selected from the group consisting of organic bases having pKa of conjugate acid in water of 2 to 12, 3 to 11, 3 to 10, 3 to 9, 3 to 8, or 3.5 to 7.5, and two or more of the base may be used.

[E-7] The method according to any one of [E-1] to [E-6], wherein the base is selected from the group consisting of bases represented by formula (B1) or (B2): 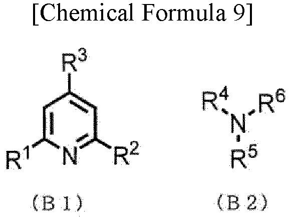
wherein R¹, R², and R³ are each independently selected from a hydrogen atom, C₁₋₆ alkyl, or C₆₋₁₀ aryl;
R⁴ and R⁵ together with a nitrogen atom to which they are attached form a 5- to 7-membered saturated heterocycle, the heterocycle optionally containing O, S, or N, or R⁴ and R⁵ are each independently C₁₋₆ alkyl, C₆₋₁₀ aryl, or tri(C₁₋₆ alkyl)silyl; and
R⁶ is a hydrogen atom or C₁₋₆ alkyl, and
two or more of the base may be used.

[E-8] The method according to [E-7], wherein the R¹ and R² are C₁₋₆ alkyl, and R³ is a hydrogen atom or C₁₋₆ alkyl;
R⁴ and R⁵ together with a nitrogen atom to which they are attached form a 5- to 7-membered saturated heterocycle, the heterocycle optionally containing O, or R⁴ and R⁵ are each independently C₁₋₆ alkyl or tri(C₁₋₆ alkyl)silyl; and
R⁶ is a hydrogen atom or C₁₋₆ alkyl.

[E-9] The method according to any one of [E-1] to [E-8], wherein the base is selected from the group consisting of 2,6-lutidine, N,N-diisopropylethylamine, N-methylmorpholine, 2,6-di-tert-butylpyridine, and di(trimethylsilyl)amine, and two or more of the base may be used.

[E-10] The method according to any one of [E-1] to [E-9], wherein the base is selected from the group consisting of 2,6-lutidine, N-methylmorpholine, and 2,6-di-tert-butylpyridine, and two or more of the base may be used.

[E-11] The method according to any one of [E-1] to [E-10], wherein the treating is performed in the presence of a solvent.

[E-12] The method according to any one of [E-1] to [E-11], wherein the treating is performed in the presence of a solvent, and the solvent is selected from the group consisting of an ether-based solvent, a halogen-based solvent, a nitrile-based solvent, an aromatic hydrocarbon-based solvent, and an ester-based solvent, and two or more of the solvent may be used.

[E-13] The method according to [E-12], wherein the ether-based solvent is selected from the group consisting of tetrahydrofuran, 2-methyltetrahydrofuran, 4-methyltetrahydropyran, 1,2-dimethoxyethane, diethyl ether, and 1,4-dioxane.

[E-14] The method according to [E-12], wherein the halogen-based solvent is selected from the group consisting of dichloromethane, 1,2-dichloroethane, and chloroform.

[E-15] The method according to [E-12], wherein the nitrile-based solvent is selected from the group consisting of acetonitrile and propionitrile.

[E-16] The method according to [E-12], wherein the aromatic hydrocarbon-based solvent is selected from the group consisting of benzene, toluene, and xylene.

[E-17] The method according to any one of [E-1] to [E-16], wherein a molar ratio of the metal triflate or rare-earth metal halide and the base used is metal triflate or rare-earth metal halide/base = 10/1 to 1/20.

[E-18] The method according to any one of [E-1] to [E-17], wherein a molar ratio of the metal triflate or rare-earth metal halide and the base used is metal triflate or rare-earth metal halide/base = 5/1 to 1/10, 2/1 to 1/5, 1/1 to 1/5, or 1/1 to 1/2.

[E-19] The method according to any one of [E-1] to [E-18], wherein a molar ratio of the metal triflate or rare-earth metal halide to the compound having a Boc group on a nitrogen atom is 100 to 0.1 equivalents.

[E-20] The method according to any one of [E-1] to [E-19], wherein a molar ratio of the metal triflate or rare-earth metal halide used to the compound having a Boc group on a nitrogen atom is 70 to 0.2 equivalents, 50 to 0.5 equivalents, 30 to 1 equivalents, or 20 to 1 equivalents.

[E-21] The method according to any one of [E-1] to [E-20], wherein the treating is performed at a reaction temperature of 0°C to 120°C.

[E-22] The method according to any one of [E-1] to [E-21], wherein the treating is performed at a reaction temperature of 10°C to 100°C, 15°C to 100°C, 20°C to 90°C, 25°C to 80°C, 30°C to 80°C, 35°C to 80°C, or 40°C to 80°C.

[E-23] The method according to any one of [E-1] to [E-22], wherein the compound having a Boc group on a nitrogen atom is a resin for solid-phase synthesis to which the compound having a Boc group is attached via a linker.

[E-24] The method according to any one of [E-1] to [E-23], wherein the compound having a Boc group on a nitrogen atom is a compound represented by formula (A): 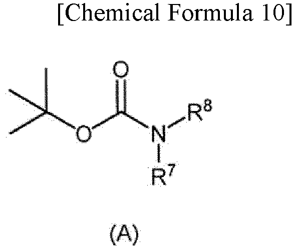 wherein R⁷ and R⁸ together with a nitrogen atom to which they are attached form a 5- to 7-membered saturated heterocycle, where the heterocycle is optionally substituted with an arbitrary substituent, or R⁷ and R⁸ are each independently a hydrogen atom, C₁₋₆ alkyl, C₂₋₆ alkenyl, C₂₋₆ alkynyl, C₃₋₈ cycloalkyl, C₇₋₁₄ aralkyl, C₆₋₁₀ aryl, or 5- to 10-membered heteroaryl containing one or more ring heteroatoms independently selected from O, N, and S, each of which is optionally substituted with one or more arbitrary substituents, or
wherein the compound having a Boc group on a nitrogen atom is a resin for solid-phase synthesis to which the compound represented by formula (A) is attached via a linker.

[E-25] The method according to [E-24], wherein the R⁷ and R⁸ together with a nitrogen atom to which they are attached form a 5- to 7-membered saturated heterocycle, and the heterocycle is optionally substituted with one or more substituents independently selected from the group consisting of a halogen atom, cyano, C₁₋₆ alkyl, C₁₋₆ alkoxy, (C₁₋₆ alkoxy)carbonyl, (C₁₋₆ alkoxy)carbonylamino, (C₁₋₆ alkyl)carbonylamino, (C₆₋₁₀ aryl)carbonylamino, 5- to 10-membered heteroarylcarbonylamino containing one or more ring heteroatoms independently selected from O, N and S, di(C₁₋₆ alkyl)amino, 4- to 8-membered cyclic amino, aminocarbonyl, (C₁₋₆ alkyl)aminocarbonyl, di(C₁₋₆ alkyl)aminocarbonyl, and 4- to 8-membered cyclic aminocarbonyl.

[E-26] The method according to any one of [E-1] to [E-25], wherein the compound having a Boc group on a nitrogen atom comprises a structure capable of being decomposed by treatment under an acidic condition, the structure being other than a Boc group.

[E-27] The method according to [E-26], wherein the structure capable of being decomposed by treatment under an acidic condition is selected from the group consisting of an alkoxybenzyl ether structure, a trialkoxybenzylaminocarbonyl structure, a dialkoxyphenyl-alkoxyphenylmethylaminocarbonyl structure, and an alkoxyxanthen-9-ylaminocarbonyl structure.

[E-28] The method according to [E-26] or [E-27], wherein the structure capable of being decomposed by treatment under an acidic condition is an alkoxybenzyl ether structure.

[E-29] The method according to any one of [E-23] to [E-28], wherein the linker comprises a structure selected from the group consisting of an alkoxybenzyl ether structure, a trialkoxybenzylaminocarbonyl structure, a dialkoxyphenyl-alkoxyphenylmethylaminocarbonyl structure, and an alkoxyxanthen-9-ylaminocarbonyl structure as the structure capable of being decomposed by treatment under an acidic condition.

[E-30] The method according to any one of [E-23] to [E-28], wherein the linker comprises an alkoxybenzyl ether structure as the structure capable of being decomposed by treatment under an acidic condition.

[E-31] The method according to any one of [E-23] to [E-30], wherein the resin for solid-phase synthesis is selected from the group consisting of a Carboxylic resin, a Wang resin, a PAL AM resin, a Rink amide resin, and a Sieber amide resin.

[E-32] The method according to any one of [E-23] to [E-30], wherein the resin for solid-phase synthesis is a Carboxylic resin.

[E-33] The method according to any one of [E-23] to [E-32], wherein the resin for solid-phase synthesis is selected from the group consisting of a Carboxylic resin, a Wang resin, a PAL AM resin, a Rink amide resin, and a Sieber amide resin, and the linker comprises a structure selected from the group consisting of an alkoxybenzyl ether structure, a trialkoxybenzylaminocarbonyl structure, a dialkoxyphenyl-alkoxyphenylmethylaminocarbonyl structure, and an alkoxyxanthen-9-ylaminocarbonyl structure as a structure capable of being decomposed by treatment under an acidic condition.

[E-34] The method according to any one of [E-23] to [E-33], wherein the resin for solid-phase synthesis is a Carboxylic resin, and the linker comprises an alkoxybenzyl ether structure as the structure capable of being decomposed by treatment under an acidic condition.

[E-35] The method according to [E-26], wherein the treatment underh an acidic condition is treating in dichloromethane, 1,2-dichloroethane, chloroform, toluene, acetonitrile, tetrahydrofuran, 1,4-dioxane, 1,2-dimethoxyethane, diethyl ether, acetone, ethyl acetate, N,N-dimethylformamide, methanol, ethanol, isopropanol, 1-butanol, 2,2,2-trifluoroethanol or water containing 10 v/v% trifluoroacetic acid.

[E-36] The method according to any one of [E-1] to [E-35], wherein the method comprises treating a mixture containing two or more different compounds having a Boc group on a nitrogen atom.

[E-37] The method according to any one of [E-1] to [E-36], wherein the yield is improved compared to a reaction condition using trifluoroacetic acid.

[E-38] The method according to [E-37], wherein the reaction condition using trifluoroacetic acid is using 10 v/v% trifluoroacetic acid in dichloromethane, 1,2-dichloroethane, chloroform, toluene, acetonitrile, tetrahydrofuran, 1,4-dioxane, 1,2-dimethoxyethane, diethyl ether, acetone, ethyl acetate, N,N-dimethylformamide, methanol, ethanol, isopropanol, 1-butanol, 2,2,2-trifluoroethanol, or water.

[E-39] The method according to [E-12], wherein the ester-based solvent is selected from the group consisting of ethyl acetate, isopropyl acetate, and butyl acetate.

[E-40] The method according to any one of [E-26] to [E-39], wherein the linker comprises the structure capable of being decomposed by treatment under an acidic condition.

[F-1] A method for suppressing a generation of a byproduct in a deprotection reaction of a tert-butoxycarbonyl (Boc) group in a compound having a Boc group on a nitrogen atom, comprising treating the compound with a trifluoromethanesulfonic acid metal salt (metal triflate: M(OTf)ₙ) or rare-earth metal halide (MXₙ) in the presence of a base to obtain a compound in which the Boc group is converted to H-.

[F-2] The method according to [F-1], wherein the metal triflate or rare-earth metal halide is selected from the group consisting of tin triflate (Sn(OTf)₂), calcium triflate (Ca(OTf)₂), barium triflate (Ba(OTf)₂), zinc triflate (Zn(OTf)₂), aluminum triflate (Al(OTf)₃), gallium triflate (Ga(OTf)₃), indium triflate (In(OTf)₃), bismuth triflate (Bi(OTf)₃), lithium triflate (Li(OTf)), scandium triflate (Sc(OTf)₃), yttrium triflate (Y(OTf)₃), lanthanum triflate (La(OTf)₃), cerium triflate (Ce(OTf)₃, Ce(OTf)₄), praseodymium triflate (Pr(OTf)₃), neodymium triflate (Nd(OTf)₃), samarium triflate (Sm(OTf)₃), europium triflate (Eu(OTf)₃), gadolinium triflate (Gd(OTf)₃), terbium triflate (Tb(OTf)₃), dysprosium triflate (Dy(OTf)₃), holmium triflate (Ho(OTf)₃), erbium triflate (Er(OTf)₃), thulium triflate (Tm(OTf)₃), ytterbium triflate (Yb(OTf)₃), lutetium triflate (Lu(OTf)₃), hafnium triflate (Hf(OTf)₄), erbium chloride (ErCl₃), erbium bromide (ErBr₃), erbium iodide (ErI₃), thulium chloride (TmCl₃), thulium bromide (TmBr₃), thulium iodide (TmI₃), ytterbium chloride (YbCl₃), ytterbium bromide (YbBr₃), and ytterbium iodide (YbI₃), and two or more of the metal triflate or rare-earth metal halide may be used.

[F-3] The method according to [F-1] or [F-2], wherein the metal triflate or rare-earth metal halide is selected from the group consisting of Sn(OTf)₂, Er(OTf)₃, Tm(OTf)₃, Y(OTf)₃, Yb(OTf)₃, ErCl₃, ErBr₃, and ErI₃, and two or more of the metal triflate or rare-earth metal halide may be used.

[F-4] The method according to any one of [F-1] to [F-3], wherein the metal triflate is selected from the group consisting of Sn(OTf)₂, Er(OTf)₃, Tm(OTf)₃, and Y(OTf)₃, and two or more of the metal triflate may be used.

[F-5] The method according to any one of [F-1] to [F-4], wherein the base is selected from the group consisting of organic bases having pKa of conjugate acid in water of 1 to 14, and two or more of the base may be used.

[F-6] The method according to any one of [F-1] to [F-5], wherein the base is selected from the group consisting of organic bases having pKa of conjugate acid in water of 2 to 12, 3 to 11, 3 to 10, 3 to 9, 3 to 8, or 3.5 to 7.5, and two or more of the base may be used.

[F-7] The method according to any one of [F-1] to [F-6], wherein the base is selected from the group consisting of bases represented by formula (B1) or (B2): 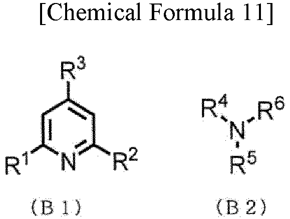
wherein R¹, R², and R³ are each independently selected from a hydrogen atom, C₁₋₆ alkyl, or C₆₋₁₀ aryl;
R⁴ and R⁵ together with a nitrogen atom to which they are attached form a 5- to 7-membered saturated heterocycle, the heterocycle optionally containing O, S, or N, or R⁴ and R⁵ are each independently C₁₋₆ alkyl, C₆₋₁₀ aryl, or tri(C₁₋₆ alkyl)silyl; and
R⁶ is a hydrogen atom or C₁₋₆ alkyl, and
two or more of the base may be used.

[F-8] The method according to [F-7], wherein the R¹ and R² are C₁₋₆ alkyl, and R³ is a hydrogen atom or C₁₋₆ alkyl;
R⁴ and R⁵ together with a nitrogen atom to which they are attached form a 5- to 7-membered saturated heterocycle, the heterocycle optionally containing O, or R⁴ and R⁵ are each independently C₁₋₆ alkyl or tri(C₁₋₆ alkyl)silyl; and
R⁶ is a hydrogen atom or C₁₋₆ alkyl.

[F-9] The method according to any one of [F-1] to [F-8], wherein the base is selected from the group consisting of 2,6-lutidine, N,N-diisopropylethylamine, N-methylmorpholine, 2,6-di-tert-butylpyridine, and di(trimethylsilyl)amine, and two or more of the base may be used.

[F-10] The method according to any one of [F-1] to [F-9], wherein the base is selected from the group consisting of 2,6-lutidine, N-methylmorpholine, and 2,6-di-tert-butylpyridine, and two or more of the base may be used.

[F-11] The method according to any one of [F-1] to [F-10], wherein the treating is performed in the presence of a solvent.

[F-12] The method according to any one of [F-1] to [F-11], wherein the treating is performed in the presence of a solvent, and the solvent is selected from the group consisting of an ether-based solvent, a halogen-based solvent, a nitrile-based solvent, an aromatic hydrocarbon-based solvent, and an ester-based solvent, and two or more of the solvent may be used.

[F-13] The method according to [F-12], wherein the ether-based solvent is selected from the group consisting of tetrahydrofuran, 2-methyltetrahydrofuran, 4-methyltetrahydropyran, 1,2-dimethoxyethane, diethyl ether, and 1,4-dioxane.

[F-14] The method according to [F-12], wherein the halogen-based solvent is selected from the group consisting of dichloromethane, 1,2-dichloroethane, and chloroform.

[F-15] The method according to [F-12], wherein the nitrile-based solvent is selected from the group consisting of acetonitrile and propionitrile.

[F-16] The method according to [F-12], wherein the aromatic hydrocarbon-based solvent is selected from the group consisting of benzene, toluene, and xylene.

[F-17] The method according to any one of [F-1] to [F-16], wherein a molar ratio of the metal triflate or rare-earth metal halide and the base used is metal triflate or rare-earth metal halide/base = 10/1 to 1/20.

[F-18] The method according to any one of [F-1] to [F-17], wherein a molar ratio of the metal triflate or rare-earth metal halide and the base used is metal triflate or rare-earth metal halide/base = 5/1 to 1/10, 2/1 to 1/5, 1/1 to 1/5, or 1/1 to 1/2.

[F-19] The method according to any one of [F-1] to [F-18], wherein a molar ratio of the metal triflate or rare-earth metal halide used to the compound having a Boc group on a nitrogen atom is 100 to 0.1 equivalents.

[F-20] The method according to any one of [F-1] to [F-19], wherein a molar ratio of the metal triflate or rare-earth metal halide used to the compound having a Boc group on a nitrogen atom is 70 to 0.2 equivalents, 50 to 0.5 equivalents, 30 to 1 equivalents, or 20 to 1 equivalents.

[F-21] The method according to any one of [F-1] to [F-20], wherein the treating is performed at a reaction temperature of 0°C to 120°C.

[F-22] The method according to any one of [F-1] to [F-21], wherein the treating is performed at a reaction temperature of 10°C to 100°C, 15°C to 100°C, 20°C to 90°C, 25°C to 80°C, 30°C to 80°C, 35°C to 80°C, or 40°C to 80°C.

[F-23] The method according to any one of [F-1] to [F-22], wherein the compound having a Boc group on a nitrogen atom is a resin for solid-phase synthesis to which the compound having a Boc group is attached via a linker.

[F-24] The method according to any one of [F-1] to [F-23], wherein the compound having a Boc group on a nitrogen atom is a compound represented by formula (A): 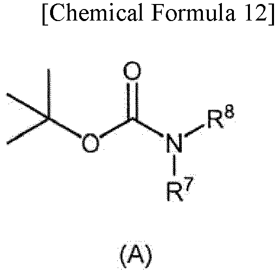 wherein R⁷ and R⁸ together with a nitrogen atom to which they are attached form a 5- to 7-membered saturated heterocycle, where the heterocycle is optionally substituted with an arbitrary substituent, or R⁷ and R⁸ are each independently a hydrogen atom, C₁₋₆ alkyl, C₂₋₆ alkenyl, C₂₋₆ alkynyl, C₃₋₈ cycloalkyl, C₇₋₁₄ aralkyl, C₆₋₁₀ aryl, or 5- to 10-membered heteroaryl containing one or more ring heteroatoms independently selected from O, N, and S, each of which is optionally substituted with one or more arbitrary substituents, or
wherein the compound having a Boc group on a nitrogen atom is a resin for solid-phase synthesis to which the compound represented by formula (A) is attached via a linker.

[F-25] The method according to [F-24], wherein the R⁷ and R⁸ together with a nitrogen atom to which they are attached form a 5- to 7-membered saturated heterocycle, and the heterocycle is optionally substituted with one or more substituents independently selected from the group consisting of a halogen atom, cyano, C₁₋₆ alkyl, C₁₋₆ alkoxy, (C₁₋₆ alkoxy)carbonyl, (C₁₋₆ alkoxy)carbonylamino, (C₁₋₆ alkyl)carbonylamino, (C₆₋₁₀ aryl)carbonylamino, 5- to 10-membered heteroarylcarbonylamino containing one or more ring heteroatoms independently selected from O, N and S, di(C₁₋₆ alkyl)amino, 4- to 8-membered cyclic amino, aminocarbonyl, (C₁₋₆ alkyl)aminocarbonyl, di(C₁₋₆ alkyl)aminocarbonyl, and 4- to 8-membered cyclic aminocarbonyl.

[F-26] The method according to any one of [F-1] to [F-25], wherein the compound having a Boc group on a nitrogen atom comprises a structure capable of being decomposed by treatment under an acidic condition, the structure being other than a Boc group.

[F-27] The method according to [F-26], wherein the structure capable of being decomposed by treatment under an acidic condition is selected from the group consisting of an alkoxybenzyl ether structure, a trialkoxybenzylaminocarbonyl structure, a dialkoxyphenyl-alkoxyphenylmethylaminocarbonyl structure, and an alkoxyxanthen-9-ylaminocarbonyl structure.

[F-28] The method according to [F-26] or [F-27], wherein the structure capable of being decomposed by treatment under an acidic condition is an alkoxybenzyl ether structure.

[F-29] The method according to any one of [F-23] to [F-28], wherein the linker comprises a structure selected from the group consisting of an alkoxybenzyl ether structure, a trialkoxybenzylaminocarbonyl structure, a dialkoxyphenyl-alkoxyphenylmethylaminocarbonyl structure, and an alkoxyxanthen-9-ylaminocarbonyl structure as a structure capable of being decomposed by treatment under an acidic condition.

[F-30] The method according to any one of [F-23] to [F-28], wherein the linker comprises an alkoxybenzyl ether structure as a structure capable of being decomposed by treatment under an acidic condition.

[F-31] The method according to any one of [F-23] to [F-30], wherein the resin for solid-phase synthesis is selected from the group consisting of a Carboxylic resin, a Wang resin, a PAL AM resin, a Rink amide resin, and a Sieber amide resin.

[F-32] The method according to any one of [F-23] to [F-30], wherein the resin for solid-phase synthesis is a Carboxylic resin.

[F-33] The method according to any one of [F-23] to [F-32], wherein the resin for solid-phase synthesis is selected from the group consisting of a Carboxylic resin, a Wang resin, a PAL AM resin, a Rink amide resin, and a Sieber amide resin, and the linker comprises a structure selected from the group consisting of an alkoxybenzyl ether structure, a trialkoxybenzylaminocarbonyl structure, a dialkoxyphenyl-alkoxyphenylmethylaminocarbonyl structure, and an alkoxyxanthen-9-ylaminocarbonyl structure as a structure capable of being decomposed by treatment under an acidic condition.

[F-34] The method according to any one of [F-23] to [F-33], wherein the resin for solid-phase synthesis is a Carboxylic resin, and the linker comprises an alkoxybenzyl ether structure as a structure capable of being decomposed by treatment under an acidic condition.

[F-35] The method according to [F-26], wherein the treatment under an acidic condition is treating in dichloromethane, 1,2-dichloroethane, chloroform, toluene, acetonitrile, tetrahydrofuran, 1,4-dioxane, 1,2-dimethoxyethane, diethyl ether, acetone, ethyl acetate, N,N-dimethylformamide, methanol, ethanol, isopropanol, 1-butanol, 2,2,2-trifluoroethanol or water containing 10 v/v% trifluoroacetic acid.

[F-36] The method according to [F-12], wherein the ester-based solvent is selected from the group consisting of ethyl acetate, isopropyl acetate, and butyl acetate.

[F-37] The method according to any one of [F-26] to [F-36], wherein the linker comprises the structure capable of being decomposed by treatment under an acidic condition.

[F-38] The method according to any one of [F-1] to [F-37], wherein the method comprises treating a mixture containing two or more different compounds having a Boc group on a nitrogen atom.

### ADVANTAGEOUS EFFECTS OF INVENTION

In one aspect, the present invention enables improvement of the conversion rate and/or suppression of byproducts in the deprotection reaction of a Boc group by using the metal triflate or rare-earth metal halide and a base.

### DESCRIPTION OF EMBODIMENTS

In one aspect, the present invention provides a method for producing a compound, comprising treating a compound having a tert-butoxycarbonyl (Boc) group on a nitrogen atom with a trifluoromethanesulfonic acid metal salt (metal triflate: M(OTf)ₙ) or rare-earth metal halide (MXₙ) in the presence of a base to obtain a compound in which the Boc group is removed. The Boc group can be introduced in the production of the compound having a Boc group on a nitrogen atom by known methods, for example, by reacting a compound having an H-N group with di-tert-butyl dicarbonate (Boc₂O) in the presence of a base. The nitrogen atom having a Boc group is not particularly limited, and examples thereof include a nitrogen atom of a primary amine or a secondary amine having a basicity. As used herein, the secondary amine includes a cyclic amine.

The Boc group on the nitrogen atom is converted to a hydrogen atom by removing the Boc group. The compound in which the Boc group is removed is not particularly limited as long as the compound has an H-N structure, and examples thereof include a primary amine or a secondary amine having a basicity.

As used herein, the trifluoromethanesulfonic acid metal salt is also referred to as metal triflate ((CF₃SO₂)ₙM, M(OTf)ₙ). The metal triflate is not particularly limited, and examples thereof for M and n include tin triflate (Sn(OTf)₂), calcium triflate (Ca(OTf)₂), barium triflate (Ba(OTf)₂), zinc triflate (Zn(OTf)₂), aluminum triflate (Al(OTf)₃), gallium triflate (Ga(OTf)₃), indium triflate (In(OTf)₃), bismuth triflate (Bi(OTf)₃), lithium triflate (Li(OTf)), scandium triflate (Sc(OTf)₃), yttrium triflate (Y(OTf)₃), lanthanum triflate (La(OTf)₃), cerium triflate (Ce(OTf)₃, Ce(OTf)₄), praseodymium triflate (Pr(OTf)₃), neodymium triflate (Nd(OTf)₃), samarium triflate (Sm(OTf)₃), europium triflate (Eu(OTf)₃), gadolinium triflate (Gd(OTf)₃), terbium triflate (Tb(OTf)₃), dysprosium triflate (Dy(OTf)₃), holmium triflate (Ho(OTf)₃), erbium triflate (Er(OTf)₃), thulium triflate (Tm(OTf)₃), ytterbium triflate (Yb(OTf)₃), lutetium triflate (Lu(OTf)₃), and hafnium triflate (Hf(OTf)₄). In one aspect of the present invention, the metal triflate acts as a Lewis acid. Preferred examples of the metal triflate include tin triflate (Sn(OTf)₂), erbium triflate (Er(OTf)₃), thulium triflate (Tm(OTf)₃), yttrium triflate (Y(OTf)₃), and ytterbium triflate (Yb(OTf)₃). The metal triflate may be used singly or may be used as a mixture of two or more different metal triflates. In one aspect of the present invention, the metal triflate is a metal triflate selected from the group consisting of Sn(OTf)₂, Er(OTf)₃, Tm(OTf)₃, Y(OTf)₃, and Yb(OTf)₃, and may be used singly or may be used as a mixture of two or more different metal triflates as the metal triflate.

As used herein, the rare earth metal includes 17 metal elements consisting of scandium (atomic number 21), yttrium (atomic number 39), and lanthanoid (atomic numbers 57 to 71), and examples thereof include lanthanum, cerium, neodymium, praseodymium, dysprosium, samarium, yttrium, gadolinium, erbium, ytterbium, holmium, terbium, europium, thulium, and lutetium. The rare-earth metal halide is represented herein as MXₙ. As used herein, the rare-earth metal halide is not particularly limited as long as it is a rare-earth metal halide that can be stably present, and M, X, and n represent a rare earth metal element, a halogen atom, and an integer corresponding to each rare-earth metal halide, respectively.

In one aspect of the present invention, the rare-earth metal halide is represented by formula MX₃, wherein M is a rare earth metal element selected from rare earth metals such as lanthanum, cerium, neodymium, praseodymium, dysprosium, samarium, yttrium, gadolinium, erbium, ytterbium, holmium, terbium, europium, thulium, and lutetium, and X is a chlorine atom, a bromine atom, or an iodine atom. In one aspect of the present invention, the rare-earth metal halide is rare earth metal chloride or rare earth metal bromide.

In one aspect of the present invention, the base used in combination with the metal triflate or rare-earth metal halide is selected from the group consisting of organic bases having pKa of conjugate acid in water of 1 to 14, 2 to 12, 3 to 11, 3 to 10, 3 to 9, 3 to 8, or 3.5 to 7.5. As used herein, the base may be used singly or may be used as a mixture containing two or more bases. The pKa of the conjugate acid in water of the base here can be determined by conventional methods. For example, the values measured at 25°C by the method described in the 5th series of experimental chemistry "Thermal Measurement and Equilibrium", page 460 (edited by the Chemical Society of Japan, published by Maruzen Publishing Co., Ltd.) can be used. Also, the values described in publicly known literature Eur. J. Org. Chem. 2019, 6735-6748, the values calculated by using Advanced Chemistry Development (ACD/Labs) Software V11.02 ((c) 1994-2019 ACD/Labs), ADMET predictor (version 9.5), the values described in the catalog of Sigma-Aldrich Co. LLC, the values described in Chemical Book (https://www.chemicalbook.com), or the values described in PubChem (https://pubchem.ncbi.nlm.nih.gov) can be appropriately referred to as reference values. The pKa values of conjugate acids in water described in Chemical Book (https://www.chemicalbook.com) are, for example, 6.65 for 2,6-lutidine, 10.98 (± 0.28) for N,N-diisopropylethylamine, 7.38 for N-methylmorpholine, and 3.58 for 2,6-di-tert-butylpyridine. Also, the pKa value of the conjugate acid in water described in PubChem (https://pubchem.ncbi.nlm.nih.gov) is, for example, 7.55 for di(trimethylsilyl)amine.

In one aspect of the present invention, the base may be a base represented by formula (B1) or (B2): 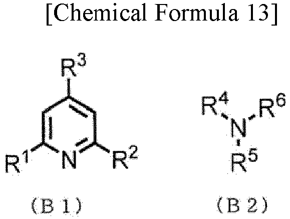
wherein R¹, R², and R³ are each independently selected from a hydrogen atom, C₁₋₆ alkyl, or C₆₋₁₀ aryl;
R⁴ and R⁵ together with a nitrogen atom to which they are attached form a 5- to 7-membered saturated heterocycle, the heterocycle optionally containing O, S, or N, or R⁴ and R⁵ are each independently C₁₋₆ alkyl, C₆₋₁₀ aryl, or tri(C₁₋₆ alkyl)silyl; and
R⁶ is a hydrogen atom or C₁₋₆ alkyl.

In one aspect of the present invention, the deprotection reaction of a Boc group with a metal triflate or rare-earth metal halide can be performed, for example, in a solvent selected from an ether-based solvent such as tetrahydrofuran, 2-methyltetrahydrofuran, 4-methyltetrahydropyran, 1,2-dimethoxyethane, diethyl ether, or 1,4-dioxane; a halogen-based solvent such as dichloromethane, 1,2-dichloroethane, or chloroform; a nitrile-based solvent such as acetonitrile or propionitrile; an aromatic hydrocarbon-based solvent such as benzene, toluene, or xylene; and an ester-based solvent such as ethyl acetate, isopropyl acetate, or butyl acetate (a single solvent may be used, or a mixture of a plurality of solvents may be used), at a temperature of 0°C to 120°C, 10°C to 100°C, 15°C to 100°C, 20°C to 90°C, 25°C to 80°C, 30°C to 80°C, 35°C to 80°C, or 40°C to 80°C, using a base selected from 2,6-lutidine, N,N-diisopropylethylamine, N-methylmorpholine, 2,6-di-tert-butylpyridine, and di(trimethylsilyl)amine.

The present invention provides, as one aspect, the following:
A method for producing a compound, comprising treating a compound having a Boc group on a nitrogen atom with at least one metal triflate or rare-earth metal halide selected from the group consisting of Sn(OTf)₂, Er(OTf)₃, Tm(OTf)₃, Y(OTf)₃, Yb(OTf)₃, ErCl₃, ErBr₃, and ErI₃, in the presence of at least one base selected from the group consisting of 2,6-lutidine, N,N-diisopropylethylamine, N-methylmorpholine, 2,6-di-tert-butylpyridine, and di(trimethylsilyl)amine to obtain a compound in which the Boc group is removed.

The present invention provides, as one aspect, the following:
A method for producing a compound, comprising treating a compound having a Boc group on a nitrogen atom with at least one metal triflate or rare-earth metal halide selected from the group consisting of Sn(OTf)₂, Er(OTf)₃, Tm(OTf)₃, Y(OTf)₃, Yb(OTf)₃, ErCl₃, ErBr₃, and ErI₃, and at least one base selected from the group consisting of 2,6-lutidine, N,N-diisopropylethylamine, N-methylmorpholine, 2,6-di-tert-butylpyridine, and di(trimethylsilyl)amine, in the presence of at least one solvent selected from the group consisting of an ether-based solvent, a halogen-based solvent, a nitrile-based solvent, an aromatic hydrocarbon-based solvent, and an ester-based solvent to obtain a compound in which the Boc group is removed.

As used herein, examples of the C₆₋₁₀ aryl include phenyl and naphthyl.

Examples of the 5- to 10-membered heteroaryl containing one or more ring heteroatoms independently selected from O, N and S include pyrrolyl, thienyl, furyl, pyridyl, thiazolyl, isothiazolyl, pyrazolyl, oxazolyl, isoxazolyl, imidazolyl, triallyl, pyrimidyl, pyrazinyl, pyridazinyl, quinolinyl, isoquinolinyl, 4H-quinolidinyl, phthalazinyl, naphthyridinyl, quinoxalinyl, quinazolinyl, cinnolinyl, pteridinyl, indolyl, indolinyl, benzothiophenyl, benzofuranyl, benzisothiazolyl, benzisoxazolyl, indazolyl, benzimidazolyl, benzotriazolyl, azaindolyl, and imidazopyridyl.

As used herein, the 5- to 7-membered saturated heterocycle is a saturated heterocycle having 5 to 7 ring atoms and contains one or more heteroatoms selected from O, S, or N as a ring atom. Examples thereof include pyrrolidine, piperidine, piperazine, morpholine, thiomorpholine, homopiperazine, and homopiperidine.

As used herein, C₁₋₆ alkyl is a linear and branched monovalent saturated aliphatic group having 1 to 6 carbon atoms. Specific examples thereof include methyl, ethyl, n-propyl, isopropyl, n-butyl, isobutyl, sec-butyl, t-butyl, 1-methylpropyl, n-pentyl, isopentyl, 2-methylbutyl, 1,1-dimethylpropyl, 1-ethylpropyl, hexyl, 4-methylpentyl, and 2-ethylbutyl.

As used herein, C₂₋₆ alkenyl is a linear and branched monovalent group of 2 to 6 carbon atoms having one or more double bonds. Examples thereof include ethenyl (vinyl), 1-propenyl, 2-propenyl (allyl), propen-2-yl, and 3-butenyl (homoallyl).

As used herein, C₂₋₆ alkynyl refers to a linear or branched monovalent group of 2 to 6 carbon atoms having one or more triple bonds, and examples thereof include ethynyl, 1-propynyl, 2-propynyl, 1-butynyl, 2-butynyl, and 3-butynyl.

As used herein, C₃₋₈ cycloalkyl refers to a cyclic saturated aliphatic hydrocarbon group of 3 to 8 carbon atoms. Specific examples thereof include cyclopropyl, cyclobutyl, cyclopentyl, and cyclohexyl.

As used herein, C₇₋₁₄ aralkyl refers to aryl-substituted alkyl having a total of 7 to 14 carbon atoms. Examples thereof include benzyl, 1-phenethyl, 2-phenethyl, 1-naphthylmethyl, and 2-naphthylmethyl.

As used herein, C₁₋₆ alkoxy refers to a C₁₋₆ alkyl-O- group, wherein the C₁₋₆ alkyl is as already defined. Specific examples thereof include methoxy, ethoxy, 1-propoxy, 2-propoxy, n-butoxy, i-butoxy, sec-butoxy, and t-butoxy.

As used herein, (C₁₋₆ alkoxy)carbonyl refers to a C₁₋₆ alkoxy-C(=O)- group, wherein the C₁₋₆ alkoxy is as already defined.

As used herein, (C₁₋₆ alkyl)carbonyl refers to a C₁₋₆ alkyl-C(=O)- group, wherein the C₁₋₆ alkyl is as already defined.

As used herein, "(C₁₋₆ alkoxy)carbonyl" of (C₁₋₆ alkoxy)carbonylamino is as already defined.

As used herein, "(C₁₋₆ alkyl)" of (C₁₋₆ alkyl)amino is as already defined.

As used herein, "(C₁₋₆ alkyl)" of di(C₁₋₆ alkyl)amino is as already defined and may be the same or different.

As used herein, the 4- to 8-membered cyclic amino includes groups such as aziridinyl, azetidinyl, pyrrolidinyl, piperidinyl, piperazinyl, and morpholinyl, which bind with a nitrogen atom.

As used herein, "(C₆₋₁₀ aryl)" of (C₆₋₁₀ aryl)carbonylamino is as already defined.

As defined herein, "5- to 10-membered heteroaryl containing one or more ring heteroatoms independently selected from O, N, and S" of 5- to 10-membered heteroarylcarbonylamino containing one or more ring heteroatoms independently selected from O, N, and S.

As used herein, aminocarbonyl means -CONH₂.

"(C₁₋₆ alkyl)" of (C₁₋₆ alkyl)aminocarbonyl is as already defined.

As used herein, "(C₁₋₆ alkyl)" of di(C₁₋₆ alkyl)aminocarbonyl is as already defined and may be the same or different.

As used herein, the 4- to 8-membered cyclic amino of 4- to 8-membered cyclic aminocarbonyl is as already defined, which binds to the carbonyl with a nitrogen atom.

As used herein, "(C₆₋₁₀ aryl)" of (C₆₋₁₀ aryl)carbonyl is as already defined.

As defined herein, "5- to 10-membered heteroaryl containing one or more ring heteroatoms independently selected from O, N, and S" of 5- to 10-membered heteroarylcarbonyl containing one or more ring heteroatoms independently selected from O, N, and S.

As used herein, "(C₁₋₆ alkyl)" of tri(C₁₋₆ alkyl)silyl is as already defined and may be the same or different. Examples thereof include trimethylsilyl, triethylsilyl, and t-butyldimethylsilyl.

As used herein, "halogen atom" refers to a fluorine atom, a chlorine atom, a bromine atom, an iodine atom, and the like. In the present invention, when a halogen atom is a substituent for an aryl, a heteroaryl, and the like, examples of the preferred halogen atom include a fluorine atom, a chlorine atom, and a bromine atom. In the present invention, when a halogen atom is a substituent for an alkyl or a group containing an alkyl as a part thereof (alkoxy, alkenyl, alkylthio, and the like), examples of the preferred halogen atom include a fluorine atom. Specific examples of the group having a halogen atom as a substituent include trifluoromethyl, pentafluoroethyl, trifluoromethoxy, pentafluoroethoxy, trifluoromethylthio, and pentafluoroethylthio.

In the definition of formula (A) herein, when a predetermined group is substituted with one or more substituents, the number of such substituents may range from 1 to the number of substitutable sites, for example, may be 1 to 5, 1 to 4, 1 to 3, 1 or 2, or 1.

Examples of C₁₋₆ alkyl optionally substituted with one or more fluorine atoms include trifluoromethyl.

In one aspect of the present invention, the reaction time of the deprotection reaction of the Boc group can be appropriately set by a person skilled in the art, for example, it can be in the range of 1 minute to 96 hours, 5 minutes to 72 hours, 10 minutes to 48 hours, 15 to 48 hours, or 30 minutes to 24 hours.

In one aspect of the present invention, the method includes removing impurities after the deprotection reaction of the Boc group. The removing impurities can be performed by methods commonly performed in the art of the present invention. Examples of the impurities include impurities derived from reaction reagents, unreacted reaction reagents, decomposed products generated by the reaction, coexisting bases, and reaction solvents. In particular, specific examples of the impurities include metal triflates such as tin triflate (Sn(OTf)₂), bases such as 2,6-lutidine, decomposed products of alkoxybenzyl ether structures, solvents such as tetrahydrofuran. Examples of the methods for removing impurities include liquid-liquid separation, reduced-pressure distillation, methods using solid phase reagents, purification by normal phase or reverse-phase silica gel column chromatography, and purification by GPC (molecular sieve).

The liquid-liquid separation for removing impurities can be performed by methods commonly performed in the art of the present invention. The liquid-liquid separation is not particularly limited as long as it is a combination of solvents separated into multiple layers to separate the group of desired products from the group of impurities (unnecessities). For example, the liquid-liquid separation can be performed by combining a solvent selected from ester-based solvents such as ethyl acetate and isopropyl acetate, ether-based solvents such as diethyl ether, diisopropyl ether, t-butylmethyl ether, cyclopentyl methyl ether, 2-methyltetrahydrofuran, 4-methyltetrahydropyran, 1,2-dimethoxyethane, and 1,4-dioxane, halogen-based solvents such as dichloromethane, chloroform, and 1,2-dichloroethane, aromatic hydrocarbon-based solvents such as benzene and toluene, and hydrocarbon-based solvents such as hexane, cyclohexane, and heptane, with a solvent selected from water, an acidic aqueous solution such as an aqueous hydrochloric acid solution, and a basic aqueous solution such as an aqueous sodium bicarbonate solution. A single solvent may be used, or a mixture solvent of multiple solvents may be used. In addition, a combination of organic solvents to be separated into layers, such as a combination of hexane and acetonitrile, can be used when it is suitable to separate the group of desired products from the group of impurities (unnecessities).

The reduced-pressure distillation for removing impurities can be performed by methods commonly performed in the art of the present invention. The conditions for reduced-pressure distillation can be appropriately set depending on the impurities to be removed. For example, the pressure may be 100 to 400 mbar, 50 to 100 mbar, 5 to 50 mbar, 0.1 to 5 mbar or the like, and the temperature may be 20 to 100°C, 25 to 50°C, or 35 to 45°C, or the like.

The removing impurities using a solid phase reagent can be performed by methods commonly performed in the art of the present invention. Examples of the solid phase reagent include macroporous triethylammonium methyl polystyrene carbonate, macroporous polystyrene sulfonic acid, amine-supported silica gel, and carboxylic acid-supported silica gel.

In one aspect of the present invention, the method includes removing impurities after the deprotection reaction of the Boc group in solid phase synthesis. Examples of the impurities here include unreacted reaction reagents, impurities derived from reaction reagents, decomposed products cleaved from the resin for solid-phase synthesis by reaction, and coexisting bases. In particular, specific examples of the impurities include metal triflates such as tin triflate (Sn(OTf)₂), bases such as 2,6-lutidine, decomposed products cleaved from the resin for solid-phase synthesis by decomposition of the alkoxybenzyl ether structure. As the method for removing impurities, the impurities can be removed by washing the resin for solid-phase synthesis. For example, the impurities can be removed by transferring the resin for solid-phase synthesis to a column equipped with a filter and washing with an NMP solution of DMEDA, an NMP solution of 4,4'-di-tert-butyl-2,2'-bipyridyl, an NMP solution of mixed TBAHSO4 and DTBP, an NMP solution of DIPEA, a mixed solution of NMP and water, NMP, MeOH, DCM, and the like. These washes can be combined, and optionally repeated a plurality of times.

In one aspect of the present invention, the compound having a Boc group on a nitrogen atom attached to a resin for solid-phase synthesis used as a substrate for a deprotection reaction of the Boc group may be a plurality of different compounds. For example, a resin for solid-phase synthesis to which 2 or more, 3 or more, 4 or more, 5 or more, 7 or more, or 10 or more different compounds having a Boc group on a nitrogen atom are attached can be used as a substrate. The resin for solid-phase synthesis used as the solid-phase support is not particularly limited as long as it is commonly used. Examples thereof include a Carboxylic resin, a CTC resin, a Trt resin, a SASRIN resin, an Rink amide resin, a PAL AM resin, a Seiber amide resin, a Merrifield resin, a Wang resin, 2-(4-bromomethylphenoxy)ethyl polystyrene, and a solid phase support having any functional group such as a carboxyl group, an amino group, an aminomethyl group, a hydroxy group, or a hydroxymethyl group on polystyrene. Furthermore, the resin for solid-phase synthesis may have a design in which cleavage can be made between the linker and compound 1 or 2 by using the linker that covalently connects the support and the compound. Examples of such a linker structure include an alkoxybenzyl ether structure, a trialkoxybenzylaminocarbonyl structure, a dialkoxyphenyl-alkoxyphenylmethylaminocarbonyl structure, and an alkoxyxanthen-9-ylaminocarbonyl structure. The support is also not particularly limited, and examples thereof include polystyrene and polyethylene glycol (PEG).

Methods and reaction conditions for attaching a compound to a resin for solid-phase synthesis can be appropriately set by a person skilled in the art based on the methods described in publicly known literature and the like. In one aspect of the present invention, a resin for solid-phase synthesis to which a compound having a Boc group on a nitrogen atom is attached can be prepared by performing the compound having a Boc group on a nitrogen atom or a precursor compound thereof supported on the resin for solid-phase synthesis. The supporting of the compound to the resin for solid-phase synthesis can be performed by reacting the reactive group of the side chain of the resin for solid-phase synthesis with the reactive group of the compound to form a covalent bond. In one aspect of the present invention, a resin for solid-phase synthesis to which a compound having a Boc group on a nitrogen atom is attached, as a substrate can be prepared by chemically modifying a resin for solid-phase synthesis supporting a precursor compound.

The reaction conditions for cleaving the compound from the resin for solid-phase synthesis can be appropriately set by a person skilled in the art based on the chemical structure of the resin for solid-phase synthesis used. Examples of the reagents used for cleavage can include hydrochloric acid, carboxylic acids such as trifluoroacetic acid (TFA), fluoroalcohols such as 2,2,2-trifluoroethanol (TFE) or 1,1,1,3,3,3-hexafluoroisopropyl alcohol (HFIP), Brensted acid with pKa of 10 or less in water, or any Lewis acid. In one aspect, a compound cleaved from a resin for solid-phase synthesis can be used as a compound for screening for pharmaceutical exploration.

Hereinafter, the present invention will be described in more detail using Reference Examples and Examples, but the present invention is not limited to these Examples.

Hereinafter, the present invention will be described in more detail using Reference Examples and Examples, but the present invention is not limited to these Examples.

### EXAMPLES

All starting materials, reagents, and solvents were obtained from commercial suppliers, or synthesized by known methods. The reagents and solvents were of reagent quality or better and were used as obtained from various commercial sources, unless otherwise noted. For metal triflates, information such as commercial source and catalog code is shown below.

### [Table 1]

**[Table 1]**

| Metal triflate | Commercial source | Catalog code |
|---|---|---|
| Sn(OTf)₂ | Sigma-Aldrich | 388122 |
| Er(OTf)₃ | Sigma-Aldrich | 425672 |
| Tm(OTf)₃ | Sigma-Aldrich | 425737 |
| | Combi-Blocks | QB-1723 |
| Y(OTf)₃ | Sigma-Aldrich | 425745 |
| Yb(OTf)₃ | Sigma-Aldrich | 430595 |

For silica gel of the column chromatography, Biotage(R) SNAP MLtra, Biotage(R) Sfaer D (Duo) (60 µm), or Biotage(R) Sfaer HC D (Duo) (20 µm), or the like was appropriately used.

For amino silica gel of the column chromatography, Biotage(R) SNAP Isolute NH₂ (50 µm) or Biotage(R) SNAP Cartridge KP-NH, or the like was appropriately used.

For reverse phase silica gel of the column chromatography, Biotage(R) SNAP Mltra C18 (25 µm), Biotage(R) Sfaer C18 (30 µm), or the like was appropriately used.

¹H-NMR and ¹³C-NMR spectra were measured, with or without Me₄Si as the internal standard substance, using ECP-400 (manufactured by JEOL Ltd.), Agilent 400-MR (manufactured by Agilent Technologies), AVANCE3 Cryo-TCI, AVANCE3 400, AVANCE3 HD 400, AVANCE NEO 400, AVANCE3 HD 300, AVANCE3 300, AVANCE2 300, AVANCE NEO 300 (manufactured by Bruker) or the like as appropriate (s = singlet, brs = broad singlet, d = doublet, t = triplet, q = quartet, dd = double doublet, ddd = double double doublet, dt = double triplet, td = triple doublet, and m = multiplet).

The reaction tracking and purity measurement were carried out, unless otherwise noted, by performing retention time measurement and mass spectrometry using 2020 (manufactured by Shimadzu Corporation), under the analysis conditions shown in the following tables.

In Examples, the following abbreviations were used.

**[Table 2]**

| **Abbreviation** | |
|---|---|
| **Boc** | tert- Butoxycarbonyl |
| **TFA** | Trifluoroacetic acid |
| **DCM** | Dichloromethane |
| **DMF** | N,N-Dimethylformamide |
| **DMSO** | Dimethyl sulfoxide |
| **MeCN** | Acetonitrile |
| **MeOH** | Methanol |
| **NMP** | N-Methylpyrrolidone |
| **THF** | Tetrahydrofuran |
| **HFIP** | Hexafluoro-2-propanol |
| **TMSOTf** | Trimethylsilyl trifluoromethanesulfonate |
| **Sn(OTf)₂** | Tin(II) trifluoromethanesulfonate |
| **Er(OTf)₃** | Erbium(III) trifluoromethanesulfonate |
| **Tm(OTf)₃** | Thulium(III) trifluoromethanesulfonate |
| **Y(OTf)₃** | Yttrium(III) trifluoromethanesulfonate |
| **Yb(OTf)₃** | Ytterbium(III) trifluoromethanesulfonate |
| **Cs₂CO₃** | Cesium carbonate |
| **DTBP** | 2,6-Di-tert-butylpyridine |
| **HMDS** | 1,1,1,3,3,3-Hexamethyldisilazane |
| **NMM** | N-Methylmorpholine |
| **DIPEA** | N,N-Diisopropylethylamine |
| **DMEDA** | N,N'-Dimethylethylenediamine |
| **TBAHSO₄** | Tetrabutylammonium bisulfate |
| **PMBCI** | 4-Methoxybenzyl chloride |
| **DIC** | N,N'-Diisopropylcarbodiimide |
| **HOAt** | 1-Hydroxy-7-azabenzotriazole |
| **PipCIU** | Chlorodipiperidinocarbenium hexafluorophosphate (CAS: 161308-40-3) |
| **NMI** | 1-Methylimidazole |
| **Pyoxim** | [Ethylcyano(hydroxyimino)acetato-O²]tri-1-pyrrolidinylphosphonium hexafluorophosphate (CAS: 153433-21-7) |

The analysis conditions of LCMS are shown in the following table.

**[Table 3]**

| **Analytical method** | **Device** | **Column** | **Mobile phase A** | **Mobile phase B** | **Gradient** | **Flow rate** |
|---|---|---|---|---|---|---|
| **FA05-1** | **nexera/2020** | **Ascentis Express C18** | **0.1 %FA H2O** | **0.1 %FA MeCN** | **AlB=95/5 →** | **1 mL/min** |
| | | | | | **0/100(1.5 min) →** | |
| | | **2.1mml.D.x50mm,2.7um** | | | **0/100(0.5 min)** | |
| **RPAmideTFA10cm** | **nexera/2020** | **Ascentis Express RP-Amide** | **0.05%TFA H2O** | **0.05%TFA MeCN** | **A/B=95/5 →** | **0.5 mL/min** |
| | | | | | **0/70(10 min) →** | |
| | | **2.1mml.D.x100mm,2.7um** | | | **0/100(0.5 min),** | |
| | | | | | **0/100 (0.5 min)** | |

The descriptions of m/z [M+H]⁺ and (M+H)⁺ noted in the LCMS analysis results in Examples all indicate values detected in positive mode, unless otherwise noted. Also, the UV area% in LCMS indicated values with PDA (190 to 400 nm or 210 to 400 nm), unless otherwise noted. When a specific wavelength (for example, 299 nm) is described, the UV area% at wavelengths up to +/- 4 nm centered on the described wavelength was shown. Note that the blank in the table indicates that it was below the detection limit.

The expression "concentration under reduced pressure" refers to the evaporation and removal of a solvent under reduced pressure with a rotary evaporator, a mechanical oil vacuum pump, or a mechanical oil-free vacuum pump.

The expression "drying overnight under reduced pressure" refers to the evaporation and removal of a solvent under reduced pressure with a rotary evaporator, a mechanical oil vacuum pump, or a mechanical oil-free vacuum pump.

The expressions "overnight" and "all night" refer to about 8 to 14 hours unless otherwise noted.

The expressions "room temperature" and "rt" refer to about 20 to 25°C unless otherwise noted.

The solid phase reaction can be performed in an appropriate container, such as a glass vial that can be tightly sealed by a cap equipped with a Teflon(R) packing or a column having a frit filter and an appropriate stopper. The container size is selected as appropriate such that there is sufficient space for the solvent and sufficient room for the resin to be effectively stirred, taking into account that certain resins may be significantly swollen when treated with organic solvents.

Stirring in the solid phase reaction was performed at 50 to 200 rpm using an appropriate shaker (for example, Tokyo Rikakikai Co., Ltd., EYELA, MMS-320, MMS-220H, or Asone, MyBL-100CS, or TAITEC, M·BR-104) or an agitator (combination of Asahi Glassplant inc., separable flask, and NAKAMURA SCIENTIFIC INSTRUMENT CO., LTD., sealing mixer UZU, and AQUATECHS Co., Ltd., centrifugal agitator C-Mix) as appropriate in order to ensure adequate mixing, which is a factor generally accepted as important for successful reaction on the resin.

In order to monitor the progress of the reaction on the solid phase, it is necessary to collect the resin from the reaction vessel. At that time, using a micropipette fitted with a pipette tip cut at the appropriate length from the end, the resin was collected by sucking approximately 10 µL to ensure that the resin was included and was transferred onto the filter of a pipette tip equipped with a filter (for example, Thermo Scientific, tip with ART filter, ART20P, 2149P-05). Thereafter, the compound supported on the resin was cleaved from the resin by the following representative procedures for the resin on the filter. The resin was washed 3 times with DMF (0.1 mL), 3 times with MeOH (0.1 mL), and 3 times with DCM (0.1 mL), and then immersed in a 10% TFA/DCM solution containing 0.02 M pentamethylbenzene (0.05 mL) for 2 minutes. After filtration, the solid phase was washed with DMF (0.05 mL). MeCN (0.25 mL) was then added to the filtrate to prepare an LC sample, and the progress of the reaction was measured by LCMS measurement.

The expression "cleavage" from the solid phase indicates the removal of the compound supported on the resin from the resin, such as a treatment of the resin with a 10% TFA/DCM solution containing 0.02 M pentamethylbenzene to recover the supported compound into the solution.

Compound No. used in Examples was indicated by the combination of arbitrary alphabets, numbers, and symbols. For compounds in the state wherein they are supported on the solid phase, "R" was added at the end, for example, "A02-1R". In contrast, the compound cleaved from the solid phase was indicated as "A02" without "-1R".

The notation 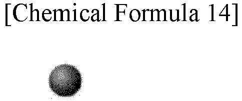 used in the chemical structure representations in Examples refer to polystyrene resin and indicate the state wherein the compound is supported on the solid phase.

In "-1R" used in Examples to indicate that the compound is supported on the solid phase, the number indicates the lot of resin used.

Notation example of "-1R"

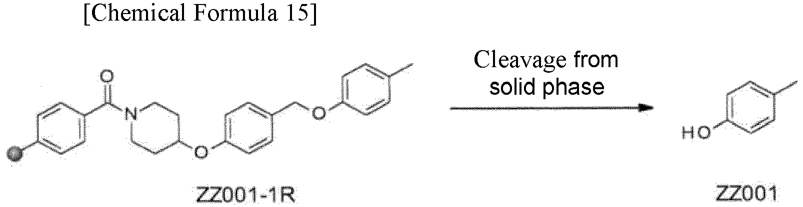

For the solid phase-supported compound used in the solid phase synthesis, the loading rate (mmol/g) is shown, which indicates the amount of support calculated when the cleaved compound is assumed to be 100% supported on the solid phase.

Even when the solid phase-supported compound is the same, the loading rate may vary from lot to lot, but the same compound No. may be used for the compound No.

### Example 1: Synthesis of substrates used in the present specification

### Example 1-1: Synthesis of a substrate for liquid-phase reaction study

### Example 1-1-1: Synthesis of 1-methoxy-4-((4-(2-methoxyethyl)phenoxy)methyl)benzene (A02)

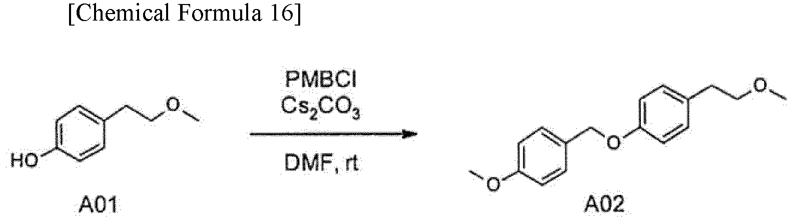

To a glass vial, 4-(2-methoxyethyl)phenol (A01) (0.500 g, 3.29 mmol), cesium carbonate (2.68 g, 8.21 mmol), and DMF (6.57 mL) were added. After 4-methoxybenzyl chloride (0.535 mL, 3.94 mmol) was added, replacement by nitrogen was performed three times in the glass vial and the mixture was stirred at room temperature for 3 hours. Water was added to the reaction solution and the mixture was extracted three times with hexane/ethyl acetate = 4/1. The combined organic layers were filtered through a Phase Separator and the filtrate was concentrated. The resulting residue was purified by silica gel column chromatography (Sfaer HC D 25 g, 0 to 30% ethyl acetate/hexane) to obtain the title compound A02 (0.8638 g, 3.17 mmol, 97% yield) of white solid.

¹H-NMR (400 MHz, CDCl₃) δ: 7.35 (2H, d, J = 8.3 Hz), 7.13 (2H, d, J = 8.3 Hz), 6.93-6.89 (4H, m), 4.96 (2H, s), 3.81 (3H, s), 3.56 (2H, t, J = 7.1 Hz), 3.35 (3H, s), 2.82 (2H, t, J = 7.1 Hz).
Retention time: 1.252 min (analysis condition FA05-1)
Retention time: 7.773 min (analysis condition RPAmideTFA 10 cm).

### Example 1-1-2: Synthesis of 1-O-tert-butyl 4-O-methyl 4-[[4-(4-methoxyphenyl)benzoyl]amino]piperidine-1,4-dicarboxylate (I03)

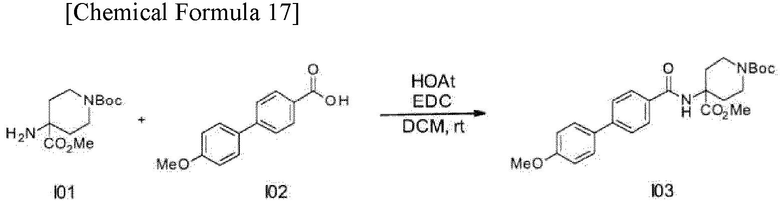

To a round-bottom flask, 1-O-tert-butyl 4-O-methyl 4-aminopiperidine-1,4-dicarboxylate (I01, CAS No. 115655-44-2) (1.00 g, 3.87 mmol) and EDC (1-(3-dimethylaminopropyl)-3-ethylcarbodiimide hydrochloride) (891 mg, 4.65 mmol), HOAt (263 mg, 1.94 mmol), and DCM (12.9 mL) were added. After 4-(4-methoxyphenyl)benzoic acid (I02, CAS No. 725-14-4) (1.06 g, 4.65 mmol) was added, the mixture was stirred at room temperature for 1 hour. Ice water was added to the reaction solution and the mixture was extracted twice with DCM. The combined organic layers were washed with an aqueous sodium bicarbonate solution, then dried over magnesium sulfate and concentrated under reduced pressure. The resulting residue was purified by silica gel column chromatography (Biotage(R), Sfaer HC D 50 g, 10 to 60% ethyl acetate/hexane) to obtain the title compound I03 (1.40 g, 2.99 mmol, 77% yield) of white solid.
LRMS: m/z 369 [M-Boc+2H]⁺
Retention time: 1.100 min (analysis condition FA05-1)

### Example 1-1-3: Synthesis of methyl 4-[[4-(4-methoxyphenyl)benzoyl]amino]piperidine-4-carboxylate hydrochloride (I04)

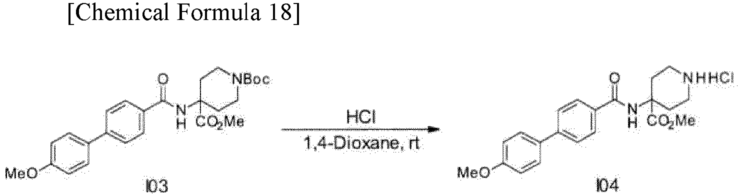

To a round-bottom flask, I03 (1.40 g, 2.99 mmol) and a solution of 4 M hydrochloric acid-1,4-dioxane (7.47 mL) were added, and the mixture was stirred at room temperature for 2 hours. After the reaction solution was concentrated under reduced pressure, the resulting residue was filtered with DCM to obtain the title compound I04 (1.18 g, 2.91 mmol, 98% yield) of white solid.
LRMS: m/z 369 [M-Cl]⁺
Retention time: 0.704 min (analysis condition FA05-1)

### Example 1-1-4: Synthesis of methyl 1-[3,5-bis(trifluoromethyl)benzoyl]-4-[[4-(4-methoxyphenyl)benzoyl]amino]piperidine-4-carboxylate (I06)

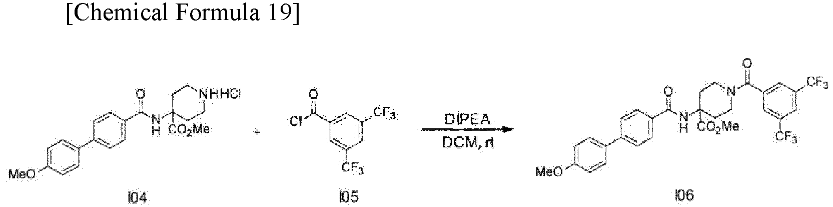

To a round-bottom flask, I04 (1.18 g, 2.92 mmol) and 3,5-bis(trifluoromethyl)benzoyl chloride (I05, CAS No. 785-56-8) (629 µL, 3.50 mmol), and DCM (29.2 mL) were added. After DIPEA (1.27 mL, 7.29 mmol) was added, the mixture was stirred at room temperature for 1 hour. Ice water was added to the reaction solution and the mixture was extracted twice with DCM. The combined organic layers were washed with an aqueous sodium bicarbonate solution, then the organic layers were dried over magnesium sulfate and concentrated under reduced pressure. The resulting residue was purified by silica gel column chromatography (Biotage(R), Sfaer HC D 50 g, 10 to 50% ethyl acetate/hexane) to obtain the title compound I06 (2.12 g, 3.48 mmol) of white solid.
LRMS: m/z 609 [M+H]⁺
Retention time: 1.159 min (analysis condition FA05-1)

### Example 1-1-5: Synthesis of 1-[3,5-bis(trifluoromethyl)benzoyl]-4-[[4-(4-methoxyphenyl)benzoyl]amino]piperidine-4-carboxylic acid (I07)

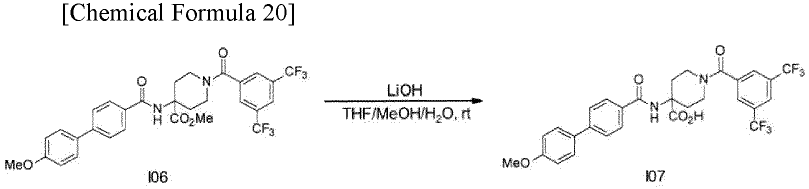

To a round-bottom flask, I06 (1.78 g, 2.93 mmol), THF (16.3 mL), methanol (6.50 mL), and water (6.50 mL) were added. After lithium hydroxide (299 mg, 12.5 mmol) was added, the round-bottom flask was stirred at room temperature for 3 hours. After the reaction solution was concentrated under reduced pressure, 1 M hydrochloric acid was added to the resulting residue, and the mixture was extracted twice with ethyl acetate. The combined organic layers were dried over sodium sulfate and then concentrated under reduced pressure to dryness to obtain the title compound I07 (1.38 g, 2.32 mmol, 79% crude yield) of white solid as a crude product.
LRMS: m/z 595 [M+H]⁺
Retention time: 1.061 min (analysis condition FA05-1)

### Example 1-1-6: Synthesis of 1-[3,5-bis(trifluoromethyl)benzoyl]-4-[[4-(4-methoxyphenyl)benzoyl]amino]-N-[(2A6-trimethoxyphenyl)methyl]piperidine-4-carboxamide (I09)

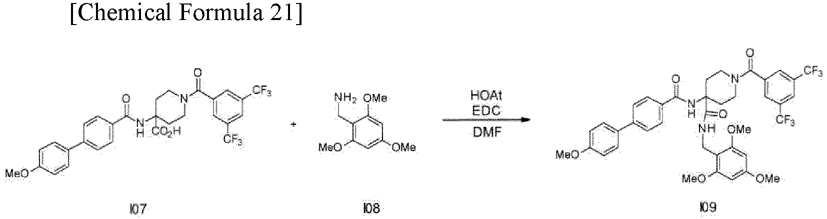

To a glass vial, I07 (crude product 100 mg, 168 pmol), EDC (1-(3-dimethylaminopropyl)-3-ethylcarbodiimide hydrochloride) (38.7 mg, 202 µmol), HOAt (24.0 mg, 177 pmol), and DMF (336 µL) were added. After (2,4,6-trimethoxyphenyl)methanamine (I08, CAS No. 77648-20-5) (34.8 mg, 177 µmol) was added, the glass vial was shaken at room temperature for 20 hours. After further shaking at 50°C for 5 hours, I08 (34.8 mg, 177 pmol) was added and then the vial was shaken at 50°C for 2.5 hours. Water was added to the reaction solution and the mixture was extracted twice with DCM. The combined organic layers were washed with an aqueous sodium bicarbonate solution, then the organic layers were dried over sodium sulfate and concentrated under reduced pressure. The resulting residue was purified by silica gel column chromatography (Biotage(R), Sfaer HC D 5 g, 10 to 60% ethyl acetate/hexane) to obtain the title compound I09 (89.0 mg, 117 pmol, 70% yield) of white solid.
LRMS: m/z 774 [M+H]⁺
Retention time: 1.347 min (analysis condition FA05-1)

### Example 1-1-7: Synthesis of 1-[3,5-bis(trifluoromethyl)benzoyl]-4-[[4-(4-methoxyphenyl)benzoyl]amino]-N-[(2,4-dimethoxyphenyl)-(4-methoxyphenyl)methyl]-4-[[4-(4-methoxyphenyl)benzoyl]amino]piperidine-4-carboxamide (I12)

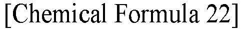

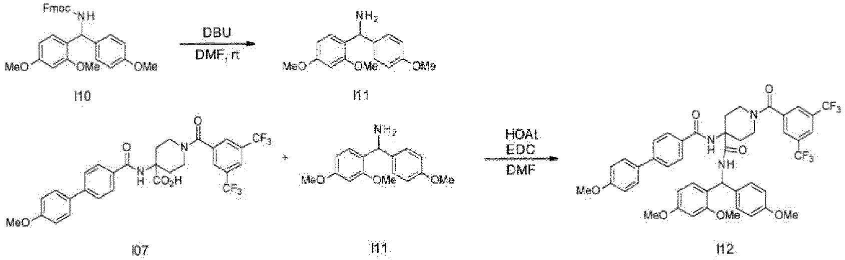

To a glass vial, 9H-fluoren-9-ylmethyl N-[(2,4-dimethoxyphenyl)-(4-methoxyphenyl)methyl]carbamate (I10, CAS No. 133566-68-4) (88.0 mg, 177 pmol) and DMF (177 µL) were added. After DBU (1,8-diazabicyclo[5.4.0]undec-7-ene) (27.9 µL, 185 pmol) was added, the glass vial was shaken at room temperature for 1 hour to prepare a DMF solution of I11 ((2,4-dimethoxyphenyl)-(4-methoxyphenyl)methanamine, CAS No. 161110-81-2) (1.00 M). To another glass vial, I07 (crude product 100 mg, 168 µmol), EDC (1-(3-dimethylaminopropyl)-3-ethylcarbodiimide hydrochloride) (38.7 mg, 202 pmol), HOAt (24.0 mg, 177 pmol), and DMF (336 µL) were added. To this glass vial, a previously prepared DMF solution of I11 (1.00 M, 177 µL) was added and the vial was shaken at room temperature for 17 hours. Then, after shaking at 50°C for 7.5 hours, a newly prepared DMF solution of I11 (1.00 M, 88.5 µL) was added and the vial was shaken at 50°C for 17.5 hours. Subsequently, a newly prepared DMF solution of I11 (1.00 M, 88.5 µL) was added, and the vial was shaken at 50°C for 30 minutes. Then, EDC (19.4 mg, 101 pmol) and HOAt (12.0 mg, 88.5 pmol) were added, and the vial was shaken at 50°C for 4.5 hours. A newly prepared DMF solution of I11 (1,00 M, 88.5 µL) was further added, and the vial was shaken at 50°C for 13 hours, then at room temperature for 52 hours. Water was added to the reaction solution and the mixture was extracted twice with DCM. The combined organic layers were washed with an aqueous sodium bicarbonate solution, then the organic layers were dried over sodium sulfate and concentrated under reduced pressure. The resulting residue was purified by silica gel column chromatography (Biotage(R), Sfaer HC D 5 g, 10 to 60% ethyl acetate/hexane) to obtain the title compound I12 (61.2 mg, 72.0 µmol, 43% yield) of white solid.
LRMS: m/z 872 [M+Na]⁺
Retention time: 1.428 min (analysis condition FA05-1)

### Example 1-2: Synthesis of a substrate for solid-phase reaction study

### Example 1-2-1: Synthesis of compound D02-1R

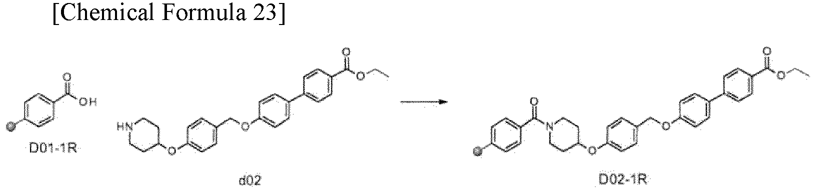

Under a nitrogen atmosphere, to an 800 mL column with a filter, Carboxylic Resin (D01-1R) (Rapp Polymer, 1.70 mmol/g, 40.0 g) and NMP (600 mL) were added. The column was capped to prevent leakage of the reaction solution and shaken at room temperature for 1 hour. HOAt (9.26 g, 68.0 mmol), DIC (10.6 mL, 68.0 mmol) and ethyl 4-[4-[(4-piperidin-4-yloxyphenyl)methoxy]phenyl]benzoate (d02) (4.13 g, 9.58 mmol) were added, and then the column was capped to prevent leakage of the reaction solution and shaken at room temperature for 3.5 hours. HOAt (18.5 g, 136 mmol), DIC (21.2 mL, 136 mmol) and piperidine (16.8 mL, 170 mmol) were added, and then the column was capped to prevent leakage of the reaction solution and shaken at room temperature all night.

### Solid-phase purification

The whole of the suspension of the reaction solution and the solid phase was transferred to a 2 L separable flask, and washed 3 times with NMP (800 mL), 3 times with MeOH (800 mL), 3 times with DCM (800 mL), and 3 times with heptane (800 mL), and the resulting solid phase was dried overnight under reduced pressure to obtain compound D02-1R (0.200 mmol/g, 47.9 g).

### Reaction tracking

10 µL of the suspension of the solid phase and the liquid phase was transferred to a tip with a filter and washed 3 times with NMP (0.1 mL), 3 times with MeOH (0.1 mL), and 3 times with DCM (0.1 mL). The resultant was immersed in a 10% TFA/DCM solution (0.05 M, 0.1 mL) of pentamethylbenzene for 5 minutes, and washed with DMF (0.05 mL). 100 µL of the combined filtrates was diluted with MeCN (0.25 mL) and then subjected to LCMS measurement to measure the progress of the reaction. As a result, the target material D02 was observed as 100%.

Compound D02

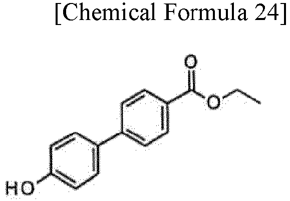

LRMS: m/z 243 [M+H]⁺
Retention time: 1.081 min (analysis condition FA05-1, 299 nm).

### Example 1-2-2: Synthesis of compound D03-1R

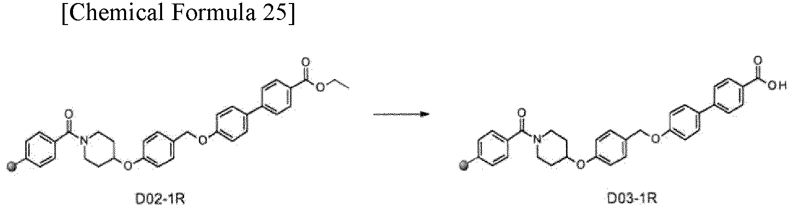

Under a nitrogen atmosphere, to a 2 L separable flask, compound D02-1R (0.200 mmol/g, 47.9 g), NMP (503 mL), and 2-methyl-2-butanol (144 mL) were added, and the mixture was stirred at room temperature for 1 hour. After the suspension was cooled to 5°C, an aqueous tetrabutylammonium hydroxide solution (1 M, 14.4 mL, 14.4 mmol) was added dropwise, and the mixture was stirred at 25°C for 3.5 hours. To this, an aqueous tetrabutylammonium hydroxide solution (1 M, 2.39 mL, 2.39 mmol) was added dropwise, and the mixture was stirred at 25°C for 1 hour and 15 minutes. To this, an aqueous tetrabutylammonium hydroxide solution (1 M, 2.39 mL, 2.39 mmol) was added dropwise, and the mixture was stirred at 25°C for 1 hour.

### Solid-phase purification

The suspension of the reaction solution and the solid phase was washed 3 times with an NMP solution of HOAt (0.1 M, 800 mL), 3 times with NMP (800 mL), 3 times with MeOH (800 mL), 3 times with DCM (800 mL), and 3 times with heptane (800 mL), and the resulting solid phase was dried for 6 days under reduced pressure to obtain compound D03-1R (0.201 mmol/g, 49.8 g).

### Reaction tracking

12 µL of the suspension of the solid phase and the liquid phase was transferred to a tip with a filter and washed 3 times with NMP (0.1 mL), 3 times with MeOH (0.1 mL), and 3 times with DCM (0.1 mL). The resultant was immersed in a 10% TFA/DCM solution (0.02 M, 0.05 mL) of pentamethylbenzene for 5 minutes, and washed with DMF (0.05 mL). The combined filtrates were diluted with MeCN (0.25 mL) and then subjected to LCMS measurement to measure the progress of the reaction. As a result, the target material D03 was observed as 100%.

Compound D03

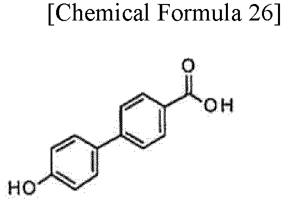

Maximum wavelength: 294 nm
Retention time: 0.761 min (analysis condition FA05-1, 299 nm).

### Example 1-2-3: Synthesis of compound D04-1R

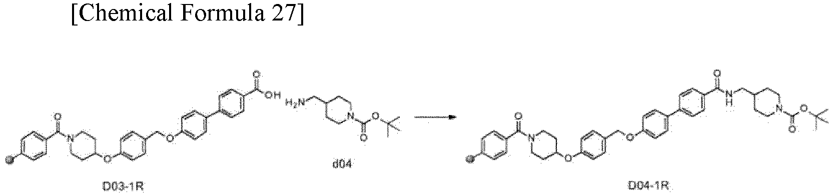

To a 50 mL Biotage column with a filter, D03-1R (0.201 mmol/g, 2.00 g) and DCM (24 mL) were added, and then the column was capped to prevent leakage of the reaction solution and shaken at room temperature for 1 hour. To this, 4-(aminomethyl)piperidine-1-carboxylic acid tert-butyl (d04) (0.215 g, 1.01 mmol) was added. A MeCN solution of mixed PipClU and 1-methylimidazole (both 1.0 M, 1 mL) was added, and then the column was capped to prevent leakage of the reaction solution and shaken at room temperature for 1 hour. The whole of the suspension of the solid phase and the liquid phase was transferred to eight 6 mL columns with filters using NMP/water = 1/1, and each was washed 3 times with NMP/water = 1/1 (5 mL), 3 times with NMP (5 mL), 3 times with MeOH (5 mL), 3 times with DCM (5 mL), and 2 times with heptane (5 mL). The resulting solid phase was dried overnight under reduced pressure to obtain compound D04-1R (0.193 mmol/g, 2.27 g).

### Reaction tracking

10 µL of the suspension of the solid phase and the liquid phase was transferred to a tip with a filter and washed 3 times with NMP (0.1 mL), 3 times with MeOH (0.1 mL), and 3 times with DCM (0.1 mL). The resultant was immersed in a 10% TFA/DCM solution (0.05 M, 0.05 mL) of pentamethylbenzene for 5 minutes, and washed with DMF (0.1 mL). 100 µL of the combined filtrates was diluted with MeCN (0.25 mL) and then subjected to LCMS measurement to measure the progress of the reaction. As a result, the target material D04 was observed as 86.3% and compound E04 in which the Boc-deprotection reaction was progressed by the cleavage solution (10% TFA/DCM) was observed as 13.7%.

Compound D04

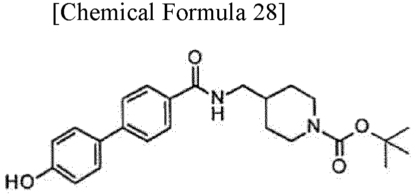

LRMS: m/z 355 [M-tBu+H]⁺
Retention time: 1.035 min (analysis condition FA05-1, 299 nm)

### Compound E04

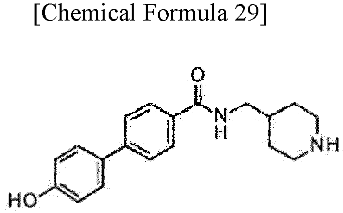

LRMS: m/z 311 [M+H]⁺
Retention time: 0.545 min (analysis condition FA05-1, 299 nm)

### Example 1-2-4: Synthesis of compound D02-2R

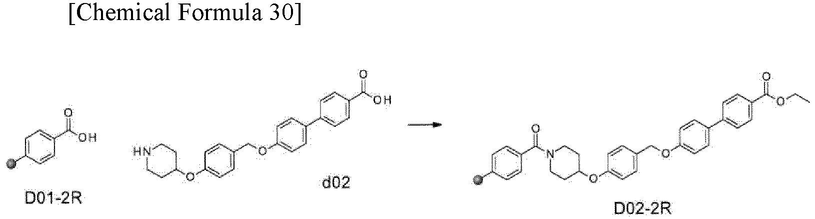

Under a nitrogen atmosphere, to an 800 mL empty column with a filter, Carboxylic Resin (D01-2R) (WATANABE CHEMICAL INDUSTRIES, LTD., loading rate 1.70 mmol/g, 24.7 g, 42.0 mmol) and NMP (371 mL) were added, and the column was shaken at room temperature for 45 minutes. HOAt (5.72 g, 42.0 mmol) and DIC (6.55 mL, 42.0 mmol), ethyl 4-[4-[(4-piperidin-4-yloxyphenyl)methoxy]phenyl]benzoate (d02) (2.56 g, 5.93 mmol) were added, and the column was shaken at room temperature for 4.5 hours. HOAt (11.5 g, 84.0 mmol), DIC (13.1 mL, 84.0 mmol) and piperidine (10.4 mL, 105 mmol) were added, and the column was shaken at room temperature for 21 hours.

### Solid-phase purification

The reaction solution was discharged from the column, and the solid phase was washed 3 times with NMP (500 mL), 3 times with MeOH (500 mL), 3 times with DCM (500 mL) and 3 times with heptane (500 mL), and the resulting solid phase was dried overnight under reduced pressure to obtain compound D02-2R (loading rate 0.200 mmol/g, 30.5 g, 6.1 mmol).

D02-2R was washed 3 times with DCM (0.10 mL), and then immersed in a 10% TFA/DCM solution containing 0.2 M pentamethylbenzene (0.05 mL) for 5 minutes. After filtration, DMF (0.05 mL) and MeCN (0.20 mL) were added to dilute, and the diluted solution was subjected to LCMS measurement. As a result, the target material D02 was observed as 100%.

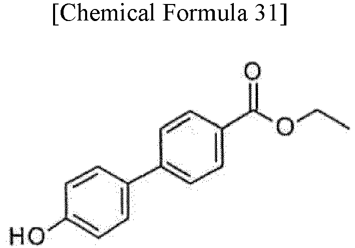

### Compound D02

LRMS: m/z 243 [M+H]⁺
Retention time: 1.071 min (analysis condition FA05-1, 299 nm).

### Example 1-2-5: Synthesis of compound D03-2R

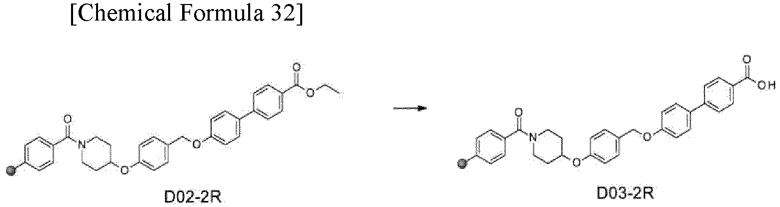

Under a nitrogen atmosphere, to an 800 mL empty column with a filter, compound D02-2R (loading rate 0.200 mmol/g, 29.6 g, 5.92 mmol), NMP (311 mL), and 2-methyl-2-butanol (89 mL) were added, and the mixture was stirred at room temperature for 1 hour. To the suspension, an aqueous normal tetrabutylammonium hydroxide solution (1 M, 11.8 mL, 11.8 mmol) was added dropwise, and the mixture was stirred at 25°C for 7 hours.

### Solid-phase purification

The suspension of the reaction solution and the solid phase was washed 3 times with an HOAt/NMP solution (0.1 M, 500 mL), 3 times with NMP (500 mL), 3 times with MeOH (500 mL), 3 times with DCM (500 mL), and 3 times with heptane (500 mL), and the resulting solid phase was dried for 4 days under reduced pressure to obtain compound D03-2R (loading rate 0.201 mmol/g, 30.6 g, 6.2 mmol).

D03-2R was washed 3 times with DCM (0.10 mL), and then immersed in a 10% TFA/DCM solution containing 0.2 M pentamethylbenzene (0.05 mL) for 5 minutes. After filtration, DMF (0.05 mL) and MeCN (0.2 mL) were added to dilute, and the diluted solution was subjected to LCMS measurement. As a result, the target material D03 was observed as 99.9%.

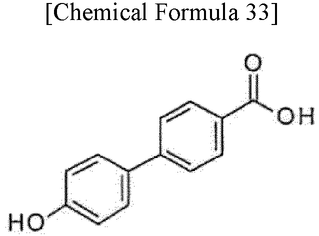

### Compound D03

Maximum wavelength: 293 nm
Retention time: 0.775 min (analysis condition FA05-1, 299 nm)

### Example 1-2-6: Synthesis of compound D05-2R

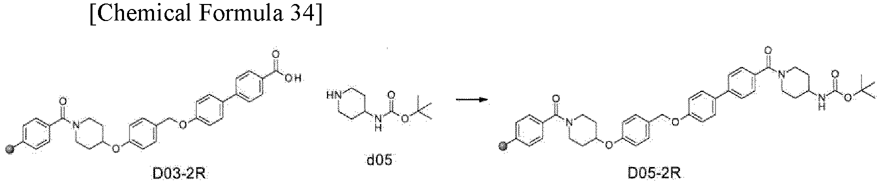

To a 50 mL Biotage column with a filter, D03-2R (0.201 mmol/g, 2.50 g) and DCM (37.5 mL) were added, and then the column was capped to prevent leakage of the reaction solution and the column was shaken at room temperature for 1 hour. To this, tert-butyl N-piperidin-4-ylcarbamate (d05) (0.201 g, 1.01 mmol), PipClU (0.363 g, 1.01 mmol) and 1-methylimidazole (0.160 mL, 2.01 mmol) were added, and then the column was capped to prevent leakage of the reaction solution and shaken at room temperature for 2 hours. The reaction solution was discharged from the column and the solid phase was washed 1 time with NMP (50 mL), 3 times with NMP/water = 1/1 (50 mL), 3 times with NMP (50 mL), 3 times with MeOH (50 mL), 3 times with DCM (50 mL), and 3 times with heptane (50 mL), and the resulting solid phase was dried overnight under reduced pressure to obtain compound D05-2R (0.194 mmol/g, 3.17 g).

### Reaction tracking

10 µL of the suspension of the solid phase and the liquid phase was transferred to a tip with a filter and washed 3 times with DMF (0.1 mL), 3 times with MeOH (0.1 mL), and 3 times with DCM (0.1 mL). The resultant was immersed in a 10% TFA/DCM solution (0.2 M, 0.05 mL) of pentamethylbenzene for 1 minute, and washed with DMF (0.05 mL). The combined filtrates were diluted with MeCN (0.20 mL) and then subjected to LCMS measurement to measure the progress of the reaction. As a result, the target material D05 was observed as 96.5% and compound E05 in which the Boc-deprotection reaction was progressed by the cleavage solution (10% TFA/DCM) was observed as 3.1%.

### Compound D05

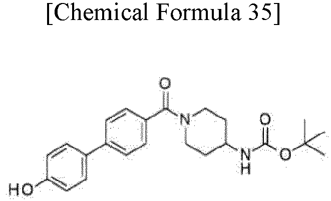

LRMS: m/z 341 [M-tBu+H]⁺
Retention time: 0.972 min (analysis condition FA05-1, 299 nm)

### Compound E05

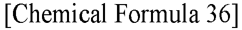

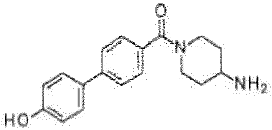

LRMS: m/z 297 [M+H]⁺
Retention time: 0.557 min (analysis condition FA05-1, 299 nm)

### Example 1-2-7: Synthesis of compound D06-2R

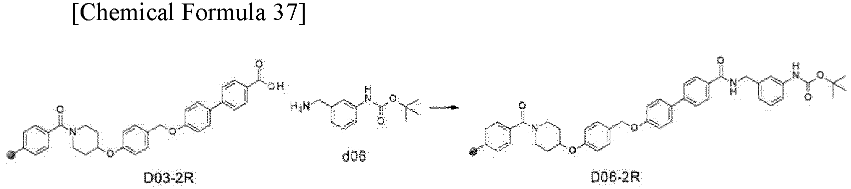

To a 50 mL Biotage column with a filter, D03-2R (0.201 mmol/g, 2.50 g) and DCM (37.5 mL) were added, and then the column was capped to prevent leakage of the reaction solution and shaken at room temperature for 30 minutes. To this, tert-butyl N-[3-(aminomethyl)phenyl]carbamate (d06) (0.224 g, 1.01 mmol), PipClU (0.363 g, 1.01 mmol) and 1-methylimidazole (0.160 mL, 2.01 mmol) were added, and then the column was capped to prevent leakage of the reaction solution and shaken at room temperature for 2 hours. The reaction solution was discharged from the column and the solid phase was washed 1 time with NMP (50 mL), 3 times with NMP/water = 1/1 (50 mL), 3 times with NMP (50 mL), 3 times with MeOH (50 mL), 3 times with DCM (50 mL), and 3 times with heptane (50 mL), and the resulting solid phase was dried overnight under reduced pressure to obtain compound D06-2R (0.193 mmol/g, 2.85 g).

### Reaction tracking

10 µL of the suspension of the solid phase and the liquid phase was transferred to a tip with a filter and washed 3 times with DMF (0.1 mL), 3 times with MeOH (0.1 mL), and 3 times with DCM (0.1 mL). The resultant was immersed in a 10% TFA/DCM solution (0.2 M, 0.05 mL) of pentamethylbenzene for 1 minute, and washed with DMF (0.05 mL). The combined filtrates were diluted with MeCN (0.20 mL) and then subjected to LCMS measurement to measure the progress of the reaction. As a result, the target material D06 was observed as 96.2% and compound E06 in which the Boc-deprotection reaction was progressed by the cleavage solution (10% TFA/DCM) was observed as 3.6%.

Compound D06

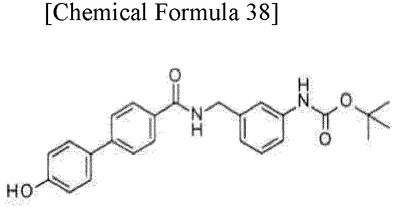

LRMS: m/z 363 [M-tBu+H]⁺
Retention time: 1.061 min (analysis condition FA05-1, 299 nm).

Compound E06

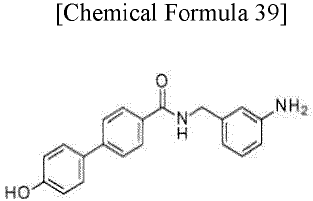

LRMS: m/z 319 [M+H]⁺
Retention time: 0.660 min (analysis condition FA05-1, 299 nm)

### Example 1-2-8: Synthesis of compound D11-3R

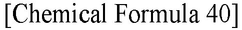

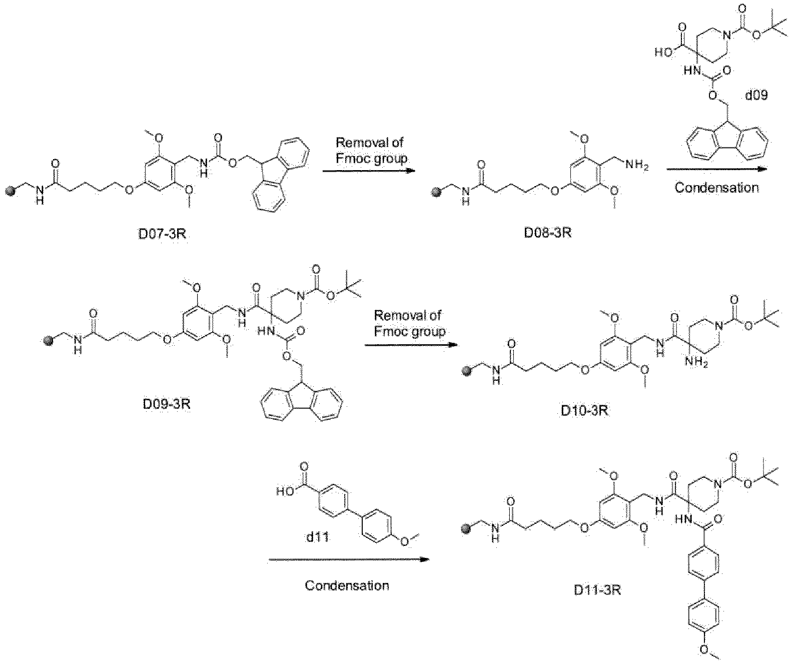

### First de-Fmoc step

Under a nitrogen atmosphere, to a 20 mL column with a filter, Fmoc-PAL AM resin (D07-3R, Novabiochem, Cat. No. 855133) (0.61 mmol/g, 1.00 g), DMF (12 mL), and piperidine (3 mL) were added. The column was capped to prevent leakage of the reaction solution and shaken at room temperature for 2 hours. After filtration, the resultant was washed 5 times with DMF (20 mL) to obtain D08-3R.

### First condensation step

Under a nitrogen atmosphere, N-Boc-4-(Fmoc-amino)piperidine-4-carboxylic acid (d09, CAS No. 183673-66-7) (0.569 g, 1.22 mmol), DMF (10 mL), DIPEA (0.426 mL, 2.44 mmol), and Pyoxim (0.643 g, 1.22 mmol) were added. The column was capped to prevent leakage of the reaction solution and shaken at room temperature for 15 hours. After filtration, the resultant was washed 5 times with DMF (20 mL) to obtain D09-3R.

### Second de-Fmoc step

Under a nitrogen atmosphere, DMF (12 mL) and piperidine (3 mL) were added, and then the column was capped to prevent leakage of the reaction solution and shaken at room temperature for 2.5 hours. After filtration, the resultant was washed 5 times with DMF (20 mL) to obtain D10-3R.

### Second condensation step

Under a nitrogen atmosphere, 4-(4-methoxyphenyl)benzoic acid (d11, CAS No. 725-14-4) (0.557 g, 2.44 mmol) and DMF (10 mL), DIPEA (0.852 mL, 4.88 mmol), and Pyoxim (1.287 g, 2.44 mmol) were added. The column was capped to prevent leakage of the reaction solution and shaken at room temperature all night. After filtration, the resultant was washed 3 times with DMF (20 mL), 3 times with MeOH (20 mL), 3 times with DCM (20 mL), and 1 time with pentane (20 mL). The resulting solid phase was dried overnight under reduced pressure to obtain D11-3R (1.01 g, 0.539 mmol/g).

After shaking for the second condensation, 12 µL of the suspension of the solid phase and the liquid phase was transferred to a tip with a filter and washed 3 times with DMF (0.1 mL), 3 times with MeOH (0.1 mL), and 3 times with DCM (0.1 mL). The resultant was immersed in a 30% TFA/HFIP solution (0.05 mL) for 5 minutes, filtered, and washed with DMF (0.05 mL). The combined filtrates were diluted with MeCN (0.25 mL) and then subjected to LCMS measurement to measure the progress of the reaction. As a result, compound E11 in which the Boc-deprotection reaction of the target material D11 was progressed by the cleavage solution (30% TFA/HFIP) was observed as 92.9%.

### Compound E11

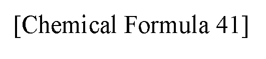

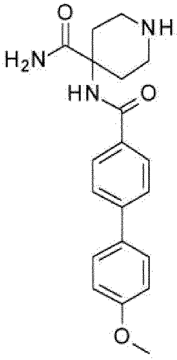

LRMS: m/z 354 [M+H]⁺
Retention time: 0.628 min (analysis condition FA05-1)

### Example 1-2-9: Synthesis of compound D11-4R

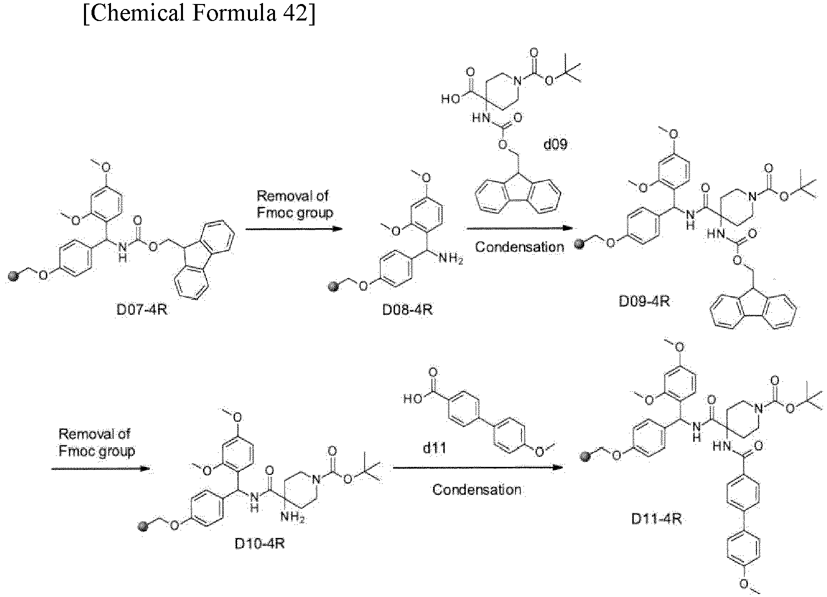

### First de-Fmoc step

Under a nitrogen atmosphere, to a 20 mL column with a filter, Rink Amide resin (D07-4R, Novabiochem, Cat. No. 855001) (0.54 mmol/g, 1.00 g), DMF (12 mL), and piperidine (3 mL) were added. The column was capped to prevent leakage of the reaction solution and shaken at room temperature for 30 minutes. After filtration, the resultant was washed 5 times with DMF (20 mL) to obtain D08-4R.

### First condensation step

Under a nitrogen atmosphere, N-Boc-4-(Fmoc-amino)piperidine-4-carboxylic acid (d09, CAS No. 183673-66-7) (0.504 g, 1.08 mmol), DMF (10 mL), DIPEA (0.189 mL, 1.08 mmol), and Pyoxim (0.570 g, 1.08 mmol) were added. The column was capped to prevent leakage of the reaction solution and shaken at room temperature for 17 hours. After filtration, the resultant was washed 5 times with DMF (20 mL) to obtain D09-4R.

### Second de-Fmoc step

Under a nitrogen atmosphere, DMF (12 mL) and piperidine (3 mL) were added, and then the column was capped to prevent leakage of the reaction solution and shaken at room temperature for 1.5 hours. After filtration, the resultant was washed 5 times with DMF (20 mL) to obtain D10-4R.

### Second condensation step

Under a nitrogen atmosphere, 4-(4-methoxyphenyl)benzoic acid (d11, CAS No. 725-14-4) (0.493 g, 2.16 mmol) and DMF (10 mL), DIPEA (0.377 mL, 2.16 mmol), and Pyoxim (1.14 g, 2.16 mmol) were added. The column was capped to prevent leakage of the reaction solution and shaken at room temperature for 21 hours. After filtration, the resultant was washed 3 times with DMF (20 mL), 3 times with MeOH (20 mL), 3 times with DCM (20 mL), and 1 time with pentane (20 mL). The resulting solid phase was dried overnight under reduced pressure to obtain D11-4R (1.13 g, 0.437 mmol/g).

After shaking for the second condensation, 12 µL of the suspension of the solid phase and the liquid phase was transferred to a tip with a filter and washed 3 times with DMF (0.1 mL), 3 times with MeOH (0.1 mL), and 3 times with DCM (0.1 mL). The resultant was immersed in a 30% TFA/HFIP solution (0.05 mL) for 5 minutes, filtered, and washed with DMF (0.05 mL). The combined filtrates were diluted with MeCN (0.25 mL) and then subjected to LCMS measurement to measure the progress of the reaction. As a result, compound E11 in which the Boc-deprotection reaction of the target material D11 was progressed by the cleavage solution (30% TFA/HFIP) was observed as 90.1%.

### Example 1-2-10: Synthesis of compound D11-5R

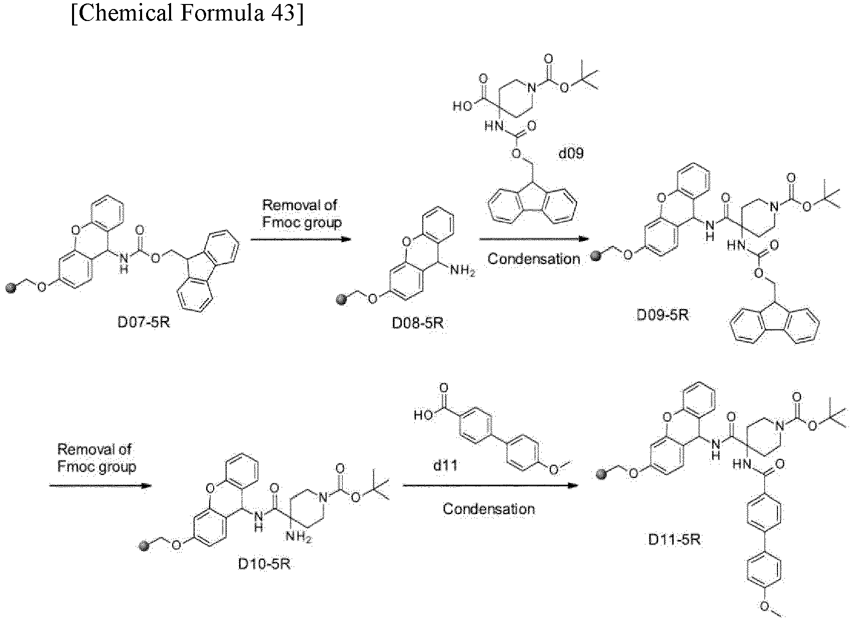

### First de-Fmoc step

Under a nitrogen atmosphere, to a 20 mL column with a filter, Sieber Amide resin (D07-5R, Novabiochem, Cat. No. 855008) (0.75 mmol/g, 1.00 g), DMF (12 mL), and piperidine (3 mL) were added. The column was capped to prevent leakage of the reaction solution and shaken at room temperature for 30 minutes. After filtration, the resultant was washed 5 times with DMF (20 mL) to obtain D08-5R.

### First condensation step

Under a nitrogen atmosphere, N-Boc-4-(Fmoc-amino)piperidine-4-carboxylic acid (d09, CAS No. 183673-66-7) (0.700 g, 1.50 mmol), DMF (10 mL), DIPEA (0.262 mL, 1.50 mmol), and Pyoxim (0.791 g, 1.50 mmol) were added. The column was capped to prevent leakage of the reaction solution and shaken at room temperature for 17 hours. After filtration, the resultant was washed 5 times with DMF (20 mL) to obtain D09-5R.

### Second de-Fmoc step

Under a nitrogen atmosphere, DMF (12 mL) and piperidine (3 mL) were added, and then the column was capped to prevent leakage of the reaction solution and shaken at room temperature for 1.5 hours. After filtration, the resultant was washed 5 times with DMF (20 mL) to obtain D10-5R.

### Second condensation step

Under a nitrogen atmosphere, 4-(4-methoxyphenyl)benzoic acid (d11, CAS No. 725-14-4) (0.685 g, 3.00 mmol), DMF (10 mL), DIPEA (0.524 mL, 3.00 mmol), and Pyoxim (1.58 g, 3.00 mmol) were added. The column was capped to prevent leakage of the reaction solution and shaken at room temperature for 21 hours. Under a nitrogen atmosphere, 4-(4-methoxyphenyl)benzoic acid (d11, CAS No. 725-14-4) (0.342 g, 1.50 mmol), DIPEA (0.524 mL, 3.00 mmol), and Pyoxim (0.79 g, 1.50 mmol) were added. The column was capped to prevent leakage of the reaction solution and shaken at room temperature for 1 hour. After filtration, the resultant was washed 3 times with DMF (20 mL), 3 times with MeOH (20 mL), 3 times with DCM (20 mL), and 1 time with pentane (20 mL). The resulting solid phase was dried overnight under reduced pressure to obtain D 11-SR (1.14 g, 0.437 mmol/g).

After shaking for the second condensation, 12 µL of the suspension of the solid phase and the liquid phase was transferred to a tip with a filter and washed 3 times with DMF (0.1 mL), 3 times with MeOH (0.1 mL), and 3 times with DCM (0.1 mL). The resultant was immersed in a 30% TFA/HFIP solution (0.05 mL) for 5 minutes, filtered, and washed with DMF (0.05 mL). The combined filtrates were diluted with MeCN (0.25 mL) and then subjected to LCMS measurement to measure the progress of the reaction. As a result, compound E11 in which the Boc-deprotection reaction of the target material D11 was progressed by the cleavage solution (30% TFA/HFIP) was observed as 98.4%.

### Example 2: Study of Boc-deprotection conditions

### Example 2-1: Study of Boc-deprotection conditions in liquid-phase reaction (Lewis acid-base combination)

### Example 2-1-1: Experiment to study Boc-deprotection conditions of 1-Boc-4-(4-bromophenyl)piperidine (B01) in presence of 1-methoxy-4-((4-(2-methoxyethyl)phenoxy)methyl)benzene (A02)

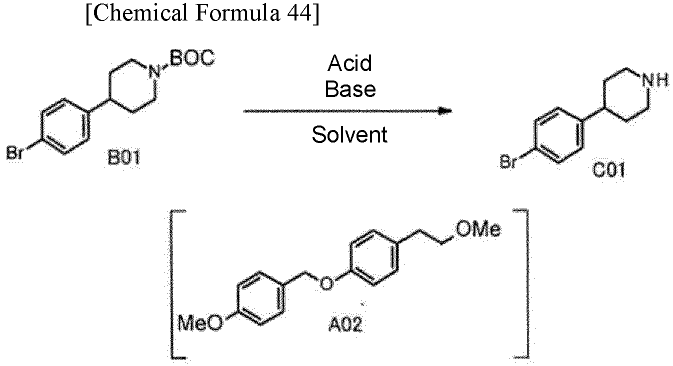

### Experimental procedures

Under a nitrogen atmosphere, to a 0.6 mL glass vial, 1-Boc-4-(4-bromophenyl)piperidine (B01) (6.8 mg, 0.020 mmol), 1-methoxy-4-((4-(2-methoxyethyl)phenoxy)methyl)benzene (A02) (5.5 mg, 0.020 mmol) and the solvent described in Table 2-1-1 (0.4 mL) were added. The base and acid described in Table 2-1-1 were added and the vial was shaken at the reaction temperature described in the table. However, for those with (a) attached to the yield, it is shaken at 25°C for 1 hour and then shaken at the reaction temperature described in the table. During the reaction, a part of the reaction solution was dispensed and then subjected to LCMS measurement to measure the progress of the reaction. The results are as shown in Table 2-1-1.

Regarding each notation "... %, ... %" in the table, the former represents the proportion of Boc-deprotected 1-Boc-4-(4-bromophenyl)piperidine (B01), that is the proportion calculated by "C01/(B01 + C01) × 100 (%)" in the UV area in the LCMS measurement. The latter represents the proportion of decomposed 1-methoxy-4-((4-(2-methoxyethyl)phenoxy)methyl)benzene (A02), that is the proportion calculated by "100 - A02/A02-derived compound × 100 (%)" in the UV area in the LCMS measurement. In practice, it was calculated by "100 - A02/(100 - (B01 + C01)) × 100 (%)" instead, because A02 produces complex decomposed products depending on the reaction conditions, while B01 produces nothing but Boc-deprotected C01. It should be noted that the analysis was performed by extracting at the wavelength of 305 nm (± 95 nm).

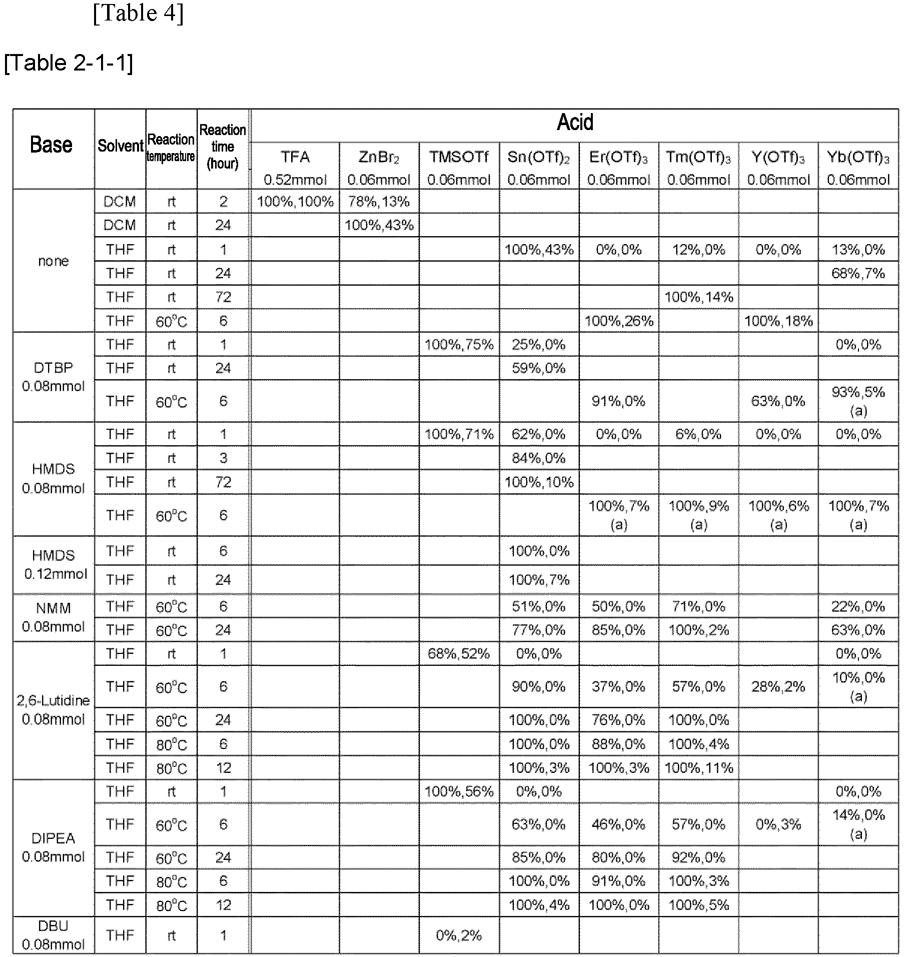

From the above results, it was shown that the Boc-deprotection reaction of secondary amine can be performed while suppressing the decomposition of the paraalkoxybenzyl ether structure by using Sn(OTf)₂, Y(OTf)₃, Er(OTf)₃, Tm(OTf)₃, or Yb(OTf)₃ in combination with a base, compared to using TFA, Sn(OTf)₂, ZnBr₂, or TMSOTf/2,6-lutidine that are conventionally used as Boc-deprotection conditions. When TFA was used, no selectivity was found between Boc-deprotection and decomposition of the paraalkoxybenzyl ether structure. Even when Sn(OTf)₂, which has been reported to be capable of performing a Boc-deprotection reaction while maintaining the tert-butyl ester structure and triisopropyl silyl ether structure (Synth. Commun. 2003, 33, 445-450.), was used alone without a base, the paraalkoxybenzyl ether structure was decomposed by 43% at room temperature for 1 hour. Even when ZnBr₂ (Synth. Commun. 1989, 19, 3139-3142.), which has been reported to be capable of gently performing a Boc-deprotection reaction, the para-alkoxybenzyl ether structure was decomposed by 43% before the Boc-deprotection reaction progressed 100%. Even when TMSOTf/2,6-lutidine conditions, which have been reported to be capable of performing a Boc-deprotection reaction while maintaining the tert-butyl ether structure, (J. Org. Chem. 1997, 62, 3880-3889.), and the combination of TMSOTf and other bases were investigated, the decomposition of the paraalkoxybenzyl ether structure could not be inhibited. On the other hand, when the combination of Sn(OTf)₂, Y(OTf)₃, Er(OTf)₃, Tm(OTf)₃, or Yb(OTf)₃ and a base was used, the decomposition of the paraalkoxybenzyl ether structure was suppressed to 10% or less while the Boc-deprotection reaction of secondary amine could be performed in high yield.

### 4-(4-Bromophenyl)piperidine (Compound C01)

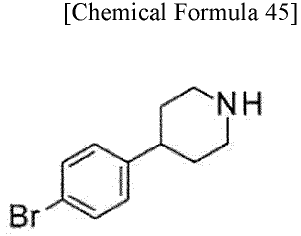

LRMS: m/z 240, 242 [M+H]⁺Retention time: 0.611 min (analysis condition FA05-1)

### Example 2-1-2: Experiment to study Boc-deprotection conditions of tert-butyl (4-bromophenethyl)carbamate (B02) in presence of 1-methoxy-4-((4-(2-methoxyethyl)phenoxy)methyl)benzene (A02)

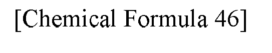

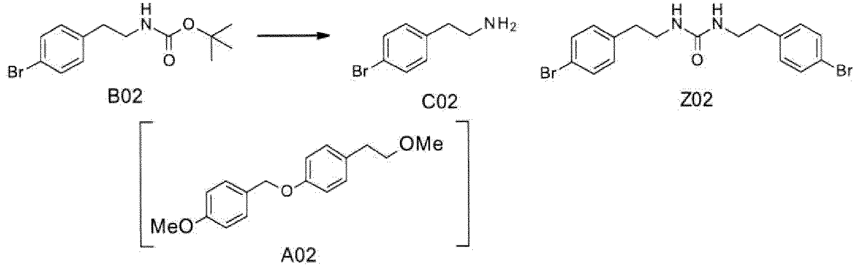

### Experimental procedures

Under a nitrogen atmosphere, to a 0.6 mL glass vial, tert-butyl (4-bromophenethyl)carbamate (B02) (3.0 mg, 0.010 mmol), 1-methoxy-4-((4-(2-methoxyethyl)phenoxy)methyl)benzene (A02) (2.7 mg, 0.010 mmol) and the solvent described in Table 2-1-2 (0.2 mL) were added. The base (0.045 mmol) and Lewis acid (0.030 mmol) described in Table 2-1-2 were added and the vial was shaken at the reaction temperature described in the table. During the reaction, a part of the reaction solution was dispensed and then subjected to LCMS measurement to measure the progress of the reaction. The results are as shown in Table 2-1-2.

Regarding each notation "... %, ... %" in the table, the former represents the proportion of Boc-deprotected tert-butyl (4-bromophenethyl)carbamate (B02), that is the proportion calculated by "C02/(B02 + C02 + Z02) × 100 (%)" in the UV area in the LCMS measurement. The latter represents the proportion of decomposed 1-methoxy-4-((4-(2-methoxyethyl)phenoxy)methyl)benzene (A02), that is the proportion calculated by "100 - A02/A02-derived compound × 100 (%)" in the UV area in the LCMS measurement. In practice, it was calculated by " 100 - A02/(100 - (B02 + C02 + Z02)) × 100 (%)" instead, because A02 produces complex decomposed products depending on the reaction conditions while B02 produces nothing but Boc-deprotected C02 and byproduct Z02 derived from C02. It should be noted that the analysis was performed by extracting at the wavelength of 305 nm (± 95 nm).

**[Table 5]**

| [Table 2-1-2] | | | | | | | | |
|---|---|---|---|---|---|---|---|---|
| Base | Solvent | Reaction temperature | Reaction time (hour) | Lewis acid | | | | |
| | | | | TMSOTf | Sn(OTf)₂ | Er(OTf)₃ | Tm(OTf)₃ | Yb(OTf)₃ |
| none | THF | 25°C | 1 | | 100%, 90% | | | |
| PhNMe₂ | THF | 80°C | 1 | | 98%, 3% | 100%, 1% | 95%, 1% | 82%, 0% |
| 2,6-Lutidine | THF | 25°C | 1 | 56%, 39% | | | | |
| 2,6-Lutidine | THF | 80°C | 6 | | 100%, 0% | 100%, 2% | 95%, 0% | 65%, 0% |
| NMM | THF | 80°C | 6 | | 100%, 1% | 100%, 1% | 77%, 0% | 81%, 0% |
| DIPEA | DCM | 25°C | 24 | | 36%, 7% | | | |
| DIPEA | THF | 80°C | 6 | | 59%, 0% | 100%, 0% | 87%, 0% | 89%, 0% |

From the above results, it was shown that the Boc-deprotection reaction of primary amine can be performed while suppressing the decomposition of the paraalkoxybenzyl ether structure by using Sn(OTf)₂, Er(OTf)₃, Tm(OTf)₃, or Yb(OTf)₃ in combination with a base, compared to using Sn(OTf)₂ or TMSOTf/2,6-lutidine that are conventionally used as Boc-deprotection conditions. Even when Sn(OTf)₂, which has been reported to be capable of performing a Boc-deprotection reaction while maintaining the tert-butyl ester structure and triisopropyl silyl ether structure (Synth. Commun. 2003, 33, 445-450.), was used alone without a base, the paraalkoxybenzyl ether structure was decomposed by 90% at 25°C for 1 hour. Even when TMSOTf/2,6-lutidine conditions, which have been reported to be capable of performing a Boc-deprotection reaction while maintaining the tert-butyl ether structure, (J. Org. Chem. 1997, 62, 3880-3889.) were investigated, the paraalkoxybenzyl ether structure was decomposed by 39% at 25°C for 1 hour while the Boc-deprotection reaction progressed only 56%. When Sn(OTf)₂/DIPEA/DCM conditions were used, the yield was no more than 36% at 25°C for 24 hours. On the other hand, when the combination of Sn(OTf)₂, Er(OTf)₃, Tm(OTf)₃, or Yb(OTf)₃ and a base was used and the reaction was heated, the decomposition of the paraalkoxybenzyl ether structure was suppressed to 3% or less while the Boc-deprotection reaction of primary amine could be performed in high yield.

### 2-(4-Bromophenyl)ethan-1-amine (Compound C02)

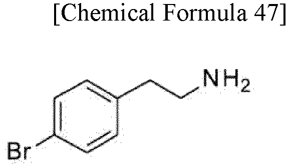

LRMS: m/z 200, 202 [M+H]⁺
Retention time: 2.336 min (analysis condition RPAmideTFA 10 cm).

### 1,3-Bis(4-bromophenethyl)urea (Compound Z02)

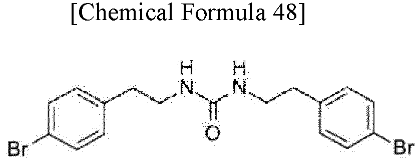

LRMS: m/z 427 [M+H]⁺
Retention time: 2.336 min (analysis condition RPAmideTFA 10 cm).

### Example 2-1-3: Experiment to study Boc-deprotection conditions of tert-butyl (6-bromonaphthalen-2-yl)carbamate (B03) in presence of 1-methoxy-4-((4-(2-methoxyethyl)phenoxy)methyl)benzene (A02)

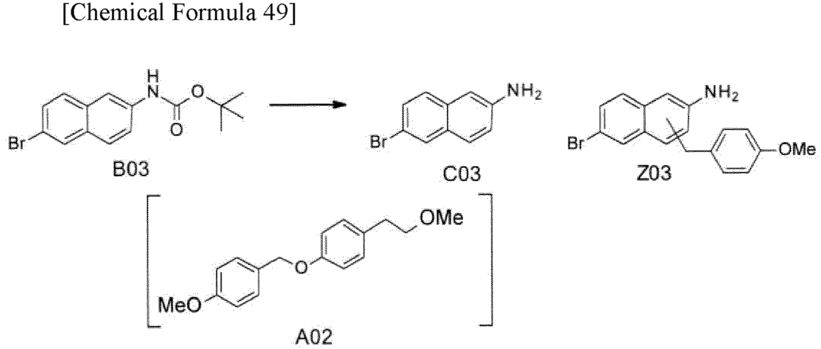

### Experimental procedures

Under a nitrogen atmosphere, to a 0.6 mL glass vial, tert-butyl (6-bromonaphthalen-2-yl)carbamate (B03) (3.2 mg, 0.010 mmol), 1-methoxy-4-((4-(2-methoxyethyl)phenoxy)methyl)benzene (A02) (2.7 mg, 0.010 mmol) and THF (0.2 mL) were added. The base (0.045 mmol) and Lewis acid (0.030 mmol) described in Table 2-1-3 were added and the vial was shaken at the reaction temperature described in the table. During the reaction, a part of the reaction solution was dispensed and then subjected to LCMS measurement to measure the progress of the reaction. The results are as shown in Table 2-1-3.

Regarding each notation "... %, ... %" in the table, the former represents the proportion of Boc-deprotected tert-butyl (6-bromonaphthalen-2-yl)carbamate (B03), that is the proportion calculated by "C03/(B03 + C03 + Z03) × 100 (%)" in the UV area in the LCMS measurement. The latter represents the proportion of decomposed 1-methoxy-4-((4-(2-methoxyethyl)phenoxy)methyl)benzene (A02), that is the proportion calculated by "100 - A02/A02-derived compound × 100 (%)" in the UV area in the LCMS measurement. In practice, it was calculated by "100 - A03/(100 - ((B03 + C03 + Z03)) × 100 (%)" instead, because A02 produces complex decomposed products depending on the reaction conditions, while B03 produces nothing but Boc-deprotected C03 and Z03 that is paramethoxybenzylated C03 (the paramethoxybenzylated position is undetermined). It should be noted that the analysis was performed by extracting at the wavelength of 305 nm (± 95 nm).

**[Table 6]**

| [Table 2-1-3] | | | | | |
|---|---|---|---|---|---|
| Base | Reaction temperature | Reaction time (hour) | Lewis acid | | |
| | | | TMSOTf | Sn(OTf)₂ | Er(OTf)₃ |
| none | 25°C | 1 | | 11%, 53% | |
| 2,6-Lutidine | 25°C | 1 | 99%, 48% | | |
| 2,6-Lutidine | 80°C | 12 | | 96%, 23% | 99%, 0% |
| NMM | 80°C | 12 | | 99%, 22% | 95%, 4% |
| DIPEA | 80°C | 12 | | 96%, 22% | 94%, 2% |

From the above results, it was shown that the Boc-deprotection reaction of aromatic amine can be performed while suppressing the decomposition of the paraalkoxybenzyl ether structure by using Sn(OTf)₂ or Er(OTf)₃ in combination with a base, compared to using Sn(OTf)₂ or TMSOTf/2,6-lutidine that are conventionally used as Boc-deprotection conditions. Even when Sn(OTf)₂, which has been reported to be capable of performing a Boc-deprotection reaction while maintaining the tert-butyl ester structure and triisopropyl silyl ether structure (Synth. Commun. 2003, 33, 445-450.), was used alone without a base, the paraalkoxybenzyl ether structure was decomposed by 53% at 25°C for 1 hour while the Boc-deprotection reaction progressed only 11%. Even when TMSOTf/2,6-lutidine conditions, which have been reported to be capable of performing a Boc-deprotection reaction while maintaining the tert-butyl ether structure (J. Org. Chem. 1997, 62, 3880-3889.), were investigated, the paraalkoxybenzyl ether structure was decomposed by 48% at 25°C for 1 hour. On the other hand, when the combination of Sn(OTf)₂ or Er(OTf)₃ and a base was used and the reaction was heated, the decomposition of the paraalkoxybenzyl ether structure was suppressed to 23% or less while the Boc-deprotection reaction of aromatic amine could be performed in high yield.

### 6-Bromonaphthalen-2-amine (Compound C03)

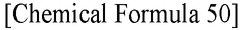

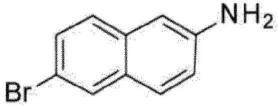

LRMS: m/z 222, 224 [M+H]⁺
Retention time: 0.980 min (analysis condition FA05-1)

### Example 2-1-4: Experiment to study Boc-deprotection reaction of 1-Boc-4-(4-bromophenyl)piperidine (B01) in presence of 1-methoxy-4-((4-(2-methoxyethyl)phenoxy)methyl)benzene (A02) using a rare-earth metal halide other than metal triflate

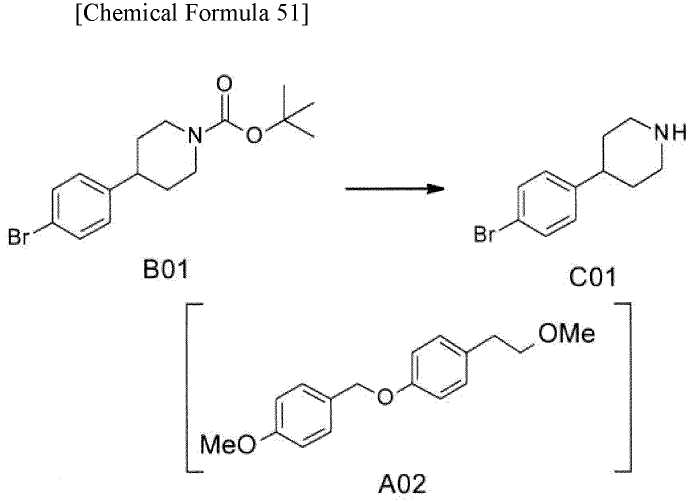

### Experimental procedures

Under a nitrogen atmosphere, to a 0.6 mL glass vial, 1-Boc-4-(4-bromophenyl)piperidine (B01) (3.4 mg, 0.010 mmol), 1-methoxy-4-((4-(2-methoxyethyl)phenoxy)methyl)benzene (A02) (2.7 mg, 0.010 mmol) and THF (0.2 mL) were added. 2,6-Lutidine (5.2 µL, 0.045 mmol) and Lewis acid (0.030 mmol) described in Table 2-1-4 were added, and the vial was shaken at 80°C. During the reaction, a part of the reaction solution was dispensed and then subjected to LCMS measurement to measure the progress of the reaction. The results are as shown in Table 2-1-4.

The target material (C01) (%) in the table represents the proportion of Boc-deprotected 1-Boc-4-(4-bromophenyl)piperidine (B01), that is the proportion calculated by "C01/(B01 + C01) × 100 (%)" in the UV area in the LCMS measurement. The decomposition of A02 (%) represents the proportion of decomposed 1-methoxy-4-((4-(2-methoxyethyl)phenoxy)methyl)benzene (A02), that is the proportion calculated by "100 - A02/A02-derived compound × 100 (%)" in the UV area in the LCMS measurement. In practice, it was calculated by "100 - A02/(100 - (B01 + C01)) × 100 (%)" instead, because A02 produces complex decomposed products depending on the reaction conditions, while B01 produces nothing but Boc-deprotected C01. It should be noted that the analysis was performed by extracting at the wavelength of 305 nm (± 95 nm).

**[Table 7]**

| [Table 2-1-4] | | | |
|---|---|---|---|
| Lewis acid | Reaction time (hour) | Target material (C01) (%) | Decomposition of A02 (%) |
| ErCl₃ | 12 | 95 | 0 |
| Erl₃ | 3 | 68 | 3 |

From the above results, it was shown that, even when the combination of rare earth metal chloride or rare earth metal iodide such as ErCl₃ or ErI₃ and a base was used, the decomposition of the paraalkoxybenzyl ether structure was suppressed to 3% or less while the Boc-deprotection reaction of amine could be performed in high yield.

### Example 2-1-5: Study of solvent in Boc-deprotection reaction of 1-Boc-4-(4-bromophenyl)piperidine (B01) in presence of 1-methoxy-4-((4-(2-methoxyethyl)phenoxy)methyl)benzene (A02)

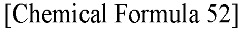

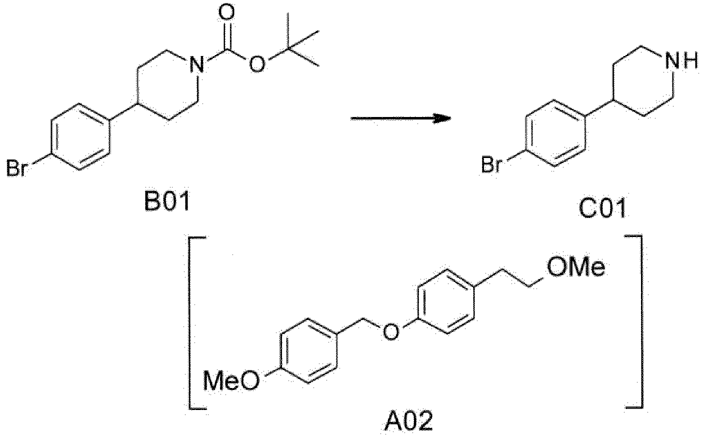

### Experimental procedures

Under a nitrogen atmosphere, to a 0.6 mL glass vial, 1-Boc-4-(4-bromophenyl)piperidine (B01) (3.2 mg, 0.010 mmol), 1-methoxy-4-((4-(2-methoxyethyl)phenoxy)methyl)benzene (A02) (2.7 mg, 0.010 mmol) and the solvent described in Table 2-1-5 (0.2 mL) were added. 2,6-Lutidine (5.2 µL, 0.045 mmol) and Er(OTf)₂ (18 mg, 0.030 mmol) were added and the vial was shaken at 80°C. During the reaction, a part of the reaction solution was dispensed and then subjected to LCMS measurement to measure the progress of the reaction. The results are as shown in Table 2-1-5.

The target material (C01) (%) in the table represents the proportion of Boc-deprotected 1-Boc-4-(4-bromophenyl)piperidine (B01), that is the proportion calculated by "C01/(B01 + C01) × 100 (%)" in the UV area in the LCMS measurement. The decomposition of A02 (%) represents the proportion of decomposed 1-methoxy-4-((4-(2-methoxyethyl)phenoxy)methyl)benzene (A02), that is the proportion calculated by "100 - A02/A02-derived compound × 100 (%)" in the UV area in the LCMS measurement. In practice, it was calculated by "100 - A02/(100 - (B01 + C01)) × 100 (%)" instead, because A02 produces complex decomposed products depending on the reaction conditions, while B01 produces nothing but Boc-deprotected C01. It should be noted that the analysis was performed by extracting at the wavelength of 305 nm (± 95 nm).

**[Table 8]**

| [Table 2-1-5] | | | |
|---|---|---|---|
| Solvent | Reaction time (hour) | Target material (C01) (%) | Decomposition of A02 (%) |
| Ethyl acetate | 12 | 81 | 1 |
| 1,2-Dichloroethane | 3 | 95 | 2 |
| 1,4-Dioxane | 3 | 96 | 4 |
| 1,2-Dimethoxyethane | 12 | 97 | 0 |
| Acetonitrile | 3 | 42 | 4 |
| 2-Methyl-2-butanol | 3 | 86 | 9 |

From the above results, it was shown that, even in the cases of using an ester-based solvent such as ethyl acetate, a halogen-based solvent such as 1,2-dichloroethane, an ether-based solvent such as 1,4-dioxane or 1,2-dimethoxyethane, a nitrile-based solvent such as acetonitrile, or an alcohol-based solvent such as 2-methyl-2-butanol as a solvent, when the combination of Er(OTf)₃ and a base was used, the decomposition of the paraalkoxybenzyl ether structure was suppressed to 9% or less while the Boc-deprotection reaction of amine could be performed in high yield.

### Example 2-1-6: Study of solvent in Boc-deprotection reaction of 1-Boc-4-(4-bromophenyl)piperidine (B01) in presence of 1-r3,5-bis(trifluoromethyl)benzoyl1-4-rr4-(4-methoxyphenyl)benzoyl]amino]-N-[(2,4,6-trimethoxyphenyl)methyl]piperidine-4-carboxamide (I09)

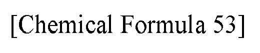

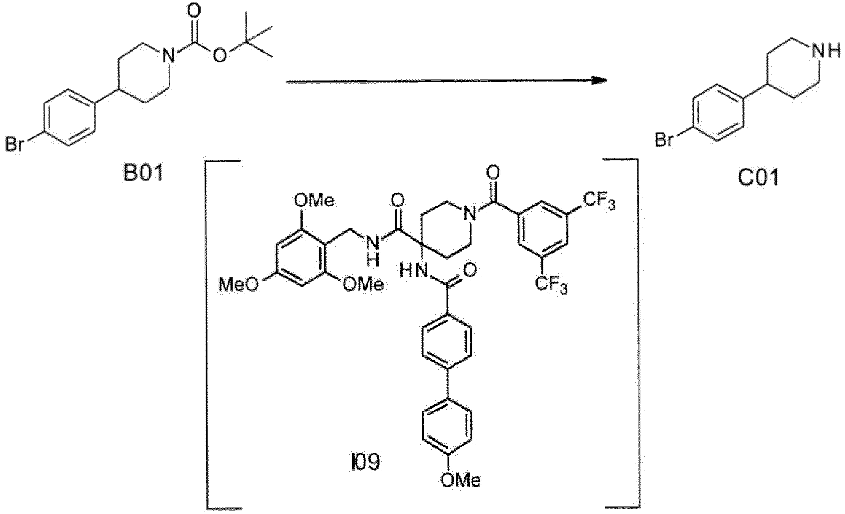

### Experimental procedures

Under a nitrogen atmosphere, to a 0.6 mL glass vial, 1-Boc-4-(4-bromophenyl)piperidine (B01) (0.85 mg, 0.0025 mmol), 1-[3,5-bis(trifluoromethyl)benzoyl]-4-[[4-(4-methoxyphenyl)benzoyl]amino]-N-[(2,4,6-trimethoxyphenyl)methyl]piperidine-4-carboxamide (I09) (1.9 mg, 0.0025 mmol) and the solvent described in Table 2-1-6 (0.05 mL) were added. The acid and base described in Table 2-1-6 were added and the vial was shaken at the reaction temperature described in Table 2-1-6. During the reaction, a part of the reaction solution was dispensed and then subjected to LCMS measurement to measure the progress of the reaction. The results are as shown in Table 2-1-6.

The target material (C01) (%) in the table represents the proportion of Boc-deprotected 1-Boc-4-(4-bromophenyl)piperidine (B01), that is the proportion calculated by "C01/(B01 + C01) × 100 (%)" in the UV area in the LCMS measurement. The decomposition(%) of I09 represents the proportion of decomposed 1-[3,5-bis(trifluoromethyl)benzoyl]-4-[[4-(4-methoxyphenyl)benzoyl]amino]-N-[(2,4,6-trimethoxyphenyl)methyl]piperidine-4-carboxamide (I09), that is the proportion calculated by " 100 - I09/I09-derived compound × 100 (%)" in the UV area in the LCMS measurement. In practice, it was calculated by " 100 - I09/(100 - (B01 + C01)) × 100 (%)" instead, because I09 produces complex decomposed products depending on the reaction conditions, while B01 produces nothing but Boc-deprotected C01. It should be noted that the analysis was performed by extracting at the wavelength of 305 nm (± 95 nm).

**[Table 9]**

| Acid | Base | Solvent | Reaction temperature | Reaction time (hour) | Target material (C01) | Decomposition of I09 |
|---|---|---|---|---|---|---|
| TFA (0.005 mL) | | DCM (0.05 mL) | 25°C | 1 | 100% | 76% |
| Er(OTf)₃ (15.4 mg, 0.025 mmol) | 2,6-Lutidine (4.4 µL, 0.038 mmol) | THF (0.05 mL) | 80°C | 24 | 100% | 8% |
| Tm(OTf)₃ (15.4 mg, 0.025 mmol) | 2,6-Lutidine (4.4 µL, 0.038 mmol) | THF (0.05 mL) | 80°C | 24 | 100% | 3% |
| Yb(OTf)₃ (15.5 mg, 0.025 mmol) | 2,6-Lutidine (4.4 µL, 0.038 mmol) | THF (0.05 mL) | 80°C | 24 | 100% | 4% |

From the above results, it was shown that, even in the presence of I09, which is decomposed by 77% at room temperature for 1 hour with 10% TFA/DCM, when the combination of Er(OTf)₃, Tm(OTf)₃, or Yb(OTf)₃ and a base was used and the reaction was heated, the decomposition of I09 was suppressed to 8% or less while the Boc-deprotection reaction of B01 could be performed in high yield. That is, it was shown that the combination of Er(OTf)₃, Tm(OTf)₃, or Yb(OTf)₃ and a base enables the Boc-deprotection reaction of amine while suppressing the decomposition of the acid-labile trialkoxybenzylaminocarbonyl structure.

### Example 2-1-7: Study of solvent in Boc-deprotection reaction of 1-Boc-4-(4-bromophenyl)piperidine (B01) in presence of 1-[3,5-bis(trifluoromethyl)benzoyl]-4-[[4-(4-methoxyphenyl)benzoyl]amino]-N-[(2,4-dimethoxyphenyl)-(4-methoxyphenyl)methyl]-4-[[4-(4-methoxyphenyl)benzoyl]amino]piperidine-4-carboxamide (I12)

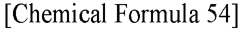

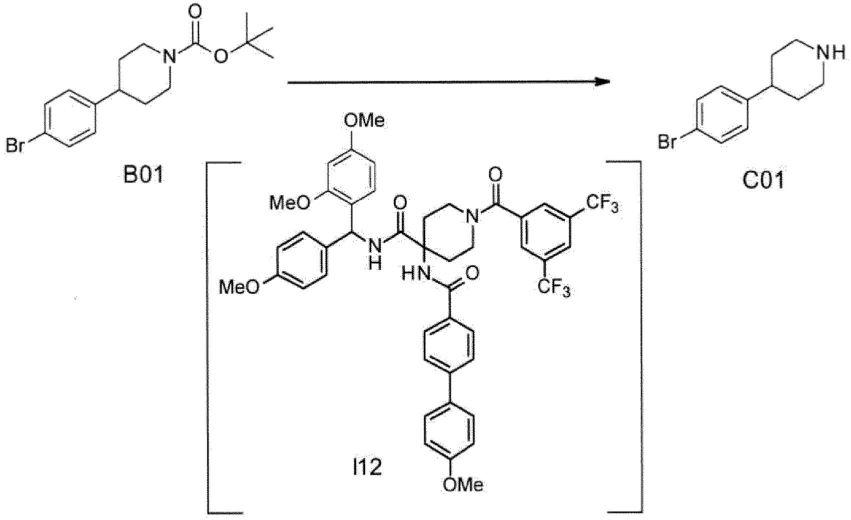

### Experimental procedures

Under a nitrogen atmosphere, to a 0.6 mL glass vial, 1-Boc-4-(4-bromophenyl)piperidine (B01) (0.85 mg, 0.0025 mmol), 1-[3,5-bis(trifluoromethyl)benzoyl]-4-[[4-(4-methoxyphenyl)benzoyl]amino]-N-[(2,4-dimethoxyphenyl)-(4-methoxyphenyl)methyl]-4-[[4-(4-methoxyphenyl)benzoyl]amino]piperidine-4-carboxamide (I12) (2.1 mg, 0.0025 mmol) and the solvent described in Table 2-1-7 (0.05 mL) were added. The acid and base described in Table 2-1-7 were added and the vial was shaken at the reaction temperature described in Table 2-1-7. During the reaction, a part of the reaction solution was dispensed and then subjected to LCMS measurement to measure the progress of the reaction. The results are as shown in Table 2-1-7.

The target material (C01) (%) in the table represents the proportion of Boc-deprotected 1-Boc-4-(4-bromophenyl)piperidine (B01), that is the proportion calculated by "C01/(B01 + C01) × 100 (%)" in the UV area in the LCMS measurement. The decomposition(%) of I12 represents the proportion of decomposed 1-[3,5-bis(trifluoromethyl)benzoyl]-4-[[4-(4-methoxyphenyl)benzoyl]amino]-N-[(2,4-dimethoxyphenyl)-(4-methoxyphenyl)methyl]-4-[[4-(4-methoxyphenyl)benzoyl]amino]piperidine-4-carboxamide (I12), that is the proportion calculated by "100 - I12/I12-derived compound × 100 (%)" in the UV area in the LCMS measurement. In practice, it was calculated by "100 - I12/(100 - (B01 + C01)) × 100 (%)" instead, because I12 produces complex decomposed products depending on the reaction conditions, while B01 produces nothing but Boc-deprotected C01. It should be noted that the analysis was performed by extracting at the wavelength of 305 nm (± 95 nm).

**[Table 10]**

| Acid | Base | Solvent | Reaction temperature | Reaction time (hour) | Target material (C01) | Decomposition of I12 |
|---|---|---|---|---|---|---|
| TFA (0.005 mL) | | DCM (0.05 mL) | 25°C | 1 | 100% | 100% |
| Sn(OTf)₂ (10.4 mg, 0.025 mmol) | 2,6-Lutidine (4.4 µL, 0.038 mmol) | THF (0.05 mL) | 60°C | 24 | 100% | 3% |
| Er(OTf)₃ (15.4 mg, 0.025 mmol) | 2,6-Lutidine (4.4 µL, 0.038 mmol) | THF (0.05 mL) | 80°C | 24 | 100% | 1% |
| Tm(OTf)₃ (15.4 mg, 0.025 mmol) | 2,6-Lutidine (4.4 µL, 0.038 mmol) | THF (0.05 mL) | 80°C | 24 | 100% | 6% |
| Yb(OTf)₃ (15.5 mg, 0.025 mmol) | 2,6-Lutidine (4.4 µL, 0.038 mmol) | THF (0.05 mL) | 80°C | 24 | 100% | 9% |

From the above results, it was shown that, even in the presence of I12, which is decomposed by 100% at room temperature for 1 hour with 10% TFA/DCM, when the combination of Sn(OTf)₂, Er(OTf)₃, Tm(OTf)₃, or Yb(OTf)₃ and a base was used and the reaction was heated, the decomposition of I12 was suppressed to 9% or less while the Boc-deprotection reaction of B01 could be performed in high yield. That is, it was shown that the combination of Er(OTf)₃, Tm(OTf)₃, or Yb(OTf)₃ and a base enables the Boc-deprotection reaction of amine while suppressing the decomposition of the acid-labile dialkoxyphenyl-alkoxyphenylmethylaminocarbonyl structure.

### Example 2-2: Study of Boc-deprotection conditions in solid-phase reactions (Lewis acid-base combination)

### Example 2-2-1: Study of Boc-deprotection conditions using D04-1R

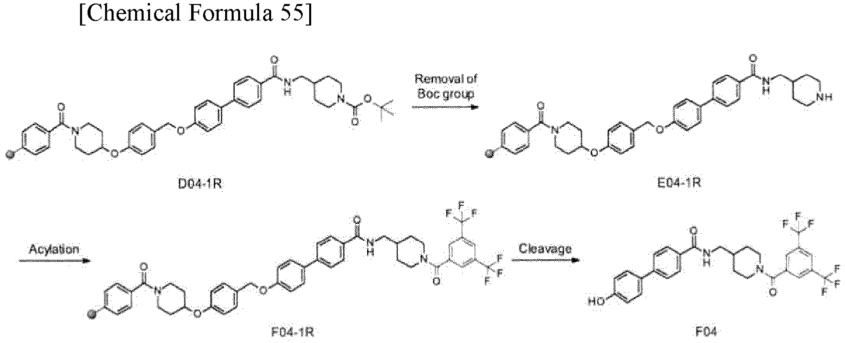

### Experimental procedures

### From Boc-deprotection reaction to washing

Under a nitrogen atmosphere, to a 1.5 mL glass vial, D04-1R (0.193 mmol/g, 50.0 mg) and the solvent (1.0 mL) described in Table 2-2-1 were added. The base and acid described in the table were added and the vial was shaken at the reaction temperature described in the table for 20 hours. The whole of the suspension of the solid phase and the liquid phase was transferred to a 3 mL column with a filter and washed 6 times with an NMP solution of DMEDA (0.2 M, 1 mL), 3 times with NMP (1 mL), 3 times with an NMP solution of mixed TBAHSO4 and DTBP (both 0.05 M, 1 mL), 3 times with an NMP solution of DIPEA (0.05 M, 1 mL), 3 times with NMP/water = 1/1 (1 mL), 3 times with NMP (1 mL), 3 times with MeOH (1.2 mL), and 3 times with DCM (1.2 mL).

### From acylation to washing

In an evaluation of this reaction, there is a concern that the Boc-deprotection reaction may progress even under the cleavage conditions from the solid phase. To properly evaluate the Boc-deprotection reaction, the amino groups exposed on the solid phase were acylated with 3,5-bis(trifluoromethyl)benzoyl chloride, and the amount thereof was quantified.

To a 3 mL column with a filter containing the resulting solid phase, DCM (1 mL) and DIPEA (0.051 mL, 0.290 mmol) were added. 3,5-Bis(trifluoromethyl)benzoyl chloride (0.035 mL, 0.193 mmol) was added, and then the column was capped to prevent leakage of the reaction solution and shaken at room temperature for 30 minutes.

12 µL of the suspension of the solid phase and the liquid phase was transferred to a tip with a filter and washed 3 times with DMF (0.1 mL), 3 times with MeOH (0.1 mL), and 3 times with DCM (0.1 mL). The resultant was immersed in a 10% TFA/DCM solution (0.02 M, 0.05 mL) of pentamethylbenzene for 1 minute, filtered, and washed with DMF (0.05 mL). The combined filtrates were diluted with MeCN (0.25 mL) and then subjected to LCMS measurement to measure the progress of the reaction. The results are as shown in Table 2-2-1. The progress of the Boc-deprotection reaction is represented by the UV area% of the acylated product. It should be noted that the analysis was performed by extracting at the wavelength of 299 nm (± 4 nm).

Subsequently, the whole of the suspension of the solid phase and the liquid phase was filtered, then washed 3 times with NMP/water = 1/1 (1 mL), 3 times with NMP (1 mL), 3 times with MeOH (1 mL), and 3 times with DCM (1 mL).

From cleavage to quantitation by ¹⁹F-NMR

To a 3 mL column with a filter containing the resulting solid phase, a 10% TFA/DCM solution of pentamethylbenzene (0. 1 M, 1.0 mL) was added. After standing still for 5 minutes, the mixture was filtered, and the same procedure was repeated 3 times. The resultant was washed 3 times with DMF (0.5 mL) and the combined filtrates were distilled off under reduced pressure (using a Genevac centrifugal evaporator).

To the resulting residue, DMSO-d6 (0.5 to 0.7 mL) and fluorobenzene (10.9 µL, 0.116 mmol) as an internal standard were added. The yield of the acylated product (F04) was calculated by performing a ¹⁹F-NMR measurement and comparing the result with the F amount of fluorobenzene. The results are as shown in Table 2-2-1.

**[Table 11]**

| [Table 2-2-1] | | | | | | |
|---|---|---|---|---|---|---|
| Run | Acid | Base | Solvent | Temperature | Yield of acylated product | UV area% of acylated product |
| 1 | TFA (0.1 mL) | none | DCM | rt | 0% | Not observed |
| 2 | TMSOTf (35.0 µL, 0.193 mmol) | 2,6-lutidine (33.7 µL, 0.290 mmol) | DCM | rt | 10% | 83% |
| 3 | TMSOTf (35.0 µL, 0.193 mmol) | 2,6-lutidine (33.7 µL, 0.290 mmol) | 2-MeTHF | rt | 17% | 90% |
| 4 | Sn(OTf)₂ (80.0 mg, 0.193 mmol) | none | 2-MeTHF | rt | 6% | 91% |
| 5 | Sn(OTf)₂ (80.0 mg, 0.193 mmol) | 2,6-lutidine (33.7 µL, 0.290 mmol) | 2-MeTHF | 60°C | 98% | 81% |
| 6 | Y(OTf)₃ (103 mg, 0.193 mmol) | DTBP (63.7 µL, 0.290 mmol) | 2-MeTHF | 60°C | 45% | 84% |
| 7 | Er(OTf)₃ (119 mg, 0.193 mmol) | DTBP (63.7 µL, 0.290 mmol) | 2-MeTHF | 60°C | 66% | 95% |
| 8 | Tm(OTf)₃ (119 mg, 0.193 mmol) | DTBP (63.7 µL, 0.290 mmol) | 2-MeTHF | 60°C | 29% | 92% |
| 9 | Yb(OTf)₃ (120 mg, 0.193 mmol) | DTBP (63.7 µL, 0.290 mmol) | 2-MeTHF | 60°C | 33% | 93% |
| 11 | Er(OTf)₃ (119 mg, 0.193 mmol) | 2,6-lutidine (33.7 µL, 0.290 mmol) | 2-MeTHF | 80°C | 94% | 92% |
| 12 | Tm(OTf)₃ (119 mg,0.193 mmol) | 2,6-lutidine (33.7 µL, 0.290 mmol) | 2-MeTHF | 80°C | 27% | 95% |
| 13 | Yb(OTf)₃ (120 mg, 0.193 mmol) | 2,6-lutidine (33.7 µL, 0.290 mmol) | 2-MeTHF | 80°C | 52% | 94% |
| 15 | Er(OTf)₃ (119 mg, 0.193 mmol) | NMM (31.8 µL, 0.290 mmol) | 2-MeTHF | 80°C | 61% | 92% |
| 16 | Tm(OTf)₃ (119 mg, 0.193 mmol) | NMM (31.8 µL, 0.290 mmol) | 2-MeTHF | 80°C | 49% | 92% |
| 17 | Yb(OTf)₃ (120 mg, 0.193 mmol) | NMM (31.8 µL, 0.290 mmol) | 2-MeTHF | 80°C | 56% | 95% |

From the above results, it was shown that the Boc-deprotection reaction of secondary amine supported on the solid phase progresses well in yield by using Sn(OTf)₂, Y(OTf)₃, Er(OTf)₃, Tm(OTf)₃, or Yb(OTf)₃ in combination with a base, compared to using TFA, Sn(OTf)₂ or TMSOTf/2,6-lutidine that are conventionally used as Boc-deprotection conditions. When TFA was used, cleavage from the solid phase progressed and no acylated product was observed (Run 1). When TMSOTf/2,6-lutidine, which has been reported to be capable of performing a Boc-deprotection reaction while suppressing cleavage from the solid phase in the solid phase synthesis reaction using Rink amide resin (Tetrahedron Lett. 1998, 39, 7439-7442.), was used, the amount of byproducts was small and the acylated product was observed but cleavage from the solid phase occurred during the Boc-deprotection step, and the yield was as low as 10 to 17% (Runs 2 and 3). When Sn(OTf)₂, which has been reported to be capable of performing a Boc-deprotection reaction while maintaining the tert-butyl ester structure and triisopropyl silyl ether structure in liquid-phase reaction (Synth. Commun. 2003, 33, 445-450.), was used without a base, the acylated product was observed but cleavage from the solid phase occurred and the yield was as low as 6% (Run 4). On the other hand, when the combination of Sn(OTf)₂, Y(OTf)₃, Er(OTf)₃, Tm(OTf)₃, or Yb(OTf)₃ and a base was used (Runs 5 to 17), the acylated product of interest could be obtained with a higher yield (27 to 98%).

Compound F04

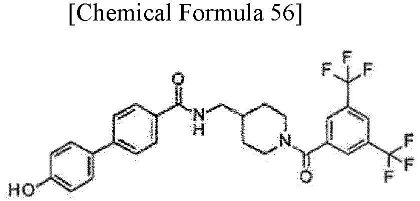

LRMS: m/z 551 [M+H]⁺
Retention time: 1.123 min (analysis condition FA05-1)
¹⁹F-NMR (376 MHz, DMSO-d6) δ: -61.3 (6F, s).

### Example 2-2-2: Study of Boc-deprotection conditions using D05-2R

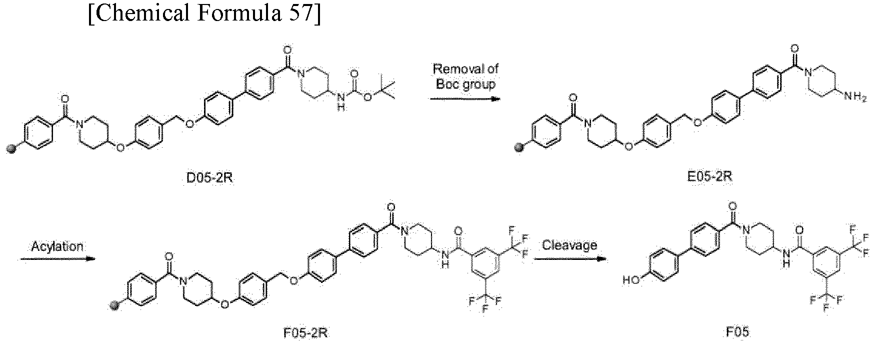

### Experimental procedures

### From Boc-deprotection reaction to washing

Under a nitrogen atmosphere, to a 1.5 mL glass vial, D05-2R (0.194 mmol/g, 50.0 mg) and 2-MeTHF (1.0 mL) were added. The base and acid described in the table were added and the vial was shaken at the reaction temperature and reaction time described in the table. 4,4'-Di-tert-butyl-2,2'-bipyridyl (78.0 mg, 0.291 mmol) and NMP (0.25 mL) were added and the vial was vigorously shaken. The whole of the suspension of the solid phase and the liquid phase was transferred to a column with a filter and washed 3 times with an NMP solution of 4,4'-di-tert-butyl-2,2'-bipyridyl (0.05 M, 1 mL), 3 times with an NMP solution of mixed TBAHSO4 and DTBP (both 0.05 M, 1 mL), 3 times with an NMP solution of DIPEA (0.05 M, 1 mL), 3 times with NMP (1 mL), 3 times with MeOH (1 mL), and 3 times with DCM (1 mL).

### From acylation reaction to washing

In an evaluation of this reaction, there is a concern that the Boc-deprotection reaction may progress even under the cleavage conditions from the solid phase. To properly evaluate the Boc-deprotection reaction, the amino groups exposed on the solid phase were acylated with 3,5-bis(trifluoromethyl)benzoyl chloride and the amount thereof was quantified.

To a column with a filter containing the resulting solid phase, DCM (1 mL) and DIPEA (0.051 mL, 0.291 mmol) were added. 3,5-Bis(trifluoromethyl)benzoyl chloride (0.035 mL, 0.194 mmol) was added, and then the column was capped to prevent leakage of the reaction solution and shaken at room temperature for 4 hours.

The whole of the suspension of the solid phase and the liquid phase was filtered, then washed 3 times with an NMP solution of DIPEA (0.05 M, 1 mL), 3 times with NMP (1 mL), 3 times with MeOH (1 mL), and 3 times with DCM (1 mL).

### From cleavage to quantitation by ¹⁹F-NMR

To a column with a filter containing the resulting solid phase, a 50% TFA/DCM solution of pentamethylbenzene (0.1 M, 1.0 mL) was added. After standing still for 5 minutes, the mixture was filtered. The resultant was washed 3 times with DMF (0.5 mL) and the combined filtrates were distilled off under reduced pressure (using a Genevac centrifugal evaporator).

To the resulting residue, DMSO-d6 (0.75 mL) and (trifluoromethoxy)benzene (6.4 µL, 0.049 mmol) as an internal standard were added. The yield of the acylated product (F05) was calculated by performing a ¹⁹F-NMR measurement and comparing the result with the F amount of (trifluoromethoxy)benzene. The results are as shown in Table 2-2-2.

**[Table 12]**

| [Table 2-2-2] | | | | | |
|---|---|---|---|---|---|
| Run | Acid | Base | Temperature | Reaction time (hour) | Yield of acylated product (F05) (19F NMR) |
| 1 | TMSOTf (35.1 µL, 0.194 mmol) | 2,6-lutidine (33.9 µL, 0.291 mmol) | 25°C | 12 | 6% |
| 2 | Sn(OTf)₂ (81.0 mg, 0.194 mmol) | none | 25°C | 12 | 3% |
| 3 | Sn(OTf)₂ (81.0 mg, 0.194 mmol) | 2,6-lutidine (33.9 µL, 0.291 mmol) | 60°C | 24 | 62% |
| 4 | Er(Otf)₃ (119 mg, 0.194 mmol) | 2,6-lutidine (33.9 µL, 0.291 mmol) | 60°C | 12 | 50% |
| 5 | Tm(OTf)₃ (120 mg, 0.194 mmol) | 2,6-lutidine (33.9 µL, 0.291 mmol) | 60°C | 24 | 52% |
| 6 | Yb(OTf)₃ (120 mg, 0.194 mmol) | 2,6-lutidine (33.9 µL, 0.291 mmol) | 60°C | 24 | 64% |

From the above results, it was shown that the Boc-deprotection reaction of primary amine supported on the solid phase progresses well in yield by using Sn(OTf)₂, Er(OTf)₃, Tm(OTf)₃, or Yb(OTf)₃ in combination with a base, compared to using Sn(OTf)₂ or TMSOTf/2,6-lutidine that are conventionally used as Boc-deprotection conditions. When TMSOTf/2,6-lutidine, which has been reported to be capable of performing a Boc-deprotection reaction while suppressing cleavage from the solid phase in the solid phase synthesis reaction using Rink amide resin (Tetrahedron Lett. 1998, 39, 7439-7442.), was used, cleavage from the solid phase occurred during the Boc-deprotection step, and the yield was as low as 6% (Run 1). When Sn(OTf)₂, which has been reported to be capable of performing a Boc-deprotection reaction while maintaining the tert-butyl ester structure and triisopropyl silyl ether structure in liquid-phase reaction (Synth. Commun. 2003, 33, 445-450.), was used without a base, cleavage from the solid phase occurred and the yield was as low as 3% (Run 2). On the other hand, when the combination of Sn(OTf)₂, Er(OTf)₃, Tm(OTf)₃, or Yb(OTf)₃ and a base was used (Runs 3 to 6), the acylated product of interest could be obtained with a higher yield (50 to 64%).

### Compound F05

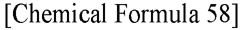

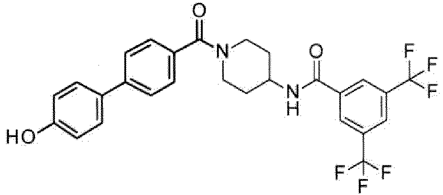

LRMS: m/z 537 [M+H]⁺
Retention time: 1.144 min (analysis condition FA05-1)
¹⁹F-NMR (376 MHz, DMSO-d6) δ: -62.7 (6F, s).

### Example 2-2-3: Study of Boc-deprotection conditions using D06-2R

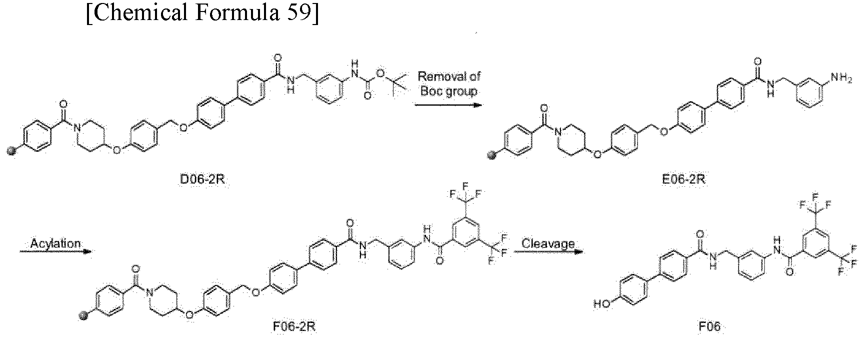

### Experimental procedures

### From Boc-deprotection reaction to washing

Under a nitrogen atmosphere, to a 1.5 mL glass vial, D06-2R (0.193 mmol/g, 50.0 mg) and 2-MeTHF (1.0 mL) were added. The base and acid described in the table were added and the vial was shaken at the reaction temperature and reaction time described in the table. 4,4'-Di-tert-butyl-2,2'-bipyridyl (78.0 mg, 0.290 mmol) and NMP (0.25 mL) were added and the vial was vigorously shaken. The whole of the suspension of the solid phase and the liquid phase was transferred to a column with a filter and washed 3 times with an NMP solution of 4,4'-di-tert-butyl-2,2'-bipyridyl (0.05 M, 1 mL), 3 times with an NMP solution of mixed TBAHSO4 and DTBP (both 0.05 M, 1 mL), 3 times with an NMP solution of DIPEA (0.05 M, 1 mL), 3 times with NMP (1 mL), 3 times with MeOH (1 mL), and 3 times with DCM (1 mL).

### From acylation to washing

In an evaluation of this reaction, there is a concern that the Boc-deprotection reaction may progress even under the cleavage conditions from the solid phase. To properly evaluate the Boc-deprotection reaction, the amino groups exposed on the solid phase were acylated with 3,5-bis(trifluoromethyl)benzoyl chloride, and the amount thereof was quantified.

To a column with a filter containing the resulting solid phase, DCM (1 mL) and DIPEA (0.051 mL, 0.290 mmol) were added. 3,5-Bis(trifluoromethyl)benzoyl chloride (0.035 mL, 0.193 mmol) was added, and then the column was capped to prevent leakage of the reaction solution. For Runs 1 and 2, after shaking at room temperature for 1 hour, DIPEA (0.051 mL, 0.290 mmol) and 3,5-bis(trifluoromethyl)benzoyl chloride (0.035 mL, 0.193 mmol) were added and the column was shaken at room temperature for 4 hours. For Runs 3 to 6, the column was shaken at room temperature for 4 hours.

Since some compounds had been partially diacylated, a hydrolysis reaction was performed to return them to the monoacylated product. NMP (0.7 mL) and 2-methyl-2-butanol (0.2 mL) were added. For Runs 1 and 2, an aqueous tetrabutylammonium hydroxide solution (1 M, 0.05 mL, 0.05 mmol) was added and the column was shaken at room temperature for 1 hour. For Runs 3 to 6, an aqueous tetrabutylammonium hydroxide solution (1 M, 0.1 mL, 0.1 mmol) was added and the column was shaken at room temperature for 1 hour.

The whole of the suspension of the solid phase and the liquid phase was filtered, then washed 3 times with an NMP solution of DIPEA (0.05 M, 1 mL), 3 times with NMP (1 mL), 3 times with MeOH (1 mL), and 3 times with DCM (1 mL).

### From cleavage to quantitation by ¹⁹F-NMR

To a column with a filter containing the resulting solid phase, a 50% TFA/DCM solution of pentamethylbenzene (0.1 M, 1.0 mL) was added. After standing still for 5 minutes, the mixture was filtered. The resultant was washed 3 times with DMF (0.5 mL) and the combined filtrates were distilled off under reduced pressure (using a Genevac centrifugal evaporator).

To the resulting residue, DMSO-d6 (0.75 mL) and (trifluoromethoxy)benzene (6.4 µL, 0.048 mmol) as an internal standard were added. The yield of the acylated product (F06) was calculated by performing a ¹⁹F-NMR measurement and comparing the result with the F amount of (trifluoromethoxy)benzene. The results are as shown in Table 2-2-3.

**[Table 13]**

| [Table 2-2-3] | | | | | |
|---|---|---|---|---|---|
| Run | Acid | Base | Temperature | Reaction time (hour) | Yield of acylated product (F06) (19F NMR) |
| 1 | TMSOTf (34.9 µL, 0.193 mmol) | 2,6-lutidine (33.7 µL, 0.290 mmol) | 25°C | 12 | 6% |
| 2 | Sn(OTf)₂ (80.0 mg, 0.193 mmol) | none | 25°C | 12 | 1% |
| 3 | Sn(OTf)₂ (80.0 mg, 0.193 mmol) | 2,6-lutidine (33.7 µL, 0.290 mmol) | 60°C | 24 | 48% |
| 4 | Er(OTf)₃ (119 mg, 0.193 mmol) | 2,6-lutidine (33.7 µL, 0.290 mmol) | 60°C | 12 | 49% |
| 5 | Tm(OTf)₃(119 mg, 0.193 mmol) | 2,6-lutidine (33.7 µL, 0.290 mmol) | 60°C | 24 | 48% |
| 6 | Yb(OTf)₃ (120 mg, 0.193 mmol) | 2,6-lutidine (33.7 µL, 0.290 mmol) | 60°C | 24 | 48% |

From the above results, it was shown that the Boc-deprotection reaction of aromatic amine supported on the solid phase progresses well in yield by using Sn(OTf)₂, Er(OTf)₃, Tm(OTf)₃, or Yb(OTf)₃ in combination with a base, compared to using Sn(OTf)₂ or TMSOTf/2,6-lutidine that are conventionally used as Boc-deprotection conditions. When TMSOTf/2,6-lutidine, which has been reported to be capable of performing a Boc-deprotection reaction while suppressing cleavage from the solid phase in the solid phase synthesis reaction using Rink amide resin (Tetrahedron Lett. 1998, 39, 7439-7442.), was used, cleavage from the solid phase occurred during the Boc-deprotection step, and the yield was as low as 6% (Run 1). When Sn(OTf)₂, which has been reported to be capable of performing a Boc-deprotection reaction while maintaining the tert-butyl ester structure and triisopropyl silyl ether structure in liquid-phase reaction (Synth. Commun. 2003, 33, 445-450.), was used without a base, cleavage from the solid phase occurred and the yield was as low as 1% (Run 2). On the other hand, when the combination of Sn(OTf)₂, Er(OTf)₃, Tm(OTf)₃, or Yb(OTf)₃ and a base was used (Runs 3 to 6), the acylated product of interest could be obtained with a higher yield (48 to 49%).

Compound F06

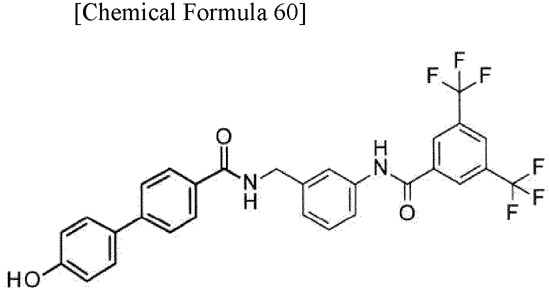

LRMS: m/z 559 [M+H]⁺
Retention time: 1.211 min (analysis condition FA05-1)
¹⁹F-NMR (376 MHz, DMSO-d6) δ: -62.7 (6F, s).

### Example 2-2-4: Study of Boc-deprotection conditions using Fmoc-PAL AM resin, D11-3R

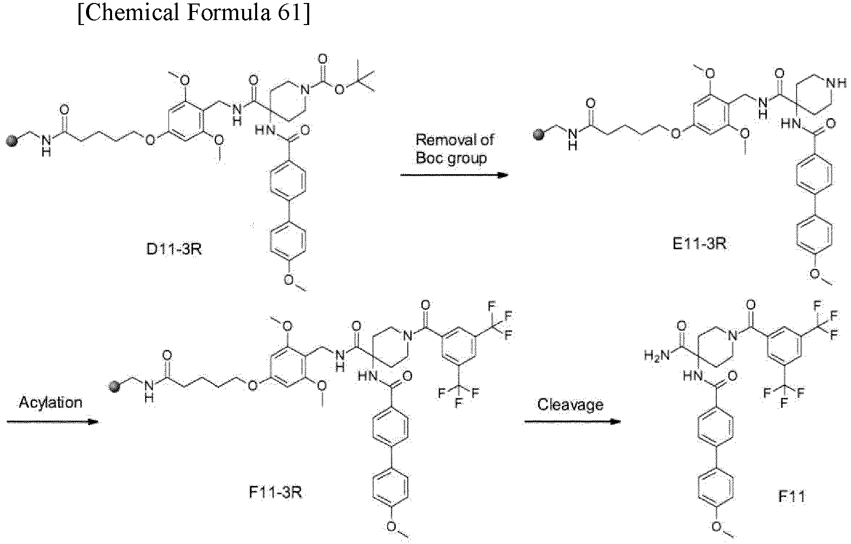

### Experimental procedures

### From Boc-deprotection to washing

Under a nitrogen atmosphere, to a 1.5 mL glass vial, D 11-3R (0.539 mmol/g, 20.0 mg) and 2-MeTHF (0.4 mL) were added. 2,6-Lutidine (14 µL, 0.12 mmol) and the acid (0.080 mmol) described in the table were added and the vial was shaken at the reaction temperature described in the table for 2 hours. 4,4'-Di-tert-butyl-2,2'-bipyridyl (32 mg, 0.12 mmol) and NMP (0.1 mL) were added and the vial was vigorously shaken. The whole of the suspension of the solid phase and the liquid phase was transferred to a column with a filter and washed 3 times with an NMP solution of 4,4'-di-tert-butyl-2,2'-bipyridyl (0.05 M, 0.4 mL), 3 times with an NMP solution of mixed TBAHSO4 and DTBP (both 0.05 M, 0.4 mL), 3 times with an NMP solution of DIPEA (0.05 M, 0.4 mL), 3 times with NMP (0.4 mL), 3 times with MeOH (0.4 mL), and 3 times with DCM (0.4 mL).

### From acylation reaction to washing

In an evaluation of this reaction, there is a concern that the Boc-deprotection reaction may progress even under the cleavage conditions from the solid phase. To properly evaluate the Boc-deprotection reaction, the amino groups exposed on the solid phase were acylated with 3,5-bis(trifluoromethyl)benzoyl chloride and the amount thereof was quantified.

To a column with a filter containing the resulting solid phase, DMF (0.4 mL) and DIPEA (0.021 mL, 0.12 mmol) were added. 3,5-Bis(trifluoromethyl)benzoyl chloride (14 µL, 0.080 mmol) was added, and then the column was capped to prevent leakage of the reaction solution and shaken at room temperature for 1 hour.

The whole of the suspension of the solid phase and the liquid phase was filtered, then washed 3 times with an NMP solution of DIPEA (0.05 M, 0.4 mL), 3 times with NMP (0.4 mL), 3 times with MeOH (0.4 mL), and 3 times with DCM (0.4 mL).

### From cleavage to quantitation by ¹⁹F-NMR

To a column with a filter containing the resulting solid phase, a solution of TFA/DCM/triisopropylsilane = 10/87.5/2.5 (0.4 mL) was added. After standing still for 10 minutes, the mixture was filtered into a test tube containing DMF (0.8 mL). The step of adding a solution of TFA/DCM/triisopropylsilane = 10/87.5/2.5 (0.4 mL), standing still for 10 minutes and then filtering was repeated two more times. A solution of TFA/DCM/triisopropylsilane = 10/87.5/2.5 (0.4 mL) was further added, and the mixture was stood still for 30 minutes and then filtered. The combined filtrates were distilled off under reduced pressure (using a Genevac centrifugal evaporator).

To the resulting residue, DMSO-d6 (0.7 mL) and 4-(trifluoromethoxy)toluene (6.4 µL, 0.043 mmol) as an internal standard were added. The yield of acylated product (F11) was calculated by performing a ¹⁹F-NMR measurement and comparing the result with the F amount of 4-(trifluoromethoxy)toluene. The results are as shown in Table 2-2-4.

**[Table 14]**

| [Table 2-2-4] | | | | | |
|---|---|---|---|---|---|
| Run | Lewis acid | Temperature | Reaction time (hour) | Yield of acylated product (F11) (19F NMR) | Yield of cyclized product (G11) (19F NMR) |
| 1 | TMSOTf (14.5 µL, 0.080 mmol) | 25°C | 2 | 0% | 80% |
| 2 | Sn(OTf)₂ (33.3 mg, 0.080 mmol) | 80°C | 2 | 75% | 0% |
| 3 | Er(OTf)₃ (49.2 mg, 0.080 mmol) | 80°C | 2 | 79% | 0% |

From the above results, it was shown that the Boc-deprotection reaction of amine supported on the PAL AM resin progresses well in yield by using Sn(OTf)₂ or Er(OTf)₃ in combination with a base, compared to using TMSOTf/2,6-lutidine that are conventionally used as Boc-deprotection conditions. When TMSOTf/2,6-lutidine, which has been reported to be capable of performing a Boc-deprotection reaction while suppressing cleavage from the solid phase in the solid phase synthesis reaction using Rink amide resin (Tetrahedron Lett. 1998, 39, 7439-7442.), was used, a cyclization reaction occurred during the Boc-deprotection step, and a cyclized product (G11) was observed in LCMS, but the acylated product of interest (F11) was not observed (Run 1). On the other hand, when the combination of Sn(OTf)₂ or Er(OTf)₃ and a base was used (Runs 2 and 3), the acylated product of interest (F11) could be obtained with a high yield (75 to 79%).

Compound F11

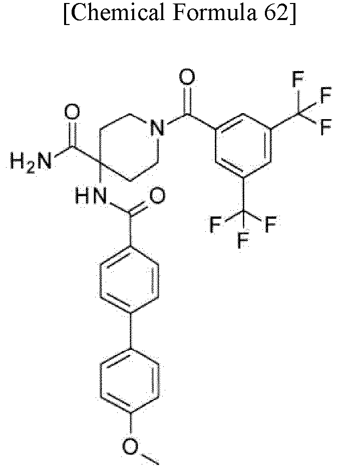

LRMS: m/z 594 [M+H]⁺
Retention time: 1.159 min (analysis condition FA05-1)

¹H-NMR (400 MHz, DMSO-d6) δ: 8.22 (1H (N-H), s), 8.20 (1H, brs), 8.16 (2H, brs), 7.99 (2H, d, J = 8.4 Hz), 7.73 (2H, d, J = 8.4 Hz), 7.70 (2H, dt, J = 9.5, 2.5 Hz), 7.16 (1H (N-H), brs), 7.06 (2H, dt, J = 9.5, 2.5 Hz), 6.97 (1H (N-H), brs), 4.26-4.22 (1H, m), 3.82 (3H, s), 3.45-3.39 (2H, m), 3.33-3.28 (1H, m), 2.38-2.34 (1H, m), 2.20-2.14 (1H, m), 2.08-2.00 (2H, m).

¹⁹F-NMR (376 MHz, DMSO-d6) δ: -61.3 (6F, s).

### Compound G11

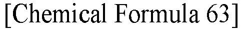

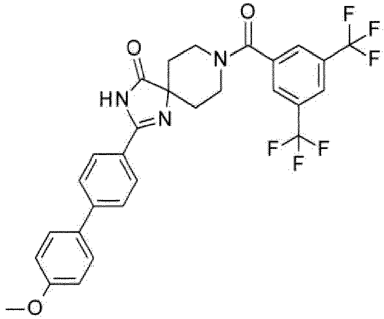

LRMS: m/z 576 [M+H]⁺
Retention time: 1.249 min (analysis condition FA05-1)
¹⁹F-NMR (376 MHz, DMSO-d6) δ: -61.3 (6F, s).

### Example 2-2-5: Study of Boc-deprotection conditions using Rink Amide Resin, D11-4R

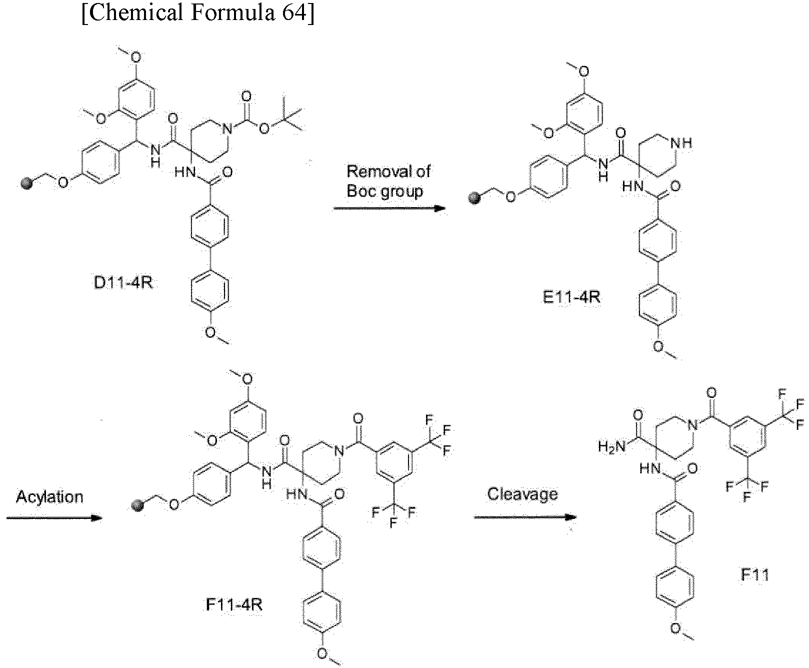

### Experimental procedures

### From Boc-deprotection reaction to washing

Under a nitrogen atmosphere, to a 1.5 mL glass vial, D11-4R (0.437 mmol/g, 20.0 mg) and 2-MeTHF (0.4 mL) were added. 2,6-Lutidine (14 µL, 0.12 mmol) and the acid (0.080 mmol) described in the table were added and the vial was shaken at the reaction temperature described in the table for 2 hours. 4,4'-Di-tert-butyl-2,2'-bipyridyl (32 mg, 0.12 mmol) and NMP (0.1 mL) were added and the vial was vigorously shaken. The whole of the suspension of the solid phase and the liquid phase was transferred to a column with a filter and washed 3 times with an NMP solution of 4,4'-di-tert-butyl-2,2'-bipyridyl (0.05 M, 0.4 mL), 3 times with an NMP solution of mixed TBAHSO4 and DTBP (both 0.05 M, 0.4 mL), 3 times with an NMP solution of DIPEA (0.05 M, 0.4 mL), 3 times with NMP (0.4 mL), 3 times with MeOH (0.4 mL), and 3 times with DCM (0.4 mL).

### From acylation reaction to washing

In an evaluation of this reaction, there is a concern that the Boc-deprotection reaction may progress even under the cleavage conditions from the solid phase. To properly evaluate the Boc-deprotection reaction, the amino groups exposed on the solid phase were acylated with 3,5-bis(trifluoromethyl)benzoyl chloride and the amount thereof was quantified.

To a column with a filter containing the resulting solid phase, DMF (0.4 mL) and DIPEA (0.021 mL, 0.12 mmol) were added. 3,5-Bis(trifluoromethyl)benzoyl chloride (14 µL, 0.080 mmol) was added, and then the column was capped to prevent leakage of the reaction solution and shaken at room temperature for 1 hour.

The whole of the suspension of the solid phase and the liquid phase was filtered, then washed 3 times with an NMP solution of DIPEA (0.05 M, 0.4 mL), 3 times with NMP (0.4 mL), 3 times with MeOH (0.4 mL), and 3 times with DCM (0.4 mL).

### From cleavage to quantitation by ¹⁹F-NMR

To a column with a filter containing the resulting solid phase, a solution of TFA/DCM/triisopropylsilane = 10/87.5/2.5 (0.4 mL) was added. After standing still for 10 minutes, the mixture was filtered into a test tube containing DMF (0.8 mL). The step of adding a solution of TFA/DCM/triisopropylsilane = 10/87.5/2.5 (0.4 mL), standing still for 10 minutes and then filtering was repeated two more times. A solution of TFA/DCM/triisopropylsilane = 10/87.5/2.5 (0.4 mL) was further added, and the mixture was stood still for 30 minutes and then filtered. The combined filtrates were distilled off under reduced pressure (using a Genevac centrifugal evaporator).

To the resulting residue, DMSO-d6 (0.7 mL) and 4-(trifluoromethoxy)toluene (5.2 µL, 0.035 mmol) as an internal standard were added. The yield of the acylated product (F11) was calculated by performing a ¹H-NMR measurement and comparing the H amount of the methyl group portion of 4-(trifluoromethoxy)toluene with the H amount of the methoxy group of the acylated product (F11). The results are as shown in Table 2-2-5.

**[Table 15]**

| [Table 2-2-5] | | | | |
|---|---|---|---|---|
| Run | Acid | Temperature | Reaction time (hour) | Yield of acylated product (F11) (1H NMR) |
| 1 | TMSOTf (14.5 µL, 0.080 mmol) | 25°C | 2 | 27% |
| 2 | Sn(OTf)₂ (33.3 mg, 0.080 mmol) | 80°C | 2 | 58% |
| 3 | Er(OTf)₃ (49.2 mg, 0.080 mmol) | 80°C | 2 | 62% |

From the above results, it was shown that the Boc-deprotection reaction of amine supported on the Rink Amide resin progresses well in yield by using Sn(OTf)₂ or Er(OTf)₃ in combination with a base, compared to using TMSOTf/2,6-lutidine that are conventionally used as Boc-deprotection conditions. When TMSOTf/2,6-lutidine, which has been reported to be capable of performing a Boc-deprotection reaction while suppressing cleavage from the solid phase in the solid phase synthesis reaction using Rink amide resin (Tetrahedron Lett. 1998, 39, 7439-7442.), was used, the yield was slightly lower of 27% (Run 1). On the other hand, when the combination of Sn(OTf)₂ or Er(OTf)₃ and a base was used (Runs 2 and 3), the acylated product of interest (F11) could be obtained with a higher yield (58 to 62%).

### Example 2-2-6: Study of Boc-deprotection conditions using Sieber Amide Resin, D11-5R

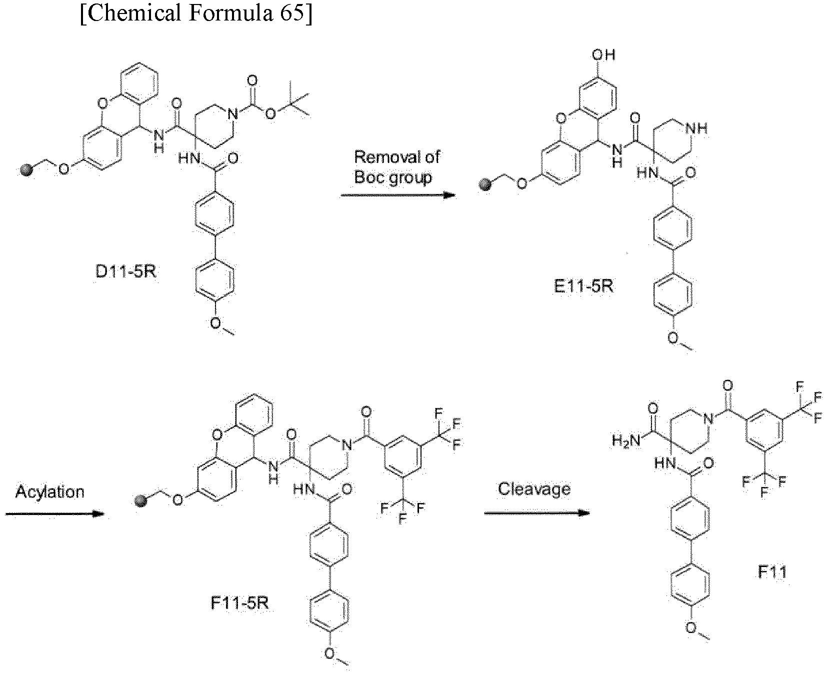

### Experimental procedures

### From Boc-deprotection reaction to washing

Under a nitrogen atmosphere, to a 1.5 mL glass vial, D11-5R (0.575 mmol/g, 20.0 mg) and 2-MeTHF (0.4 mL) were added. 2,6-Lutidine (14 µL, 0.12 mmol) and the acid (0.080 mmol) described in the table were added and the vial was shaken at the reaction temperature described in the table for 2 hours. 4,4'-Di-tert-butyl-2,2'-bipyridyl (32 mg, 0.12 mmol) and NMP (0.1 mL) were added and the vial was vigorously shaken. The whole of the suspension of the solid phase and the liquid phase was transferred to a column with a filter and washed 3 times with an NMP solution of 4,4'-di-tert-butyl-2,2'-bipyridyl (0.05 M, 0.4 mL), 3 times with an NMP solution of mixed TBAHSO4 and DTBP (both 0.05 M, 0.4 mL), 3 times with an NMP solution of DIPEA (0.05 M, 0.4 mL), 3 times with NMP (0.4 mL), 3 times with MeOH (0.4 mL), and 3 times with DCM (0.4 mL).

### From acylation reaction to washing

In an evaluation of this reaction, there is a concern that the Boc-deprotection reaction may progress even under the cleavage conditions from the solid phase. To properly evaluate the Boc-deprotection reaction, the amino groups exposed on the solid phase were acylated with 3,5-bis(trifluoromethyl)benzoyl chloride and the amount thereof was quantified.

To a column with a filter containing the resulting solid phase, DMF (0.4 mL) and DIPEA (0.021 mL, 0.12 mmol) were added. 3,5-Bis(trifluoromethyl)benzoyl chloride (14 µL, 0.080 mmol) was added, and then the column was capped to prevent leakage of the reaction solution and shaken at room temperature for 1 hour.

The whole of the suspension of the solid phase and the liquid phase was filtered, then washed 3 times with an NMP solution of DIPEA (0.05 M, 0.4 mL), 3 times with NMP (0.4 mL), 3 times with MeOH (0.4 mL), and 3 times with DCM (0.4 mL).

### From cleavage to quantitation by ¹⁹F-NMR

To a column with a filter containing the resulting solid phase, a solution of TFA/DCM/triisopropylsilane = 10/87.5/2.5 (0.4 mL) was added. After standing still for 10 minutes, the mixture was filtered into a test tube containing DMF (0.8 mL). The step of adding a solution of TFA/DCM/triisopropylsilane = 10/87.5/2.5 (0.4 mL), standing still for 10 minutes and then filtering was repeated two more times. A solution of TFA/DCM/triisopropylsilane = 10/87.5/2.5 (0.4 mL) was further added, and the mixture was stood still for 30 minutes and then filtered. The combined filtrates were distilled off under reduced pressure (using a Genevac centrifugal evaporator).

To the resulting residue, DMSO-d6 (0.7 mL) and 4-(trifluoromethoxy)toluene (6.9 µL, 0.046 mmol) as an internal standard were added. The yield of the acylated product (F11) was calculated by performing a ¹⁹F-NMR measurement and comparing the result with the F amount of 4-(trifluoromethoxy)toluene. The results are as shown in Table 2-2-6.

**[Table 16]**

| [Table 2-2-6] | | | | |
|---|---|---|---|---|
| Run | Acid | Temperature | Reaction time (hour) | Yield of acylated product (F11) (19F NMR) |
| 1 | TMSOTf (14.5 µL, 0.080 mmol) | 25°C | 2 | 16% |
| 2 | Sn(OTf)₂ (33.3 mg, 0.080 mmol) | 80°C | 2 | 62% |
| 3 | Er(OTf)₃ (49.2 mg, 0.080 mmol) | 80°C | 2 | 81% |

From the above results, it was shown that the Boc-deprotection reaction of amine supported on the Sieber Amide resin progresses well in yield by using Sn(OTf)₂ or Er(OTf)₃ in combination with a base, compared to using TMSOTf/2,6-lutidine that are conventionally used as Boc-deprotection conditions. When TMSOTf/2,6-lutidine, which has been reported to be capable of performing a Boc-deprotection reaction while suppressing cleavage from the solid phase in the solid phase synthesis reaction using Rink amide resin (Tetrahedron Lett. 1998, 39, 7439-7442.), was used, cleavage from the solid phase occurred during the Boc-deprotection step, and the yield was as low as 17% (Run 1). On the other hand, when the combination of Sn(OTf)₂ or Er(OTf)₃ and a base was used (Runs 2 and 3), the acylated product of interest (F1 1) could be obtained with a high yield (62 to 81%).

### Example 2-3: Substrate generality of Boc-deprotection reaction

The yields of the Boc-deprotection reaction in Examples 2-3-1 to 2-3-10 were calculated as the percentage of Boc-protected compound (B04 to B13) converted to Boc-deprotected compound (C04 to C13), that is the proportion calculated by "Boc-deprotected compound (C04 to C13)/(Boc-protected compound (B04 to B13)-derived compound) × 100 (%)" in the UV area in the LCMS measurement. The proportion of decomposed A02 was calculated by " 100 - A02/A02-derived compound × 100 (%)" as in Examples 2-1-1 to 2-1-5. It should be noted that the analysis was performed by extracting at the wavelength of 305 nm (± 95 nm).

### Example 2-3-1: Boc-deprotection reaction of tert-butyl 3-(4-bromophenyl)azetidine-1-carboxylate (B04) in presence of 1-methoxy-4-((4-(2-methoxyethyl)phenoxy)methyl)benzene (A02)

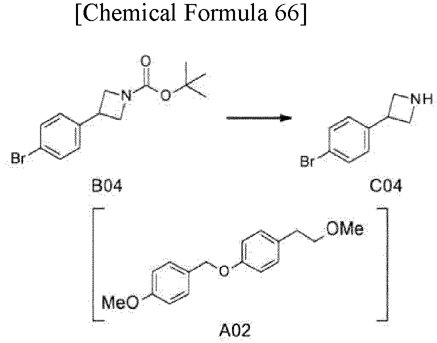

Under a nitrogen atmosphere, to a 0.6 mL glass vial, tert-butyl 3-(4-bromophenyl)azetidine-1-carboxylate (B04)(3.1 mg, 0.010 mmol), 1-methoxy-4-((4-(2-methoxyethyl)phenoxy)methyl)benzene (A02) (2.7 mg, 0.010 mmol) and THF (0.2 mL) were added. 2,6-Lutidine (5.2 µL, 0.045 mmol) and Er(OTf)₃ (18.4 mg, 0.030 mmol) were added and the vial was shaken at 80°C for 12 hours. As a result of the LCMS measurement, the Boc-deprotection reaction of interest progressed and 99% of C04 was obtained while only 5% of A02 was decomposed.

### 3-(4-Bromophenyl)azetidine (Compound C04)

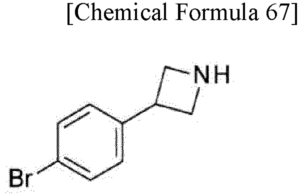

LRMS: m/z 212, 214 [M+H]⁺
Retention time: 0.548 min (analysis condition FA05-1)

### Example 2-3-2: Boc-deprotection reaction of tert-butyl 3-(4-bromophenyl)pyrrolidine-1-carboxylate (B05) in presence of 1-methoxy-4-((4-(2-methoxyethyl)phenoxy)methyl)benzene (A02)

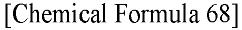

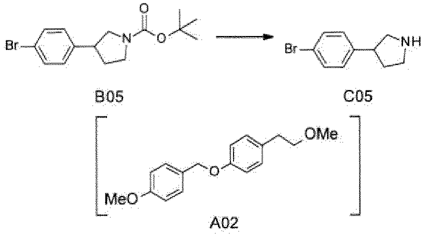

Under a nitrogen atmosphere, to a 0.6 mL glass vial, tert-butyl 3-(4-bromophenyl)pyrrolidine-1-carboxylate (B05) (3.3 mg, 0.010 mmol), 1-methoxy-4-((4-(2-methoxyethyl)phenoxy)methyl)benzene (A02) (2.7 mg, 0.010 mmol) and THF (0.2 mL) were added. 2,6-Lutidine (5.2 µL, 0.045 mmol) and Er(OTf)₃ (18.4 mg, 0.030 mmol) were added and the vial was shaken at 80°C for 12 hours. As a result of the LCMS measurement, the Boc-deprotection reaction of interest progressed and 100% of C05 was obtained while only 3% of A02 was decomposed.

### 3-(4-Bromophenyl)pyrrolidine (Compound C05)

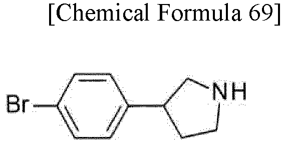

LRMS: m/z 226, 228 [M+H]⁺
Retention time: 0.589 min (analysis condition FA05-1)

### Example 2-3-3: Boc-deprotection reaction of tert-butyl 4-(4-bromophenyl)piperazine-1-carboxylate (B06) in presence of 1-methoxy-4-((4-(2-methoxyethyl)phenoxy)methyl)benzene (A02)

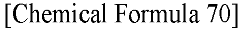

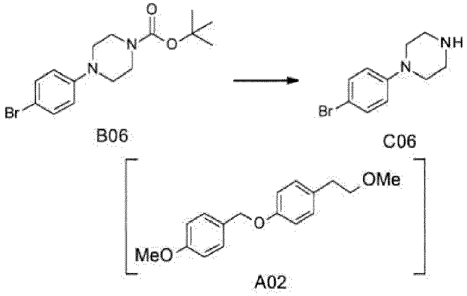

Under a nitrogen atmosphere, to a 0.6 mL glass vial, tert-butyl 4-(4-bromophenyl)piperazine-1-carboxylate (B06) (3.4 mg, 0.010 mmol), 1-methoxy-4-((4-(2-methoxyethyl)phenoxy)methyl)benzene (A02) (2.7 mg, 0.010 mmol) and THF (0.2 mL) were added. 2,6-Lutidine (5.2 µL, 0.045 mmol) and Er(OTf)₃ (18.4 mg, 0.030 mmol) were added and the vial was shaken at 80°C for 12 hours. As a result of the LCMS measurement, the Boc-deprotection reaction of interest progressed and 97% of C06 was obtained while only 4% of A02 was decomposed.

### 1-(4-Bromophenyl)piperazine (Compound C06)

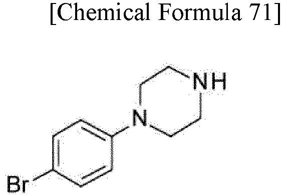

LRMS: m/z 241, 243 [M+H]⁺
Retention time: 0.603 min (analysis condition FA05-1)

### Example 2-3-4: Boc-deprotection reaction of tert-butyl (1-(4-bromophenyl)ethyl)carbamate (B07) in presence of 1-methoxy-4-((4-(2-methoxyethyl)phenoxy)methyl)benzene (A02)

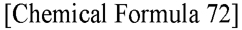

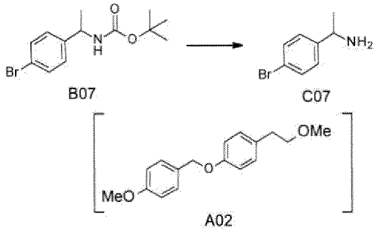

Under a nitrogen atmosphere, to a 0.6 mL glass vial, tert-butyl (1-(4-bromophenyl)ethyl)carbamate (B07) (3.0 mg, 0.010 mmol), 1-methoxy-4-((4-(2-methoxyethyl)phenoxy)methyl)benzene (A02) (2.7 mg, 0.010 mmol) and THF (0.2 mL) were added. 2,6-Lutidine (5.2 µL, 0.045 mmol) and Er(OTf)₃ (18.4 mg, 0.030 mmol) were added and the vial was shaken at 80°C for 12 hours. As a result of the LCMS measurement, the Boc-deprotection reaction of interest progressed and 100% of C07 was obtained while only 3% of A02 was decomposed.

### 1-(4-Bromophenyl)ethan-1-amine (Compound C07)

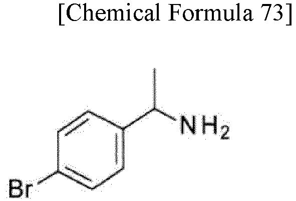

LRMS: m/z 200, 202 [M+H]⁺
Retention time: 0.511 min (analysis condition FA05-1)

### Example 2-3-5: Boc-deprotection reaction of tert-butyl (3-bromophenyl)carbamate (B08) in presence of 1-methoxy-4-((4-(2-methoxyethyl)phenoxy)methyl)benzene (A02)

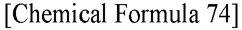

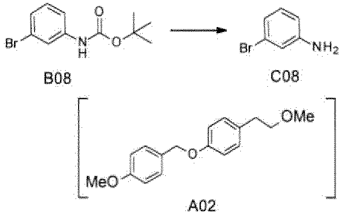

Under a nitrogen atmosphere, to a 0.6 mL glass vial, tert-butyl (3-bromophenyl)carbamate (B08) (2.7 mg, 0.010 mmol), 1-methoxy-4-((4-(2-methoxyethyl)phenoxy)methyl)benzene (A02) (2.7 mg, 0.010 mmol) and THF (0.2 mL) were added. 2,6-Lutidine (5.2 µL, 0.045 mmol) and Er(OTf)₃ (18.4 mg, 0.030 mmol) were added and the vial was shaken at 80°C for 12 hours. As a result of the LCMS measurement, the Boc-deprotection reaction of interest progressed and 100% of C08 was obtained while only 5% of A02 was decomposed.

### 3-Bromoaniline (Compound C08)

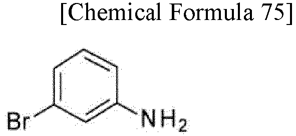

LRMS: m/z 172, 174 [M+H]⁺
Retention time: 0.759 min (analysis condition FA05-1)

### Example 2-3-6: Boc-deprotection reaction of tert-butyl (3-bromophenyl)(methyl)carbamate (B09) in presence of 1-methoxy-4-((4-(2-methoxyethyl)phenoxy)methyl)benzene (A02)

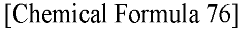

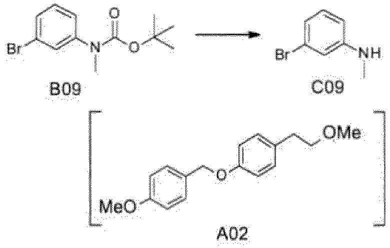

Under a nitrogen atmosphere, to a 0.6 mL glass vial, tert-butyl (3-bromophenyl)(methyl)carbamate (B09) (2.9 mg, 0.010 mmol), 1-methoxy-4-((4-(2-methoxyethyl)phenoxy)methyl)benzene (A02) (2.7 mg, 0.010 mmol) and THF (0.2 mL) were added. 2,6-Lutidine (5.2 µL, 0.045 mmol) and Er(OTf)₃ (18.4 mg, 0.030 mmol) were added and the vial was shaken at 80°C for 12 hours. As a result of the LCMS measurement, the Boc-deprotection reaction of interest progressed and 94% of C09 was obtained while only 6% of A02 was decomposed.

### 3-Bromo-N-methylaniline (Compound C09)

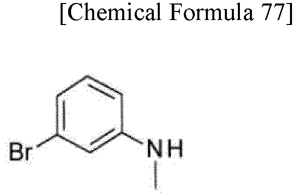

LRMS: m/z 186, 188 [M+H]⁺
Retention time: 1.016 min (analysis condition FA05-1)

### Example 2-3-7: Boc-deprotection reaction of tert-butyl (5-bromo-2-methylphenyl)carbamate (B10) in presence of 1-methoxy-4-((4-(2-methoxyethyl)phenoxy)methyl)benzene (A02)

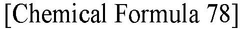

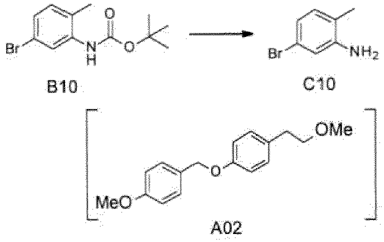

Under a nitrogen atmosphere, to a 0.6 mL glass vial, tert-butyl (5-bromo-2-methylphenyl)carbamate (B10) (2.9 mg, 0.010 mmol), 1-methoxy-4-((4-(2-methoxyethyl)phenoxy)methyl)benzene (A02) (2.7 mg, 0.010 mmol) and THF (0.2 mL) were added. 2,6-Lutidine (5.2 µL, 0.045 mmol) and Er(OTf)₃ (18.4 mg, 0.030 mmol) were added and the vial was shaken at 80°C for 12 hours. As a result of the LCMS measurement, the Boc-deprotection reaction of interest progressed and 100% of C10 was obtained while only 4% of A02 was decomposed.

### 5-Bromo-2-methylaniline (Compound C10)

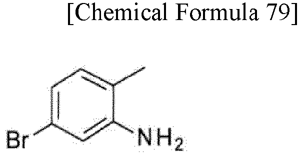

LRMS: m/z 186, 188 [M+H]⁺
Retention time: 0.933 min (analysis condition FA05-1)

### Example 2-3-8: Boc-deprotection reaction of tert-butyl (5-bromopyridin-3-yl)carbamate (B11) in presence of 1-methoxy-4-((4-(2-methoxyethyl)phenoxy)methyl)benzene (A02)

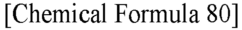

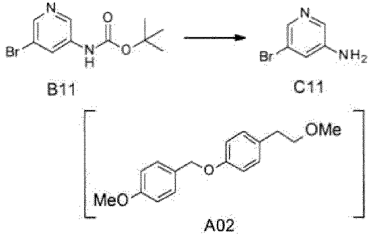

Under a nitrogen atmosphere, to a 0.6 mL glass vial, tert-butyl (5-bromopyridin-3-yl)carbamate (B11) (2.7 mg, 0.010 mmol), 1-methoxy-4-((4-(2-methoxyethyl)phenoxy)methyl)benzene (A02) (2.7 mg, 0.010 mmol) and THF (0.2 mL) were added. 2,6-Lutidine (5.2 µL, 0.045 mmol) and Er(OTf)₃ (18.4 mg, 0.030 mmol) were added and the vial was shaken at 80°C for 12 hours. As a result of the LCMS measurement, the Boc-deprotection reaction of interest progressed and 95% of C11 was obtained while only 3% of A02 was decomposed.

### 5-Bromopyridin-3-amine (Compound C11)

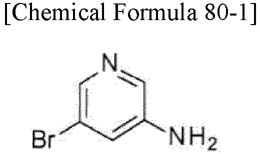

LRMS: m/z 173, 175 [M+H]⁺
Retention time: 0.268 min (analysis condition FA05-1)

### Example 2-3-9: Boc-deprotection reaction of tert-butyl (2-bromopyrimidin-5-yl)carbamate (B12) in presence of 1-methoxy-4-((4-(2-methoxyethyl)phenoxy)methyl)benzene (A02)

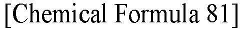

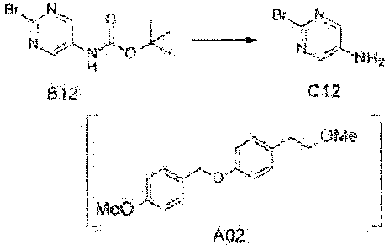

Under a nitrogen atmosphere, to a 0.6 mL glass vial, tert-butyl (2-bromopyrimidin-5-yl)carbamate(B12) (2.7 mg, 0.010 mmol), 1-methoxy-4-((4-(2-methoxyethyl)phenoxy)methyl)benzene (A02) (2.7 mg, 0.010 mmol) and THF (0.2 mL) were added. 2,6-Lutidine (5.2 µL, 0.045 mmol) and Er(OTf)₃ (18.4 mg, 0.030 mmol) were added and the vial was shaken at 80°C for 12 hours. As a result of the LCMS measurement, the Boc-deprotection reaction of interest progressed and 99% of C12 was obtained while only 3% of A02 was decomposed.

### 2-Bromopyrimidin-5-amine (Compound C12)

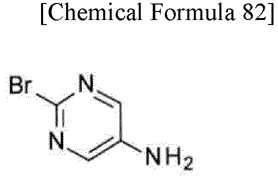

LRMS: m/z 174, 176 [M+H]⁺
Retention time: 0.387 min (analysis condition FA05-1)

### Example 2-3-10: Boc-deprotection reaction of tert-butyl (5-bromopyrazin-2-yl)carbamate (B13) in presence of 1-methoxy-4-((4-(2-methoxyethyl)phenoxy)methyl)benzene (A02)

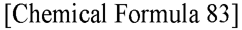

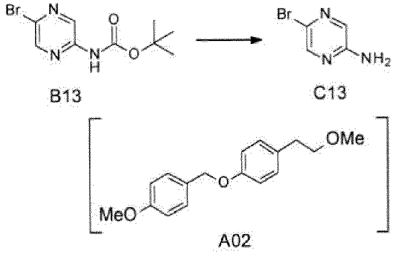

Under a nitrogen atmosphere, to a 0.6 mL glass vial, tert-butyl (5-bromopyrazin-2-yl)carbamate (B13) (2.7 mg, 0.010 mmol), 1-methoxy-4-((4-(2-methoxyethyl)phenoxy)methyl)benzene (A02) (2.7 mg, 0.010 mmol) and THF (0.2 mL) were added. 2,6-Lutidine (5.2 µL, 0.045 mmol) and Er(OTf)₃ (18.4 mg, 0.030 mmol) were added and the vial was shaken at 80°C for 12 hours. As a result of the LCMS measurement, the Boc-deprotection reaction of interest progressed and 98% of C13 was obtained while only 5% of A02 was decomposed.

### 5-Bromopyrazin-2-amine (Compound C13)

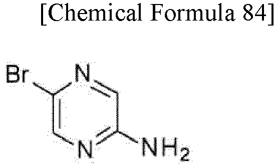

LRMS: m/z 174, 176 [M+H]⁺
Retention time: 0.557 min (analysis condition FA05-1)

### Example 3: Boc-deprotection reaction in mixture

### Example 3-1: Boc-deprotection reaction in mixture 2-1-b1C29-0

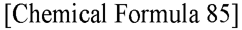

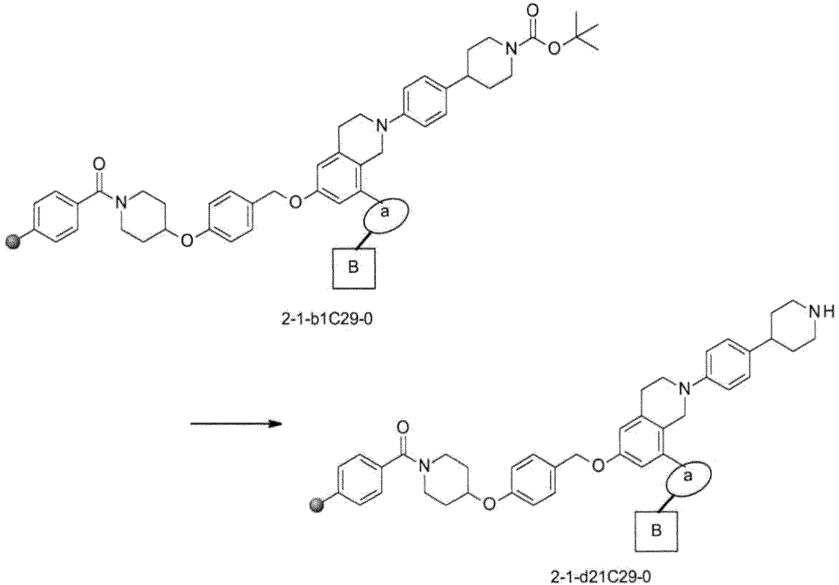

The compounds contained in the separately prepared mixture 2-1-b1C29-0 are as shown in [Table 3-1-1].

**[Table 17]**

| [Table 3-1-1] Compounds contained in mixture 2-1-b1C29-0 | | |
|---|---|---|
| **ID** | **a** | **B** |
| 2-1-b1C29-0-0001 | a2 | B11 |
| 2-1-b1C29-0-0002 | a2 | B12 |
| 2-1-b1C29-0-0003 | a2 | B13 |
| 2-1-b1C29-0-0004 | a2 | B14 |
| 2-1-b1C29-0-0005 | a1 | B01 |
| 2-1-b1C29-0-0006 | a1 | B02 |
| 2-1-b1C29-0-0007 | a2 | B15 |
| 2-1-b1C29-0-0008 | a2 | B16 |
| 2-1-b1C29-0-0009 | a1 | B03 |
| 2-1-b1C29-0-0010 | a2 | B17 |
| 2-1-b1C29-0-0011 | a1 | B04 |
| 2-1-b1C29-0-0012 | a1 | B05 |
| 2-1-b1C29-0-0013 | a1 | B06 |
| 2-1-b1C29-0-0014 | a2 | B18 |
| 2-1-b1C29-0-0015 | a1 | B07 |
| 2-1-b1C29-0-0016 | a2 | B19 |
| 2-1-b1C29-0-0017 | a1 | B08 |
| 2.1-b1C29-0-0018 | a2 | B20 |
| 2-1-b1C29-0-0019 | a1 | B09 |
| 2-1-b1C29-0-0020 | a1 | B10 |

In [Table 3-1-1], each compound contained in the mixture is represented by ID, and the structure is represented by indicating the combination of corresponding parts a and B in the formula by symbols. The correspondence between symbols of parts a and B and structural formulas is shown below.

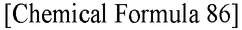

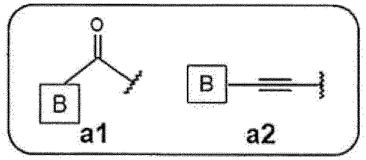

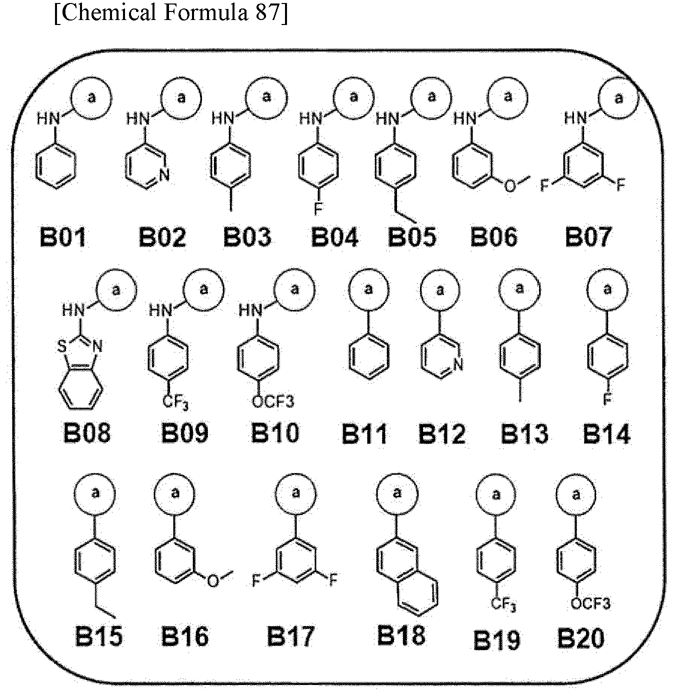

For example, compound 2-1-b1C29-0-0001 has a of a2 and B of B11 and is represented by the following structure.

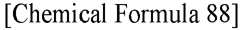

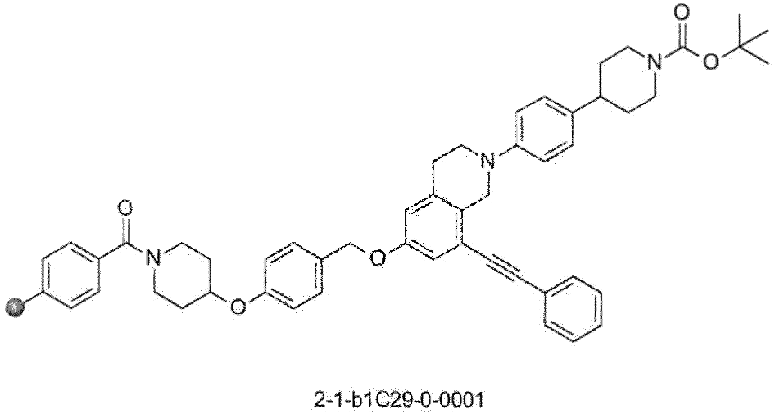

To a 4 mL glass vial, mixture 2-1-b1C29-0 (70 mg) and 2-MeTHF (1.4 mL) were added, and the vial was shaken at room temperature for 1 hour. DTBP (88 µL, 0.40 mmol) and Tm(OTf)₃ (165 mg, 0.267 mmol) were added and the vial was shaken at 60°C for 6 hours.

To the resulting reaction solution, an NMP solution of DMEDA (1 M, 0.669 mL) was added, and the vial was shaken at room temperature for 1.5 hours. The suspension of the reaction solution and solid phase was transferred to a 3 mL column with a filter, and filtered, and then washed 3 times with an NMP solution of DMEDA (0.2 M, 1.5 mL), 3 times with NMP (1.5 mL), 3 times with an NMP solution of mixed TBAHSO₄ and DTBP (both 0.05 M, 1.5 mL), 3 times with an NMP solution of BTMG (0.05 M, 1.5 mL), 3 times with an NMP solution of DTBP (0.05 M, 1.5 mL), 3 times with NMP/water = 1/1 (1.5 mL), 3 times with NMP (1.5 mL), 3 times with MeOH (1.5 mL), 3 times with DCM (1.5 mL), and 3 times with heptane (1.5 mL). The resulting solid phase was dried under reduced pressure to obtain mixture 2-1-d21C29-0.

The compounds that may be contained in the mixture 2-1-d21C29-0 are as shown in [Table 3-1-2]. In [Table 3-1-2], each compound that may be contained in the mixture is represented by ID, and the structure is represented by indicating the combination of corresponding parts a and B in the structural formula of the mixture by symbols. The correspondence between symbols of parts a and B and structural formulas is as described above.

**[Table 18]**

| [Table 3-1-2] Compounds that may be contained in mixture 2-1-d21C29-0 | | |
|---|---|---|
| I**D** | **a** | **B** |
| 2-1-d21C29-0-0001 | a2 | B11 |
| 2-1-d21C29-0-0002 | a2 | B12 |
| 2-1-d21C29-0-0003 | a2 | B13 |
| 2-1-d21C29-0-0004 | a2 | B14 |
| 2-1-d21C29-0-0005 | a1 | B01 |
| 2-1-d21C29-0-0006 | a1 | B02 |
| 2-1-d21C29-0-0007 | a2 | B15 |
| 2-1-d21C29-0-0008 | a2 | B16 |
| 2-1-d21C29-0-0009 | a1 | B03 |
| 2-1-d21C29-0-0010 | a2 | B17 |
| 2-1-d21C29-0-0011 | a1 | B04 |
| 2-1-d21C29-0-0012 | a1 | B05 |
| 2-1-d21C29-0-0013 | a1 | B06 |
| 2-1-d21C29-0-0014 | a2 | B18 |
| 2-1-d21C29-0-0015 | a1 | B07 |
| 2-1-d21C29-0-0016 | a2 | B19 |
| 2-1-d21C29-0-0017 | a1 | B08 |
| 2-1-d21C29-0-0018 | a2 | B20 |
| 2-1-d21C29-0-0019 | a1 | B09 |
| 2-1-d21C29-0-0020 | a1 | B10 |

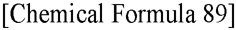

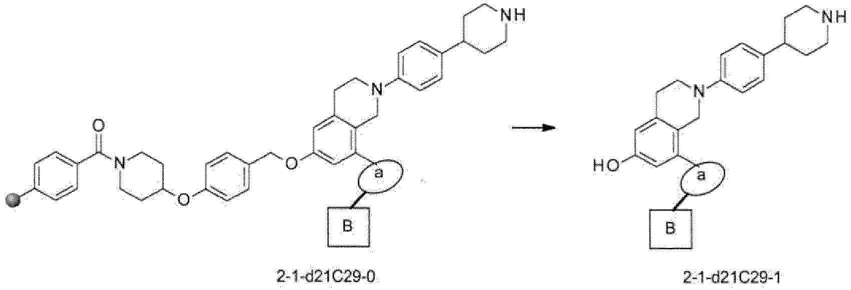

A small amount of mixture 2-1-d21C29-0 was transferred to a tip with a filter and washed 3 times with DMA (0.1 mL), 3 times with MeOH (0.1 mL), and 3 times with DCM (0.1 mL). The resultant was immersed in a 10% TFA/DCM solution (0.05 M, 0.05 mL) of pentamethylbenzene for 10 minutes, filtered, and washed with DMA (0.05 mL). The combined filtrates were diluted with MeCN (0.25 mL) to obtain a solution of mixture 2-1-d21C29-1. In LCMS measurements of this solution, all m/z ([M+H]⁺) derived from compounds that may be contained in the mixture 2-1-d21C29-1 described in [Table 3-1-3] were observed. In [Table 3-1-3], each compound that may be contained in the mixture is represented by ID, and the structure is represented by indicating the combination of corresponding parts a and B in the structural formula of the mixture by symbols. The correspondence between symbols of parts a and B and structural formulas is as described above. This result showed that the mixture 2-1-d21C29-0 described in [Table 3-1-2] was correctly synthesized.

**[Table 19]**

| [Table 3-1-3] Compounds that may be contained in mixture 2-1-d21C29-1 | | | | | |
|---|---|---|---|---|---|
| **ID** | **Exact Mass** | **m/z** | | **a** | **B** |
| 2-1-d21C29-1-0001 | 398.179 | 399 | [M+H]⁺ | a2 | B11 |
| 2-1-d21C29-1-0002 | 399.195 | 400 | [M+H]⁺ | a2 | B12 |
| 2-1-d21C29-1-0003 | 414.206 | 415 | [M+H]⁺ | a2 | B13 |
| 2-1-d21C29-1-0004 | 416.17 | 417 | [M+H]⁺ | a2 | B14 |
| 2-1-d21C29-1-0005 | 417.169 | 418 | [M+H]⁺ | a1 | B01 |
| 2-1-d21C29-1-0006 | 418.164 | 419 | [M+H]⁺ | a1 | B02 |
| 2-1-d21C29-1-0007 | 430.186 | 431 | [M+H]⁺ | a2 | B15 |
| 2-1-d21C29-1-0008 | 432.184 | 433 | [M+H]⁺ | a2 | B16 |
| 2-1-d21C29-1-0009 | 434.144 | 435 | [M+H]⁺ | a1 | B03 |
| 2-1-d21C29-1-0010 | 436.159 | 437 | [M+H]⁺ | a2 | B17 |
| 2-1-d21C29-1-0011 | 437.155 | 438 | [M+H]⁺ | a1 | B04 |
| 2-1-d21C29-1-0012 | 448.179 | 449 | [M+H]⁺ | a1 | B05 |
| 2-1-d21C29-1-0013 | 450.231 | 451 | [M+H]⁺ | a1 | B06 |
| 2-1-d21C29-1-0014 | 453.13 | 454 | [M+H]⁺ | a2 | B18 |
| 2-1-d21C29-1-0015 | 456.141 | 457 | [M+H]⁺ | a1 | B07 |
| 2-1-d21C29-1-0016 | 470.125 | 471 | [M+H]⁺ | a2 | B19 |
| 2-1-d21C29-1-0017 | 478.187 | 479 | [M+H]⁺ | a1 | B08 |
| 2-1-d21C29-1-0018 | 485.231 | 486 | [M+H]⁺ | a2 | B20 |
| 2-1-d21C29-1-0019 | 486.231 | 487 | [M+H]⁺ | a1 | B09 |
| 2-1-d21C29-1-0020 | 504.202 | 505 | [M+H]⁺ | a1 | B10 |

From the above results, it was shown that the Boc-deprotection reaction of Boc-protected aliphatic amine can be performed using the combination of Lewis acid Tm(OTf)₃ and base DTBP even when the substrate is not a single compound but a mixture of a plurality of compounds.

### Example 3-2: Boc-deprotection reaction in mixture 2-1-b2C19-0

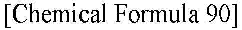

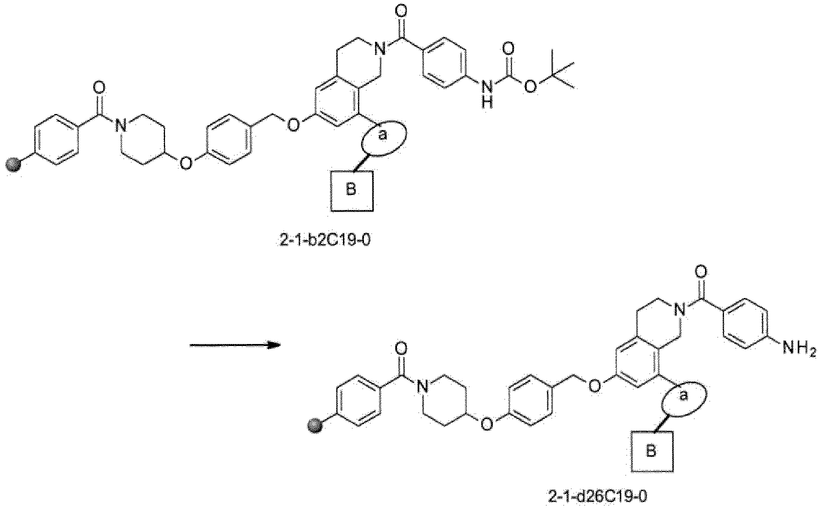

The compounds contained in the separately prepared mixture 2-1-b2C19-0 are as shown in [Table 3-2-1]. In [Table 3-2-1], each compound contained in the mixture is represented by ID, and the structure is represented by indicating the combination of corresponding parts a and B in the structural formula of the mixture. The details of parts a and B are as described above.

**[Table 20]**

| [Table 3-2-1] Compounds contained in mixture 2-1-b2C19-0 | | |
|---|---|---|
| **ID** | **a** | **B** |
| 2-1-b2C19-0-0001 | a2 | B11 |
| 2-1-b2C19-0-0002 | a2 | B12 |
| 2-1-b2C19-0-0003 | a2 | B13 |
| 2-1-b2C19-0-0004 | a2 | B14 |
| 2-1-b2C19-0-0005 | a1 | B01 |
| 2-1-b2C19-0-0006 | a1 | B02 |
| 2-1-b2C19-0-0007 | a2 | B15 |
| 2-1-b2C19-0-0008 | a2 | B16 |
| 2-1-b2C19-0-0009 | a1 | B03 |
| 2-1-b2C19-0-0010 | a2 | B17 |
| 2-1-b2C19-0-0011 | a1 | B04 |
| 2-1-b2C19-0-0012 | a1 | B05 |
| 2-1-b2C19-0-0013 | a1 | B06 |
| 2-1-b2C19-0-0014 | a2 | B18 |
| 2-1-b2C19-0-0015 | a1 | B07 |
| 2-1-b2C19-0-0016 | a2 | B19 |
| 2-1-b2C19-0-0017 | a1 | B08 |
| 2-1-b2C19-0-0018 | a2 | B20 |
| 2-1-b2C19-0-0019 | a1 | B09 |
| 2-1-b2C19-0-0020 | a1 | B10 |

To a 4 mL glass vial, mixture 2-1-b2C19-0 (70 mg) and 2-MeTHF (1.4 mL) were added, and the vial was shaken at room temperature for 1 hour. DTBP (88 µL, 0.40 mmol) and Tm(OTf)₃ (165 mg, 0.267 mmol) were added and the vial was shaken at 50°C for 15 hours.

To the resulting reaction solution, an NMP solution of DMEDA (1 M, 0.669 mL) was added, and the vial was shaken at room temperature for 1.5 hours. The suspension of the reaction solution and solid phase was transferred to a 3 mL column with a filter, filtered, and then washed 3 times with an NMP solution of DMEDA (0.2 M, 1.5 mL), 3 times with NMP (1.5 mL), 3 times with an NMP solution of mixed TBAHSO₄ and DTBP (both 0.05 M, 1.5 mL), 3 times with an NMP solution of BTMG (0.05 M, 1.5 mL), 3 times with an NMP solution of DTBP (0.05 M, 1.5 mL), 3 times with NMP/water = 1/1 (1.5 mL), 3 times with NMP (1.5 mL), 3 times with MeOH (1.5 mL), 3 times with DCM (1.5 mL), and 3 times with heptane (1.5 mL). The resulting solid phase was dried under reduced pressure to obtain mixture 2-1-d26C19-0.

The compounds contained in the mixture 2-1-d26C19-0 are as shown in [Table 3-2-2]. In [Table 3-2-2], each compound that may be contained in the mixture is represented by ID, and the structure is represented by indicating the combination of corresponding parts a and B in the structural formula of the mixture by symbols. The correspondence between symbols of parts a and B and structural formulas is as described above.

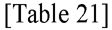

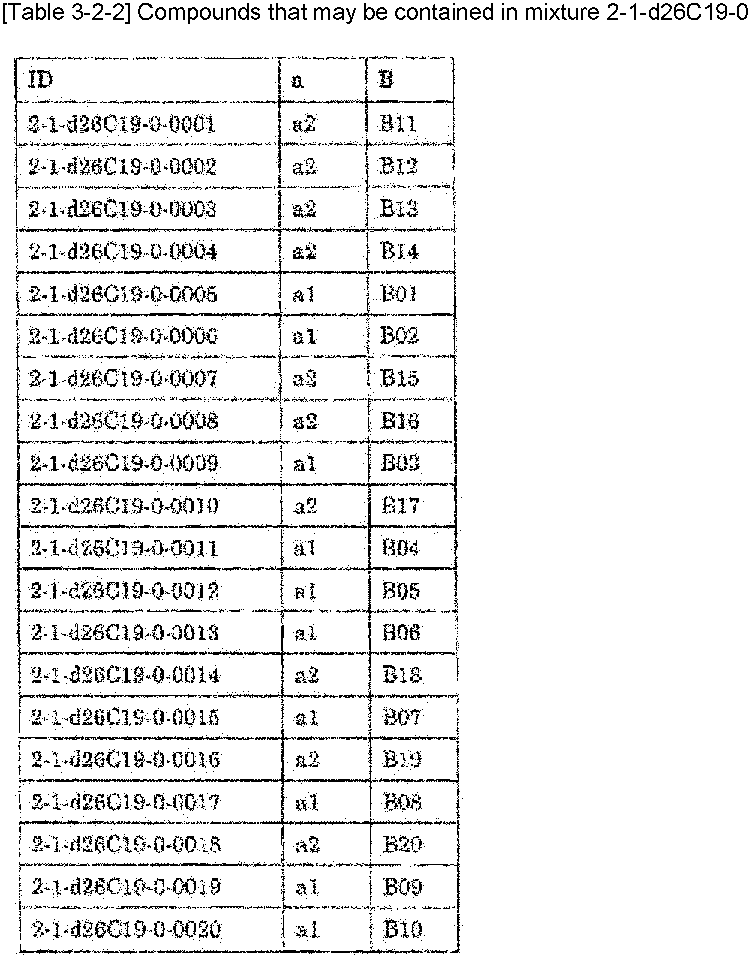

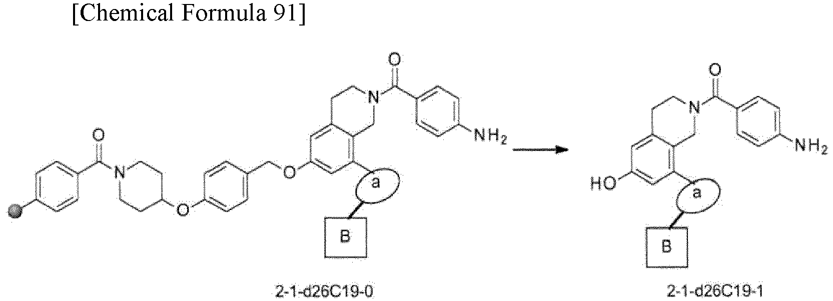

A small amount of mixture 2-1-d26C19-0 was transferred to a tip with a filter and washed 3 times with DMA (0.1 mL), 3 times with MeOH (0.1 mL), and 3 times with DCM (0.1 mL). The resultant was immersed in a 10% TFA/DCM solution (0.05 M, 0.05 mL) of pentamethylbenzene for 10 minutes, filtered, and washed with DMA (0.05 mL). The combined filtrates were diluted with MeCN (0.25 mL) to obtain a solution of mixture 2-1-d26C19-1. In LCMS measurements of this solution, all m/z ([M+H]⁺) derived from compounds that may be contained in the mixture 2-1-d26C19-1 described in [Table 3-2-3] were observed. In [Table 3-2-3], each compound that may be contained in the mixture is represented by ID, and the structure is represented by indicating the combination of corresponding parts a and B in the structural formula of the mixture by symbols. The correspondence between symbols of parts a and B and structural formulas is as described above. This result showed that the mixture 2-1-d26C19-0 described in [Table 3-2-2] was correctly synthesized.

**[Table 22]**

| [Table 3-2-3] Compounds that may be contained in mixture 2-1-d26C19-1 | | | | | |
|---|---|---|---|---|---|
| **ID** | **Exact Mass** | **m/z** | | **a** | **B** |
| 2-1-d26C19-1-0001 | 379.3.9 | 380 | [M+H]⁺ | a2 | B11 |
| 2-1-d26C19-1-0002 | 380.185 | 381 | [M+H]⁺ | a2 | B12 |
| 2-1-d26C19-1-0003 | 387.138 | 388 | [M+H]⁺ | a2 | B13 |
| 2-1-d26C19-1-0004 | 393.205 | 394 | [M+H]⁺ | a2 | B14 |
| 2-1-d26C19-1-0005 | 396.184 | 397 | [M+H]⁺ | a1 | B01 |
| 2-1-d26C19-1-0006 | 396.184 | 397 | [M+H]⁺ | a1 | B02 |
| 2-1-d26C19-1-0007 | 400.159 | 401 | [M+H]⁺ | a2 | B15 |
| 2-1-d26C19-1-0008 | 402.169 | 403 | [M+H]⁺ | a2 | B16 |
| 2-1-d26C19-1-0009 | 408.22 | 409 | [M+H]⁺ | a1 | B03 |
| 2-1-d26C19-1-0010 | 410.199 | 411 | [M+H]⁺ | a2 | B17 |
| 2-1-d26C19-1-0011 | 412.195 | 413 | [M+H]⁺ | a1 | B04 |
| 2-1-d26C19-1-0012 | 418.164 | 419 | [M+H]⁺ | a1 | B05 |
| 2-1-d26C19-1-0013 | 423.139 | 424 | [M+H]⁺ | a1 | B06 |
| 2-1-d26C19-1-0014 | 427.226 | 428 | [M+H]⁺ | a2 | B18 |
| 2-1-d26C19-1-0015 | 430.205 | 431 | [M+H]⁺ | a1 | B07 |
| 2-1-d26C19-1-0016 | 444.201 | 445 | [M+H]⁺ | a2 | B19 |
| 2-1-d26C19-1-0017 | 452.135 | 453 | [M+H]⁺ | a1 | B08 |
| 2-1-d26C19-1-0018 | 458.141 | 459 | [M+H]⁺ | a2 | B20 |
| 2-1-d26C19-1-0019 | 464.171 | 465 | [M+H]⁺ | a1 | B09 |
| 2-1-d26C19-1-0020 | 481.198 | 482 | [M+H]⁺ | a1 | B10 |

From the above results, it was shown that the Boc-deprotection reaction of Boc-protected aromatic amine can be performed using the combination of Lewis acid Tm(OTf)₃ and the base DTBP even when the substrate is not a single compound but a mixture of a plurality of compounds.

It was shown that even in the production of compound libraries with various structures, it is possible to efficiently construct a compound library by performing a step of deprotecting Boc groups on nitrogen atoms in a plurality of compounds by applying the reaction conditions shown in Examples.

## Claims

1. A method for producing a compound, comprising treating a compound having a tert-butoxycarbonyl (Boc) group on a nitrogen atom with a trifluoromethanesulfonic acid metal salt (metal triflate: M(OTf)ₙ) or rare-earth metal halide (MXₙ) in the presence of a base to obtain a compound in which the Boc group is removed.

2. The method according to claim 1, wherein the metal triflate is selected from the group consisting of tin triflate (Sn(OTf)₂), calcium triflate (Ca(OTf)₂), barium triflate (Ba(OTf)₂), zinc triflate (Zn(OTf)₂), aluminum triflate (Al(OTf)₃), gallium triflate (Ga(OTf)₃), indium triflate (In(OTf)₃), bismuth triflate (Bi(OTf)₃), lithium triflate (Li(OTf)), scandium triflate (Sc(OTf)₃), yttrium triflate (Y(OTf)₃), lanthanum triflate (La(OTf)₃), cerium triflate (Ce(OTf)₃, Ce(OTf)₄), praseodymium triflate (Pr(OTf)₃), neodymium triflate (Nd(OTf)₃), samarium triflate (Sm(OTf)₃), europium triflate (Eu(OTf)₃), gadolinium triflate (Gd(OTf)₃), terbium triflate (Tb(OTf)₃), dysprosium triflate (Dy(OTf)₃), holmium triflate (Ho(OTf)₃), erbium triflate (Er(OTf)₃), thulium triflate (Tm(OTf)₃), ytterbium triflate (Yb(OTf)₃), lutetium triflate (Lu(OTf)₃), hafnium triflate (Hf(OTf)₄), erbium chloride (ErCl₃), erbium bromide (ErBr₃), erbium iodide (ErI₃), thulium chloride (TmCl₃), thulium bromide (TmBr₃), thulium iodide (TmI₃), ytterbium chloride (YbCl₃), ytterbium bromide (YbBr₃), and ytterbium iodide (YbI₃), and two or more of the metal triflate may be used.

3. The method according to claim 1 or 2, wherein the base is selected from the group consisting of organic bases having pKa of conjugate acid in water of 1 to 14, and two or more of the base may be used.

4. The method according to any one of claims 1 to 3, wherein the base is selected from the group consisting of bases represented by formula (B1) or (B2): 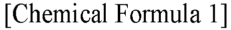 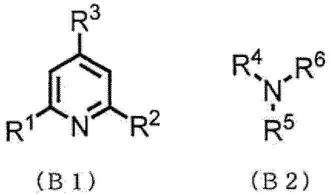
wherein R¹, R², and R³ are each independently selected from a hydrogen atom, C₁₋₆ alkyl, or C₆₋₁₀ aryl;
R⁴ and R⁵ together with a nitrogen atom to which they are attached form a 5- to 7-membered saturated heterocycle, the heterocycle optionally containing O, S, or N, or R⁴ and R⁵ are each independently C₁₋₆ alkyl, C₆₋₁₀ aryl, or tri(C₁₋₆ alkyl)silyl; and
R⁶ is a hydrogen atom or C₁₋₆ alkyl, and
two or more of the base may be used.

5. The method according to any one of claims 1 to 4, wherein the base is selected from the group consisting of 2,6-lutidine, N,N-diisopropylethylamine, N-methylmorpholine, 2,6-di-tert-butylpyridine, and di(trimethylsilyl)amine, and two or more of the base may be used.

6. The method according to any one of claims 1 to 5, wherein the treating is performed in the presence of a solvent, and the solvent is selected from the group consisting of an ether-based solvent, a halogen-based solvent, a nitrile-based solvent, an aromatic hydrocarbon-based solvent, and an ester-based solvent, and two or more of the solvent may be used.

7. The method according to any one of claims 1 to 6, wherein the treating is performed at a reaction temperature of 0°C to 120°C.

8. The method according to any one of claims 1 to 7, wherein the compound having a Boc group on a nitrogen atom is a resin for solid-phase synthesis to which the compound having a Boc group is attached via a linker.

9. The method according to any one of claims 1 to 8, wherein the compound having a Boc group on a nitrogen atom is a compound represented by formula (A): 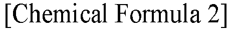 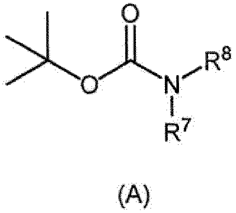
wherein R⁷ and R⁸ together with a nitrogen atom to which they are attached form a 5- to 7-membered saturated heterocycle, where the heterocycle is optionally substituted with an arbitrary substituent, or R⁷ and R⁸ are each independently a hydrogen atom, C₁₋₆ alkyl, C₂₋₆ alkenyl, C₂₋₆ alkynyl, C₃₋₈ cycloalkyl, C₇₋₁₄ aralkyl, C₆₋₁₀ aryl, or 5- to 10-membered heteroaryl containing one or more ring heteroatoms independently selected from O, N, and S, each of which is optionally substituted with one or more substituents, or
wherein the compound having a Boc group on a nitrogen atom is a resin for solid-phase synthesis to which the compound represented by formula (A) is attached via a linker.

10. The method according to any one of claims 1 to 8, wherein the method comprises, after the treating, a step of mixing with a solution containing a compound selected from the group consisting of N,N'-dimethylethylenediamine, ethylenediamine, diethylenetriamine, tris(2-aminoethyl)amine, tris(3-aminopropyl)amine, bis(3-aminopropyl)amine, 2,2'-bipyridyl, and 4,4'-di-tert-butyl-2,2'-bipyridyl.

11. The method according to any one of claims 1 to 10, wherein the compound having a Boc group on a nitrogen atom comprises a structure capable of being decomposed by treatment under an acidic condition, the structure being other than a Boc group.

12. The method according to any one of claims 1 to 11, wherein the method comprises treating a mixture containing two or more different compounds having a Boc group on a nitrogen atom.

13. The method according to any one of claims 1 to 12, for producing a compound constituting a compound library.

14. Use of a trifluoromethanesulfonic acid metal salt (metal triflate: M(OTf)ₙ) or rare-earth metal halide (MXₙ) in a deprotection reaction of a tert-butoxycarbonyl (Boc) group in a compound having a Boc group on a nitrogen atom, wherein the deprotection reaction is performed in the presence of a base.

15. A method for improving a yield in a deprotection reaction of a tert-butoxycarbonyl (Boc) group in a compound having a Boc group on a nitrogen atom, comprising treating the compound with a trifluoromethanesulfonic acid metal salt (metal triflate: M(OTf)ₙ) or rare-earth metal halide (MXₙ) in the presence of a base to obtain a compound in which the Boc group is converted to H-.
